# EUROPÄISCHE PATENTSCHRIFT

(11) **EP 1 921 150 B1**
(45) Veröffentlichungstag und Bekanntmachung des Hinweises auf die Patenterteilung: **18.09.2013**
(21) Anmeldenummer: 07123783.8
(22) Anmeldetag: 15.12.2004
(51) Int. Cl.: C12N 15/77, C07K 14/34

(54) **Pgro-Expressionseinheiten**
PGRO expression units
Unités d'expression PGRO

(30) Priorität: 18.12.2003 DE 10359595
(43) Veröffentlichungstag der Anmeldung: 14.05.2008
(62) Teilanmeldung aus: 04803884.8
(73) Patentinhaber: BASF SE, 67056 Ludwigshafen (DE)
(72) Erfinder: Kröger, Burkhard, 67117 Limburgerhof (DE); Zelder, Oskar, 67346 Speyer (DE); Klopprogge, Corinna, 68239 Mannheim (DE); Schröder, Hartwig, 69226 Nußloch (DE); Haefner, Stefan, 67346 Speyer (DE)

(56) Entgegenhaltungen:
- WO-A2-02/40679
- DE LEÓN P ET AL: "Streptomyces lividans groES, groEL1 and groEL2 genes." MICROBIOLOGY (READING, ENGLAND) NOV 1997, Bd. 143 ( Pt 11), November 1997 (1997-11), Seiten 3563-3571, XP002323102 ISSN: 1350-0872
- TAUSCHEK M ET AL: "Transcriptional analysis of the groESL operon of Neisseria gonorrhoeae" GENE: AN INTERNATIONAL JOURNAL ON GENES AND GENOMES, ELSEVIER SCIENCE PUBLISHERS, BARKING, GB, Bd. 189, Nr. 1, 11. April 1997 (1997-04-11), Seiten 107-112, XP004059567 ISSN: 0378-1119
- THIES F L ET AL: "Cloning, sequencing and molecular analysis of the Campylobacter jejuni groESL bicistronic operon." MICROBIOLOGY (READING, ENGLAND) JAN 1999, Bd. 145 ( Pt 1), Januar 1999 (1999-01), Seiten 89-98, XP002323246 ISSN: 1350-0872
- GUPTA R S: "EVOLUTION OF THE CHAPERONIN FAMILIES (HSP60, HSP10 AND TCP-1) OF PROTEINS AND THE ORIGIN OF EUKARYOTIC CELLS" MOLECULAR MICROBIOLOGY, BLACKWELL SCIENTIFIC, OXFORD, GB, Bd. 15, Nr. 1, 1995, Seiten 1-11, XP000611319 ISSN: 0950-382X
- KALINOWSKI J ET AL: "THE COMPLETE CORYNEBACTERIUM GLUTAMICUM ATCC 13032 GENONE SEQUENCE AND ITS IMPACT ON THE PRODUCTION OF L-ASPARTATE-DERIVED AMINO ACIDS AND VITAMINS" JOURNAL OF BIOTECHNOLOGY, ELSEVIER SCIENCE PUBLISHERS B.V., AMSTERDAM, NL, Bd. 104, Nr. 1-3, 4. September 2003 (2003-09-04), Seiten 5-25, XP001184752 ISSN: 0168-1656
- PÁTEK MIROSLAV ET AL: "Function of Corynebacterium glutamicum promoters in Escherichia coli, Streptomyces lividans, and Bacillus subtilis." 4. September 2003 (2003-09-04), JOURNAL OF BIOTECHNOLOGY. 4 SEP 2003, VOL. 104, NR. 1-3, PAGE(S) 325 - 334 , XP002323103 ISSN: 0168-1656 * das ganze Dokument *
- PÁTEK MIROSLAV ET AL: "Promoters of Corynebacterium glutamicum." JOURNAL OF BIOTECHNOLOGY. 4 SEP 2003, Bd. 104, Nr. 1-3, 4. September 2003 (2003-09-04), Seiten 311-323, XP002323247 ISSN: 0168-1656

### SEQUENCE LISTING

<110> BASF Aktiengesellschaft
<120> Pgro-Expressionseinheiten
<130> PF 55184/Mec
<160> 53
<210> 1
   <211> 164
   <212> DNA
   <213> Corynebacterium glutamicum
<220>
   <223> SEQ_ID_1_GROES_RXA00497_PROMOTOR
<400> 1
   cggcttaaag tttggctgcc atgtgaattt ttagcaccct caacagttga gtgctggcac 60
   tctcgggggt agagtgccaa ataggttgtt tgacacacag ttgttcaccc gcgacgacgg 120
   ctgtgctgga aacccacaac cggcacacac aaaatttttc tcat                  164
<210> 2
   <211> 177
   <212> DNA
   <213> Corynebacterium glutamicum
<220>
   <223> SEQ_ID_2_GROES_RXA00497_GESAMTE_EXPRESSIONSEINHEIT
<400> 2
   cggcttaaag tttggctgcc atgtgaattt ttagcaccct caacagttga gtgctggcac 60
   tctcgggggt agagtgccaa ataggttgtt tgacacacag ttgttcaccc gcgacgacgg 120
   ctgtgctgga aacccacaac cggcacacac aaaatttttc tcatggaggg attcatc    177
<210> 3
   <211> 1365
   <212> DNA
   <213> Corynebacterium glutamicum
<220>
   <223> SEQ_ID_3_RXA00077
<400> 3
   atgaatgatg agaatattca aagctccaac tatcagccat tcccgagttt tgacgattgg 60
   aaacagatcg aggtgtcgct cttagatgtc atcgaatcct cacgccattt ttctgatttg 120
   aaagatagca ctgatcgttc tgcgttagat gctgcgctag agagagcaaa aagagctgcc 180
   gcagttgata ccaatgccat agaaggaatc ttccaaactg atcgcggttt tacccataca 240
   gttgcaacgc aggtaggggc ttgggagcaa caaatggcga tgaaaggcaa acatgttaag 300
   cctgcgtttg acgatactct agaaggcttt gagtatgttc tcgatgcagt aactggtaga 360
   actccaatct ctcagcaatg gattagaaat ttgcacgccg tcattctgcg gagccaagaa 420
   agccacgagg tttttacagc cgttggagtc caaaatcagg cgcttcagaa aggcgagtat 480
   aaaactcagc caaatagtcc acagcgctca gatggatctg tacatgcata cgccccagtt 540
   gaagatactc ctgctgaaat ggctagattt atttcagaac ttgaatctaa ggaattctta 600
   gcagccgaga aggttattca agctgcctat gcccactatg ctttcgtatg tattcatcct 660
   tttgcagatg ggaatggacg agttgcacga gccttggcta gtgtttttct atacaaagat 720
   cctggtgtcc ctctcgtaat ctaccaagat caacgcagag attacatcca tgctctagaa 780
   gcagcggaca agaataaccc gctcctgctg attagattct ttgctgaacg agtgaccgat 840
   actattaact ctattatcgt tgatctcact accccgatcg cgggtaaatc tggttcggct 900
   aagctttcgg atgcgctacg ccccactcgc gtattaccag aattacatga tgctgcacat 960
   aggctccaag aaagtttatt tacagaaatc cgatctcgat tggatgaaga aggaaaaagg 1020
   aatgggttgg agtttctact tcaacggatt tttatcggtt ccccattcaa tctgccagag 1080
   ggctataacg ctttccctga tagctattgt ctgaccttag ctttcaatag caactctcca 1140
   aaacaaatct tccacccgct atccatagta atagcagctc gagatgggaa aagagcgagc 1200
   agcgacctcg tggcagctac ttctattgga tacaactttc acgcttacgg acgtgaagtc 1260
   gagcctgttg ttactgaaag ctttcgagaa cgtgtgaaaa tttacgccga cgggattgta 1320
   gatcacttct taaccgaact ggctaaaaag tttcaacaga attaa 1365
<210> 4
   <211> 454
   <212> PRT
   <213> corynebacterium glutamicum
<400> 4
   Met Asn Asp Glu Asn Ile Gln Ser Ser Asn Tyr Gln Pro Phe Pro Ser
   1               5                   10                  15
   Phe Asp Asp Trp Lys Gln Ile Glu Val Ser Leu Leu Asp Val Ile Glu
               20                  25                  30
   Ser Ser Arg His Phe Ser Asp Leu Lys Asp Ser Thr Asp Arg Ser Ala
           35                  40                  45
   Leu Asp Ala Ala Leu Glu Arg Ala Lys Arg Ala Ala Ala Val Asp Thr
       50                  55                  60
   Asn Ala Ile Glu Gly Ile Phe Gln Thr Asp Arg Gly Phe Thr His Thr
   65                  70                  75                  80
   Val Ala Thr Gln Val Gly Ala Trp Glu Gln Gln Met Ala Met Lys Gly
                   85                  90                  95
   Lys His Val Lys Pro Ala Phe Asp Asp Thr Leu Glu Gly Phe Glu Tyr
               100                 105                 110
   Val Leu Asp Ala Val Thr Gly Arg Thr Pro Ile Ser Gln Gln Trp Ile
           115                 120                 125
   Arg Asn Leu His Ala Val Ile Leu Arg Ser Gln Glu Ser His Glu Val
       130                 135                 140
   Phe Thr Ala Val Gly Val Gln Asn Gln Ala Leu Gln Lys Gly Glu Tyr
   145                 150                 155                 160
   Lys Thr Gln Pro Asn Ser Pro Gln Arg Ser Asp Gly Ser Val His Ala
                   165                 170                 175
   Tyr Ala Pro Val Glu Asp Thr Pro Ala Glu Met Ala Arg Phe Ile Ser
               180                 185                 190
   Glu Leu Glu Ser Lys Glu Phe Leu Ala Ala Glu Lys Val Ile Gln Ala
           195                 200                 205
   Ala Tyr Ala His Tyr Ala Phe Val Cys Ile His Pro Phe Ala Asp Gly
       210                 215                 220
   Asn Gly Arg Val Ala Arg Ala Leu Ala Ser Val Phe Leu Tyr Lys Asp
   225                 230                 235                 240
   Pro Gly Val Pro Leu Val Ile Tyr Gln Asp Gln Arg Arg Asp Tyr Ile
                   245                 250                 255
   His Ala Leu Glu Ala Ala Asp Lys Asn Asn Pro Leu Leu Leu Ile Arg
   260 265 270
   Phe Phe Ala Glu Arg Val Thr Asp Thr Ile Asn Ser Ile Ile Val Asp
   275 280 285
   Leu Thr Thr Pro Ile Ala Gly Lys Ser Gly Ser Ala Lys Leu Ser Asp
   290 295 300
   Ala Leu Arg Pro Thr Arg Val Leu Pro Glu Leu His Asp Ala Ala His
   305 310 315 320
   Arg Leu Gln Glu Ser Leu Phe Thr Glu Ile Arg Ser Arg Leu Asp Glu
   325 330 335
   Glu Gly Lys Arg Asn Gly Leu Glu Phe Leu Leu Gln Arg Ile Phe Ile
   340 345 350
   Gly Ser Pro Phe Asn Leu Pro Glu Gly Tyr Asn Ala Phe Pro Asp Ser
   355 360 365
   Tyr Cys Leu Thr Leu Ala Phe Asn Ser Asn Ser Pro Lys Gln Ile Phe
   370 375 380
   His Pro Leu Ser Ile Val Ile Ala Ala Arg Asp Gly Lys Arg Ala Ser
   385 390 395 400
   Ser Asp Leu Val Ala Ala Thr Ser Ile Gly Tyr Asn Phe His Ala Tyr
   405 410 415
   Gly Arg Glu Val Glu Pro Val Val Thr Glu Ser Phe Arg Glu Arg Val
   420 425 430
   Lys Ile Tyr Ala Asp Gly Ile Val Asp His Phe Leu Thr Glu Leu Ala
   435 440 445
   Lys Lys Phe Gln Gln Asn
   450
<210> 5
   <211> 19
   <212> DNA
   <213> Corynebacterium glutamicum
<220>
   <223> SEQ_ID_5_GRO3
<400> 5
   gccgcagcaa acccagtag 19
<210> 6
   <211> 31
   <212> DNA
   <213> Corynebacterium glutamicum
<220>
   <223> SEQ_ID_6_GRO11
<400> 6
   agtcgacacg atgaatccct ccatgagaaa a 31
<210> 7
   <211> 54
   <212> DNA
   <213> Corynebacterium glutamicum
<220>
   <223> SEQ_ID_7_PYC6
<400> 7
   tttttctcat ggagggattc atcgtgtcga ctcacacatc ttcaacgctt ccag 54
<210> 8
   <211> 23
   <212> DNA
   <213> Corynebacterium glutamicum
<220>
   <223> SEQ_ID_8_PYC3
<400> 8
   cccgcagcaa cgcacgcaag aaa 23
<210> 9
   <211> 22
   <212> DNA
   <213> Corynebacterium glutamicum
<220>
   <223> SEQ_ID_9_GRO12
<400> 9
   gcattcgcgc cgctcgtaac ta 22
<210> 10
   <211> 19
   <212> DNA
   <213> Corynebacterium glutamicum
<220>
   <223> SEQ_ID_10_PYC11
<400> 10
   ggttcccgcg ccctggtaa 19
<210> 11
   <211> 5720
   <212> DNA
   <213> Corynebacterium glutamicum
<220>
   <223> SEQ_ID_11_PK19_MOB_SACB
<220>
   <221> misc_feature
   <222> (562 )..(1356 )
   <223> KanR
<220>
   <221> misc_feature
   <222> (1385 )..(1847 )
   <223> PsacB
<220>
   <221> misc_feature
   <222> (1848 )..(3266 )
   <223> SacB
<400> 11
   ggtcgactct agaggatccc cgggtaccga gctcgaattc actggccgtc gttttacaac 60
   gtcgtgactg ggaaaaccct ggcgttaccc aacttaatcg ccttgcagca catccccctt 120
   tcgccagctg gcgtaatagc gaagaggccc gcaccgatcg cccttcccaa cagttgcgca 180
   gcctgaatgg cgaatggcga taagctagct tcacgctgcc gcaagcactc agggcgcaag 240
   ggctgctaaa ggaagcggaa cacgtagaaa gccagtccgc agaaacggtg ctgaccccgg 300
   atgaatgtca gctactgggc tatctggaca agggaaaacg caagcgcaaa gagaaagcag 360
   gtagcttgca gtgggcttac atggcgatag ctagactggg cggttttatg gacagcaagc 420
   gaaccggaat tgccagctgg ggcgccctct ggtaaggttg ggaagccctg caaagtaaac 480
   tggatggctt tcttgccgcc aaggatctga tggcgcaggg gatcaagatc tgatcaagag 540
   acaggatgag gatcgtttcg catgattgaa caagatggat tgcacgcagg ttctccggcc 600
   gcttgggtgg agaggctatt cggctatgac tgggcacaac agacaatcgg ctgctctgat 660
   gccgccgtgt tccggctgtc agcgcagggg cgcccggttc tttttgtcaa gaccgacctg 720
   tccggtgccc tgaatgaact ccaagacgag gcagcgcggc tatcgtggct ggccacgacg 780
   ggcgttcctt gcgcagctgt gctcgacgtt gtcactgaag cgggaaggga ctggctgcta 840
   ttgggcgaag tgccggggca ggatctcctg tcatctcacc ttgctcctgc cgagaaagta 900
   tccatcatgg ctgatgcaat gcggcggctg catacgcttg atccggctac ctgcccattc 960
   gaccaccaag cgaaacatcg catcgagcga gcacgtactc ggatggaagc cggtcttgtc 1020
   gatcaggatg atctggacga agagcatcag gggctcgcgc cagccgaact gttcgccagg 1080
   ctcaaggcgc ggatgcccga cggcgaggat ctcgtcgtga cccatggcga tgcctgcttg 1140
   ccgaatatca tggtggaaaa tggccgcttt tctggattca tcgactgtgg ccggctgggt 1200
   gtggcggacc gctatcagga catagcgttg gctacccgtg atattgctga agagcttggc 1260
   ggcgaatggg ctgaccgctt cctcgtgctt tacggtatcg ccgctcccga ttcgcagcgc 1320
   atcgccttct atcgccttct tgacgagttc ttctgagcgg gactctgggg ttcgctagag 1380
   gatcgatcct ttttaaccca tcacatatac ctgccgttca ctattattta gtgaaatgag 1440
   atattatgat attttctgaa ttgtgattaa aaaggcaact ttatgcccat gcaacagaaa 1500
   ctataaaaaa tacagagaat gaaaagaaac agatagattt tttagttctt taggcccgta 1560
   gtctgcaaat ccttttatga ttttctatca aacaaaagag gaaaatagac cagttgcaat 1620
   ccaaacgaga gtctaataga atgaggtcga aaagtaaatc gcgcgggttt gttactgata 1680
   aagcaggcaa gacctaaaat gtgtaaaggg caaagtgtat actttggcgt caccccttac 1740
   atattttagg tcttttttta ttgtgcgtaa ctaacttgcc atcttcaaac aggagggctg 1800
   gaagaagcag accgctaaca cagtacataa aaaaggagac atgaacgatg aacatcaaaa 1860
   agtttgcaaa acaagcaaca gtattaacct ttactaccgc actgctggca ggaggcgcaa 1920
   ctcaagcgtt tgcgaaagaa acgaaccaaa agccatataa ggaaacatac ggcatttccc 1980
   atattacacg ccatgatatg ctgcaaatcc ctgaacagca aaaaaatgaa aaatatcaag 2040
   tttctgaatt tgattcgtcc acaattaaaa atatctcttc tgcaaaaggc ctggacgttt 2100
   gggacagctg gccattacaa aacgctgacg gcactgtcgc aaactatcac ggctaccaca 2160
   tcgtctttgc attagccgga gatcctaaaa atgcggatga cacatcgatt tacatgttct 2220
   atcaaaaagt cggcgaaact tctattgaca gctggaaaaa cgctggccgc gtctttaaag 2280
   acagcgacaa attcgatgca aatgattcta tcctaaaaga ccaaacacaa gaatggtcag 2340
   gttcagccac atttacatct gacggaaaaa tccgtttatt ctacactgat ttctccggta 2400
   aacattacgg caaacaaaca ctgacaactg cacaagttaa cgtatcagca tcagacagct 2460
   ctttgaacat caacggtgta gaggattata aatcaatctt tgacggtgac ggaaaaacgt 2520
   atcaaaatgt acagcagttc atcgatgaag gcaactacag ctcaggcgac aaccatacgc 2580
   tgagagatcc tcactacgta gaagataaag gccacaaata cttagtattt gaagcaaaca 2640
   ctggaactga agatggctac caaggcgaag aatctttatt taacaaagca tactatggca 2700
   aaagcacatc attcttccgt caagaaagtc aaaaacttct gcaaagcgat aaaaaacgca 2760
   cggctgagtt agcaaacggc gctctcggta tgattgagct aaacgatgat tacacactga 2820
   aaaaagtgat gaaaccgctg attgcatcta acacagtaac agatgaaatt gaacgcgcga 2880
   acgtctttaa aatgaacggc aaatggtacc tgttcactga ctcccgcgga tcaaaaatga 2940
   cgattgacgg cattacgtct aacgatattt acatgcttgg ttatgtttct aattctttaa 3000
   ctggcccata caagccgctg aacaaaactg gccttgtgtt aaaaatggat cttgatccta 3060
   acgatgtaac ctttacttac tcacacttcg ctgtacctca agcgaaagga aacaatgtcg 3120
   tgattacaag ctatatgaca aacagaggat tctacgcaga caaacaatca acgtttgcgc 3180
   cgagcttcct gctgaacatc aaaggcaaga aaacatctgt tgtcaaagac agcatccttg 3240
   aacaaggaca attaacagtt aacaaataaa aacgcaaaag aaaatgccga tgggtaccga 3300
   gcgaaatgac cgaccaagcg acgcccaacc tgccatcacg agatttcgat tccaccgccg 3360
   ccttctatga aaggttgggc ttcggaatcg ttttccggga cgccctcgcg gacgtgctca 3420
   tagtccacga cgcccgtgat tttgtagccc tggccgacgg ccagcaggta ggccgacagg 3480
   ctcatgccgg ccgccgccgc cttttcctca atcgctcttc gttcgtctgg aaggcagtac 3540
   accttgatag gtgggctgcc cttcctggtt ggcttggttt catcagccat ccgcttgccc 3600
   tcatctgtta cgccggcggt agccggccag cctcgcagag caggattccc gttgagcacc 3660
   gccaggtgcg aataagggac agtgaagaag gaacacccgc tcgcgggtgg gcctacttca 3720
   cctatcctgc ccggctgacg ccgttggata caccaaggaa agtctacacg aaccctttgg 3780
   caaaatcctg tatatcgtgc gaaaaaggat ggatataccg aaaaaatcgc tataatgacc 3840
   ccgaagcagg gttatgcagc ggaaaagcgc tgcttccctg ctgttttgtg gaatatctac 3900
   cgactggaaa caggcaaatg caggaaatta ctgaactgag gggacaggcg agagacgatg 3960
   ccaaagagct cctgaaaatc tcgataactc aaaaaatacg cccggtagtg atcttatttc 4020
   attatggtga aagttggaac ctcttacgtg ccgatcaacg tctcattttc gccaaaagtt 4080
   ggcccagggc ttcccggtat caacagggac accaggattt atttattctg cgaagtgatc 4140
   ttccgtcaca ggtatttatt cggcgcaaag tgcgtcgggt gatgctgcca acttactgat 4200
   ttagtgtatg atggtgtttt tgaggtgctc cagtggcttc tgtttctatc agctcctgaa 4260
   aatctcgata actcaaaaaa tacgcccggt agtgatctta tttcattatg gtgaaagttg 4320
   gaacctctta cgtgccgatc aacgtctcat tttcgccaaa agttggccca gggcttcccg 4380
   gtatcaacag ggacaccagg atttatttat tctgcgaagt gatcttccgt cacaggtatt 4440
   tattcggcgc aaagtgcgtc gggtgatgct gccaacttac tgatttagtg tatgatggtg 4500
   tttttgaggt gctccagtgg cttctgtttc tatcagggct ggatgatcct ccagcgcggg 4560
   gatctcatgc tggagttctt cgcccacccc aaaaggatct aggtgaagat cctttttgat 4620
   aatctcatga ccaaaatccc ttaacgtgag ttttcgttcc actgagcgtc agaccccgta 4680
   gaaaagatca aaggatcttc ttgagatcct ttttttctgc gcgtaatctg ctgcttgcaa 4740
   acaaaaaaac caccgctacc agcggtggtt tgtttgccgg atcaagagct accaactctt 4800
   tttccgaagg taactggctt cagcagagcg cagataccaa atactgttct tctagtgtag 4860
   ccgtagttag gccaccactt caagaactct gtagcaccgc ctacatacct cgctctgcta 4920
   atcctgttac cagtggctgc tgccagtggc gataagtcgt gtcttaccgg gttggactca 4980
   agacgatagt taccggataa ggcgcagcgg tcgggctgaa cggggggttc gtgcacacag 5040
   cccagcttgg agcgaacgac ctacaccgaa ctgagatacc tacagcgtga gctatgagaa 5100
   agcgccacgc ttcccgaagg gagaaaggcg gacaggtatc cggtaagcgg cagggtcgga 5160
   acaggagagc gcacgaggga gcttccaggg ggaaacgcct ggtatcttta tagtcctgtc 5220
   gggtttcgcc acctctgact tgagcgtcga tttttgtgat gctcgtcagg ggggcggagc 5280
   ctatggaaaa acgccagcaa cgcggccttt ttacggttcc tggccttttg ctggcctttt 5340
   gctcacatgt tctttcctgc gttatcccct gattctgtgg ataaccgtat taccgccttt 5400
   gagtgagctg ataccgctcg ccgcagccga acgaccgagc gcagcgagtc agtgagcgag 5460
   gaagcggaag agcgcccaat acgcaaaccg cctctccccg cgcgttggcc gattcattaa 5520
   tgcagctggc acgacaggtt tcccgactgg aaagcgggca gtgagcgcaa cgcaattaat 5580
   gtgagttagc tcactcatta ggcaccccag gctttacact ttatgcttcc ggctcgtatg 5640
   ttgtgtggaa ttgtgagcgg ataacaattt cacacaggaa acagctatga ccatgattac 5700
   gccaagcttg catgcctgca 5720
<210> 12
   <211> 24
   <212> DNA
   <213> Corynebacterium glutamicum
<220>
   <223> SEQ_ID_12_PYC14
<400> 12
   ccggcgaagt gtctgctcgc gtga 24
<210> 13
   <211> 18
   <212> DNA
   <213> Corynebacterium glutamicum
<220>
   <223> SEQ_ID_13_PYC15
<400> 13
   accccgcccc agtttttc 18
<210> 14
   <211> 7438
   <212> DNA
   <213> Corynebacterium glutamicum
<220>
   <223> SEQ_ID_14_PK19_MOB
<220>
   <221> misc_feature
   <222> (1 )..(1422 )
   <223> SacB complement
<220>
   <221> misc_feature
   <222> (1423 )..(1885 )
   <223> PsacB complement
<220>
   <221> misc_feature
   <222> (1914 )..(2708 )
   <223> KanR
<220>
   <221> misc_feature
   <222> (3118 )..(3622 )
   <223> 5' pycA
<220>
   <221> misc_feature
   <222> (3863 )..(4039 )
   <223> Pgro
<220>
   <221> misc_feature
   <222> (4040 )..(4888 )
   <223> Teil pycA
<400> 14
   ttatttgtta actgttaatt gtccttgttc aaggatgctg tctttgacaa cagatgtttt 60
   cttgcctttg atgttcagca ggaagctcgg cgcaaacgtt gattgtttgt ctgcgtagaa 120
   tcctctgttt gtcatatagc ttgtaatcac gacattgttt cctttcgctt gaggtacagc 180
   gaagtgtgag taagtaaagg ttacatcgtt aggatcaaga tccattttta acacaaggcc 240
   agttttgttc agcggcttgt atgggccagt taaagaatta gaaacataac caagcatgta 300
   aatatcgtta gacgtaatgc cgtcaatcgt catttttgat ccgcgggagt cagtgaacag 360
   gtaccatttg ccgttcattt taaagacgtt cgcgcgttca atttcatctg ttactgtgtt 420
   agatgcaatc agcggtttca tcactttttt cagtgtgtaa tcatcgttta gctcaatcat 480
   accgagagcg ccgtttgcta actcagccgt gcgtttttta tcgctttgca gaagtttttg 540
   actttcttga cggaagaatg atgtgctttt gccatagtat gctttgttaa ataaagattc 600
   ttcgccttgg tagccatctt cagttccagt gtttgcttca aatactaagt atttgtggcc 660
   tttatcttct acgtagtgag gatctctcag cgtatggttg tcgcctgagc tgtagttgcc 720
   ttcatcgatg aactgctgta cattttgata cgtttttccg tcaccgtcaa agattgattt 780
   ataatcctct acaccgttga tgttcaaaga gctgtctgat gctgatacgt taacttgtgc 840
   agttgtcagt gtttgtttgc cgtaatgttt accggagaaa tcagtgtaga ataaacggat 900
   ttttccgtca gatgtaaatg tggctgaacc tgaccattct tgtgtttggt cttttaggat 960
   agaatcattt gcatcgaatt tgtcgctgtc tttaaagacg cggccagcgt ttttccagct 1020
   gtcaatagaa gtttcgccga ctttttgata gaacatgtaa atcgatgtgt catccgcatt 1080
   tttaggatct ccggctaatg caaagacgat gtggtagccg tgatagtttg cgacagtgcc 1140
   gtcagcgttt tgtaatggcc agctgtccca aacgtccagg ccttttgcag aagagatatt 1200
   tttaattgtg gacgaatcaa attcaggaac ttgatatttt tcattttttt gctgttcagg 1260
   gatttgcagc atatcatggc gtgtaatatg ggaaatgccg tatgtttcct tatatggctt 1320
   ttggttcgtt tctttcgcaa acgcttgagt tgcgcctcct gccagcagtg cggtagtaaa 1380
   ggttaatact gttgcttgtt ttgcaaactt tttgatgttc atcgttcatg tctccttttt 1440
   tatgtactgt gttagcggtc tgcttcttcc agccctcctg tttgaagatg gcaagttagt 1500
   tacgcacaat aaaaaaagac ctaaaatatg taaggggtga cgccaaagta tacactttgc 1560
   cctttacaca ttttaggtct tgcctgcttt atcagtaaca aacccgcgcg atttactttt 1620
   cgacctcatt ctattagact ctcgtttgga ttgcaactgg tctattttcc tcttttgttt 1680
   gatagaaaat cataaaagga tttgcagact acgggcctaa agaactaaaa aatctatctg 1740
   tttcttttca ttctctgtat tttttatagt ttctgttgca tgggcataaa gttgcctttt 1800
   taatcacaat tcagaaaata tcataatatc tcatttcact aaataatagt gaacggcagg 1860
   tatatgtgat gggttaaaaa ggatcgatcc tctagcgaac cccagagtcc cgctcagaag 1920
   aactcgtcaa gaaggcgata gaaggcgatg cgctgcgaat cgggagcggc gataccgtaa 1980
   agcacgagga agcggtcagc ccattcgccg ccaagctctt cagcaatatc acgggtagcc 2040
   aacgctatgt cctgatagcg gtccgccaca cccagccggc cacagtcgat gaatccagaa 2100
   aagcggccat tttccaccat gatattcggc aagcaggcat cgccatgggt cacgacgaga 2160
   tcctcgccgt cgggcatccg cgccttgagc ctggcgaaca gttcggctgg cgcgagcccc 2220
   tgatgctctt cgtccagatc atcctgatcg acaagaccgg cttccatccg agtacgtgct 2280
   cgctcgatgc gatgtttcgc ttggtggtcg aatgggcagg tagccggatc aagcgtatgc 2340
   agccgccgca ttgcatcagc catgatggat actttctcgg caggagcaag gtgagatgac 2400
   aggagatcct gccccggcac ttcgcccaat agcagccagt cccttcccgc ttcagtgaca 2460
   acgtcgagca cagctgcgca aggaacgccc gtcgtggcca gccacgatag ccgcgctgcc 2520
   tcgtcttgga gttcattcag ggcaccggac aggtcggtct tgacaaaaag aaccgggcgc 2580
   ccctgcgctg acagccggaa cacggcggca tcagagcagc cgattgtctg ttgtgcccag 2640
   tcatagccga atagcctctc cacccaagcg gccggagaac ctgcgtgcaa tccatcttgt 2700
   tcaatcatgc gaaacgatcc tcatcctgtc tcttgatcag atcttgatcc cctgcgccat 2760
   cagatccttg gcggcaagaa agccatccag tttactttgc agggcttccc aaccttacca 2820
   gagggcgccc cagctggcaa ttccggttcg cttgctgtcc ataaaaccgc ccagtctagc 2880
   tatcgccatg taagcccact gcaagctacc tgctttctct ttgcgcttgc gttttccctt 2940
   gtccagatag cccagtagct gacattcatc cggggtcagc accgtttctg cggactggct 3000
   ttctacgtgt tccgcttcct ttagcagccc ttgcgccctg agtgcttgcg gcagcgtgaa 3060
   gctagatgca tgctcgagcg gccgccagtg tgatggatat ctgcagaatt cgcccttccg 3120
   gcgaagtgtc tgctcgcgtg attgtgcttc ctttggctac taacccacgc gccaagatgc 3180
   gttccctgcg ccacggtttt gtgaagctgt tctgccgccg taactctggc ctgatcatcg 3240
   gtggtgtcgt ggtggcaccg accgcgtctg agctgatcct accgatcgct gtggcagtga 3300
   ccaaccgtct gacagttgct gatctggctg ataccttcgc ggtgtaccca tcattgtcag 3360
   gttcgattac tgaagcagca cgtcagctgg ttcaacatga tgatctaggc taatttttct 3420
   gagtcttaga ttttgagaaa acccaggatt gctttgtgca ctcctgggtt ttcactttgt 3480
   taagcagttt tggggaaaag tgcaaagttt gcaaagttta gaaatatttt aagaggtaag 3540
   atgtctgcag gtggaagcgt ttaaatgcgt taaacttggc caaatgtggc aacctttgca 3600
   aggtgaaaaa ctggggcggg gtaagggcga attccagcac actggcggcc gttactagct 3660
   tatcgccatt cgccattcag gctgcgcaac tgttgggaag ggcgatcggt gcgggcctct 3720
   tcgctattac gccagctggc gaaaggggga tgtgctgcaa ggcgattaag ttgggtaacg 3780
   ccagggtttt cccagtcacg acgttgtaaa acgacggcca gtgaattcaa cctgtggcgc 3840
   aacgctgtat ataacctgcg tacggcttaa agtttggctg ccatgtgaat ttttagcacc 3900
   ctcaacagtt gagtgctggc actctcgggg gtagagtgcc aaataggttg tttgacacac 3960
   agttgttcac ccgcgacgac ggctgtgctg gaaacccaca accggcacac acaaaatttt 4020
   tctcatggag ggattcatcg tgtcgactca cacatcttca acgcttccag cattcaaaaa 4080
   gatcttggta gcaaaccgcg gcgaaatcgc ggtccgtgct ttccgtgcag cactcgaaac 4140
   cggtgcagcc acggtagcta tttacccccg tgaagatcgg ggatcattcc accgctcttt 4200
   tgcttctgaa gctgtccgca ttggtaccga aggctcacca gtcaaggcgt acctggacat 4260
   cgatgaaatt atcggtgcag ctaaaaaagt taaagcagat gccatttacc cgggatacgg 4320
   cttcctgtct gaaaatgccc agcttgcccg cgagtgtgcg gaaaacggca ttacttttat 4380
   tggcccaacc ccagaggttc ttgatctcac cggtgataag tctcgcgcgg taaccgccgc 4440
   gaagaaggct ggtctgccag ttttggcgga atccaccccg agcaaaaaca tcgatgagat 4500
   cgttaaaagc gctgaaggcc agacttaccc catctttgtg aaggcagttg ccggtggtgg 4560
   cggacgcggt atgcgttttg ttgcttcacc tgatgagctt cgcaaattag caacagaagc 4620
   atctcgtgaa gctgaagcgg ctttcggcga tggcgcggta tatgtcgaac gtgctgtgat 4680
   taaccctcag catattgaag tgcagatcct tggcgatcac actggagaag ttgtacacct 4740
   ttatgaacgt gactgctcac tgcagcgtcg tcaccaaaaa gttgtcgaaa ttgcgccagc 4800
   acagcatttg gatccagaac tgcgtgatcg catttgtgcg gatgcagtaa agttctgccg 4860
   ctccattggt taccagggcg cgggaaccaa gggcgaattc ctctggataa tcatcgcggt 4920
   agttacgagc ggcgcgaatg caagggcgaa ttcgagctcg gtacccgggg atcctctaga 4980
   gtcgacctgc aggcatgcaa gcttggcgta atcatggtca tagctgtttc ctgtgtgaaa 5040
   ttgttatccg ctcacaattc cacacaacat acgagccgga agcataaagt gtaaagcctg 5100
   gggtgcctaa tgagtgagct aactcacatt aattgcgttg cgctcactgc ccgctttcca 5160
   gtcgggaaac ctgtcgtgcc agctgcatta atgaatcggc caacgcgcgg ggagaggcgg 5220
   tttgcgtatt gggcgctctt ccgcttcctc gctcactgac tcgctgcgct cggtcgttcg 5280
   gctgcggcga gcggtatcag ctcactcaaa ggcggtaata cggttatcca cagaatcagg 5340
   ggataacgca ggaaagaaca tgtgagcaaa aggccagcaa aaggccagga accgtaaaaa 5400
   ggccgcgttg ctggcgtttt tccataggct ccgcccccct gacgagcatc acaaaaatcg 5460
   acgctcaagt cagaggtggc gaaacccgac aggactataa agataccagg cgtttccccc 5520
   tggaagctcc ctcgtgcgct ctcctgttcc gaccctgccg cttaccggat acctgtccgc 5580
   ctttctccct tcgggaagcg tggcgctttc tcatagctca cgctgtaggt atctcagttc 5640
   ggtgtaggtc gttcgctcca agctgggctg tgtgcacgaa ccccccgttc agcccgaccg 5700
   ctgcgcctta tccggtaact atcgtcttga gtccaacccg gtaagacacg acttatcgcc 5760
   actggcagca gccactggta acaggattag cagagcgagg tatgtaggcg gtgctacaga 5820
   gttcttgaag tggtggccta actacggcta cactagaaga acagtatttg gtatctgcgc 5880
   tctgctgaag ccagttacct tcggaaaaag agttggtagc tcttgatccg gcaaacaaac 5940
   caccgctggt agcggtggtt tttttgtttg caagcagcag attacgcgca gaaaaaaagg 6000
   atctcaagaa gatcctttga tcttttctac ggggtctgac gctcagtgga acgaaaactc 6060
   acgttaaggg attttggtca tgagattatc aaaaaggatc ttcacctaga tccttttggg 6120
   gtgggcgaag aactccagca tgagatcccc gcgctggagg atcatccagc cctgatagaa 6180
   acagaagcca ctggagcacc tcaaaaacac catcatacac taaatcagta agttggcagc 6240
   atcacccgac gcactttgcg ccgaataaat acctgtgacg gaagatcact tcgcagaata 6300
   aataaatcct ggtgtccctg ttgataccgg gaagccctgg gccaactttt ggcgaaaatg 6360
   agacgttgat cggcacgtaa gaggttccaa ctttcaccat aatgaaataa gatcactacc 6420
   gggcgtattt tttgagttat cgagattttc aggagctgat agaaacagaa gccactggag 6480
   cacctcaaaa acaccatcat acactaaatc agtaagttgg cagcatcacc cgacgcactt 6540
   tgcgccgaat aaatacctgt gacggaagat cacttcgcag aataaataaa tcctggtgtc 6600
   cctgttgata ccgggaagcc ctgggccaac ttttggcgaa aatgagacgt tgatcggcac 6660
   gtaagaggtt ccaactttca ccataatgaa ataagatcac taccgggcgt attttttgag 6720
   ttatcgagat tttcaggagc tctttggcat cgtctctcgc ctgtcccctc agttcagtaa 6780
   tttcctgcat ttgcctgttt ccagtcggta gatattccac aaaacagcag ggaagcagcg 6840
   cttttccgct gcataaccct gcttcggggt cattatagcg attttttcgg tatatccatc 6900
   ctttttcgca cgatatacag gattttgcca aagggttcgt gtagactttc cttggtgtat 6960
   ccaacggcgt cagccgggca ggataggtga agtaggccca cccgcgagcg ggtgttcctt 7020
   cttcactgtc ccttattcgc acctggcggt gctcaacggg aatcctgctc tgcgaggctg 7080
   gccggctacc gccggcgtaa cagatgaggg caagcggatg gctgatgaaa ccaagccaac 7140
   caggaagggc agcccaccta tcaaggtgta ctgccttcca gacgaacgaa gagcgattga 7200
   ggaaaaggcg gcggcggccg gcatgagcct gtcggcctac ctgctggccg tcggccaggg 7260
   ctacaaaatc acgggcgtcg tggactatga gcacgtccgc gagggcgtcc cggaaaacga 7320
   ttccgaagcc caacctttca tagaaggcgg cggtggaatc gaaatctcgt gatggcaggt 7380
   tgggcgtcgc ttggtcggtc atttcgctcg gtacccatcg gcattttctt ttgcgttt 7438
<210> 15
   <211> 52
   <212> DNA
   <213> Corynebacterium glutamicum
<220>
   <223> SEQ_ID_15
<400> 15
   cccgggatcc gctagcggcg cgccggccgg cccggtgtga aataccgcac ag 52
<210> 16
   <211> 53
   <212> DNA
   <213> Corynebacterium glutamicum
<220>
   <223> SEQ_ID_16
<400> 16
   tctagactcg agcggccgcg gccggccttt aaattgaaga cgaaagggcc tcg 53
<210> 17
   <211> 47
   <212> DNA
   <213> Corynebacterium glutamicum
<220>
   <223> SEQ_ID_17
<400> 17
   gagatctaga cccggggatc cgctagcggg ctgctaaagg aagcgga 47
<210> 18
   <211> 38
   <212> DNA
   <213> Corynebacterium glutamicum
<220>
   <223> SEQ_ID_18
<400> 18
   gagaggcgcg ccgctagcgt gggcgaagaa ctccagca 38
<210> 19
   <211> 34
   <212> DNA
   <213> Corynebacterium glutamicum
<220>
   <223> SEQ_ID_19
<400> 19
   gagagggcgg ccgcgcaaag tcccgcttcg tgaa 34
<210> 20
   <211> 34
   <212> DNA
   <213> Corynebacterium glutamicum
<220>
   <223> SEQ_ID_20
<400> 20
   gagagggcgg ccgctcaagt cggtcaagcc acgc 34
<210> 21
   <211> 140
   <212> DNA
   <213> Corynebacterium glutamicum
<220>
   <223> SEQ_ID_21
<400> 21
   tcgaatttaa atctcgagag gcctgacgtc gggcccggta ccacgcgtca tatgactagt 60
   tcggacctag ggatatcgtc gacatcgatg ctcttctgcg ttaattaaca attgggatcc 120
   tctagacccg ggatttaaat 140
<210> 22
   <211> 140
   <212> DNA
   <213> Corynebacterium glutamicum
<220>
   <223> SEQ_ID_22
<400> 22
   gatcatttaa atcccgggtc tagaggatcc caattgttaa ttaacgcaga agagcatcga 60
   tgtcgacgat atccctaggt ccgaactagt catatgacgc gtggtaccgg gcccgacgtc 120
   aggcctctcg agatttaaat 140
<210> 23
   <211> 5091
   <212> DNA
   <213> Corynebacterium glutamicum
<220>
   <223> SEQ_ID_23_PCLIK5MCS
<220>
   <221> misc_feature
   <222> (469 )..(1260 )
   <223> KanR
<220>
   <221> misc_feature
   <222> (1527 )..(2387 )
   <223> Ori EC(pMB) complement
<220>
   <221> misc_feature
   <222> (2533 )..(3207 )
   <223> orf1
<220>
   <221> misc_feature
   <222> (3541 )..(4662 )
   <223> Rep Protein
<400> 23
   tcgatttaaa tctcgagagg cctgacgtcg ggcccggtac cacgcgtcat atgactagtt 60
   cggacctagg gatatcgtcg acatcgatgc tcttctgcgt taattaacaa ttgggatcct 120
   ctagacccgg gatttaaatc gctagcgggc tgctaaagga agcggaacac gtagaaagcc 180
   agtccgcaga aacggtgctg accccggatg aatgtcagct actgggctat ctggacaagg 240
   gaaaacgcaa gcgcaaagag aaagcaggta gcttgcagtg ggcttacatg gcgatagcta 300
   gactgggcgg ttttatggac agcaagcgaa ccggaattgc cagctggggc gccctctggt 360
   aaggttggga agccctgcaa agtaaactgg atggctttct tgccgccaag gatctgatgg 420
   cgcaggggat caagatctga tcaagagaca ggatgaggat cgtttcgcat gattgaacaa 480
   gatggattgc acgcaggttc tccggccgct tgggtggaga ggctattcgg ctatgactgg 540
   gcacaacaga caatcggctg ctctgatgcc gccgtgttcc ggctgtcagc gcaggggcgc 600
   ccggttcttt ttgtcaagac cgacctgtcc ggtgccctga atgaactgca ggacgaggca 660
   gcgcggctat cgtggctggc cacgacgggc gttccttgcg cagctgtgct cgacgttgtc 720
   actgaagcgg gaagggactg gctgctattg ggcgaagtgc cggggcagga tctcctgtca 780
   tctcaccttg ctcctgccga gaaagtatcc atcatggctg atgcaatgcg gcggctgcat 840
   acgcttgatc cggctacctg cccattcgac caccaagcga aacatcgcat cgagcgagca 900
   cgtactcgga tggaagccgg tcttgtcgat caggatgatc tggacgaaga gcatcagggg 960
   ctcgcgccag ccgaactgtt cgccaggctc aaggcgcgca tgcccgacgg cgaggatctc 1020
   gtcgtgaccc atggcgatgc ctgcttgccg aatatcatgg tggaaaatgg ccgcttttct 1080
   ggattcatcg actgtggccg gctgggtgtg gcggaccgct atcaggacat agcgttggct 1140
   acccgtgata ttgctgaaga gcttggcggc gaatgggctg accgcttcct cgtgctttac 1200
   ggtatcgccg ctcccgattc gcagcgcatc gccttctatc gccttcttga cgagttcttc 1260
   tgagcgggac tctggggttc gaaatgaccg accaagcgac gcccaacctg ccatcacgag 1320
   atttcgattc caccgccgcc ttctatgaaa ggttgggctt cggaatcgtt ttccgggacg 1380
   ccggctggat gatcctccag cgcggggatc tcatgctgga gttcttcgcc cacgctagcg 1440
   gcgcgccggc cggcccggtg tgaaataccg cacagatgcg taaggagaaa ataccgcatc 1500
   aggcgctctt ccgcttcctc gctcactgac tcgctgcgct cggtcgttcg gctgcggcga 1560
   gcggtatcag ctcactcaaa ggcggtaata cggttatcca cagaatcagg ggataacgca 1620
   ggaaagaaca tgtgagcaaa aggccagcaa aaggccagga accgtaaaaa ggccgcgttg 1680
   ctggcgtttt tccataggct ccgcccccct gacgagcatc acaaaaatcg acgctcaagt 1740
   cagaggtggc gaaacccgac aggactataa agataccagg cgtttccccc tggaagctcc 1800
   ctcgtgcgct ctcctgttcc gaccctgccg cttaccggat acctgtccgc ctttctccct 1860
   tcgggaagcg tggcgctttc tcatagctca cgctgtaggt atctcagttc ggtgtaggtc 1920
   gttcgctcca agctgggctg tgtgcacgaa ccccccgttc agcccgaccg ctgcgcctta 1980
   tccggtaact atcgtcttga gtccaacccg gtaagacacg acttatcgcc actggcagca 2040
   gccactggta acaggattag cagagcgagg tatgtaggcg gtgctacaga gttcttgaag 2100
   tggtggccta actacggcta cactagaagg acagtatttg gtatctgcgc tctgctgaag 2160
   ccagttacct tcggaaaaag agttggtagc tcttgatccg gcaaacaaac caccgctggt 2220
   agcggtggtt tttttgtttg caagcagcag attacgcgca gaaaaaaagg atctcaagaa 2280
   gatcctttga tcttttctac ggggtctgac gctcagtgga acgaaaactc acgttaaggg 2340
   attttggtca tgagattatc aaaaaggatc ttcacctaga tccttttaaa ggccggccgc 2400
   ggccgcgcaa agtcccgctt cgtgaaaatt ttcgtgccgc gtgattttcc gccaaaaact 2460
   ttaacgaacg ttcgttataa tggtgtcatg accttcacga cgaagtacta aaattggccc 2520
   gaatcatcag ctatggatct ctctgatgtc gcgctggagt ccgacgcgct cgatgctgcc 2580
   gtcgatttaa aaacggtgat cggatttttc cgagctctcg atacgacgga cgcgccagca 2640
   tcacgagact gggccagtgc cgcgagcgac ctagaaactc tcgtggcgga tcttgaggag 2700
   ctggctgacg agctgcgtgc tcggccagcg ccaggaggac gcacagtagt ggaggatgca 2760
   atcagttgcg cctactgcgg tggcctgatt cctccccggc ctgacccgcg aggacggcgc 2820
   gcaaaatatt gctcagatgc gtgtcgtgcc gcagccagcc gcgagcgcgc caacaaacgc 2880
   cacgccgagg agctggaggc ggctaggtcg caaatggcgc tggaagtgcg tcccccgagc 2940
   gaaattttgg ccatggtcgt cacagagctg gaagcggcag cgagaattat cgcgatcgtg 3000
   gcggtgcccg caggcatgac aaacatcgta aatgccgcgt ttcgtgtgcc gtggccgccc 3060
   aggacgtgtc agcgccgcca ccacctgcac cgaatcggca gcagcgtcgc gcgtcgaaaa 3120
   agcgcacagg cggcaagaag cgataagctg cacgaatacc tgaaaaatgt tgaacgcccc 3180
   gtgagcggta actcacaggg cgtcggctaa cccccagtcc aaacctggga gaaagcgctc 3240
   aaaaatgact ctagcggatt cacgagacat tgacacaccg gcctggaaat tttccgctga 3300
   tctgttcgac acccatcccg agctcgcgct gcgatcacgt ggctggacga gcgaagaccg 3360
   ccgcgaattc ctcgctcacc tgggcagaga aaatttccag ggcagcaaga cccgcgactt 3420
   cgccagcgct tggatcaaag acccggacac ggagaaacac agccgaagtt ataccgagtt 3480
   ggttcaaaat cgcttgcccg gtgccagtat gttgctctga cgcacgcgca gcacgcagcc 3540
   gtgcttgtcc tggacattga tgtgccgagc caccaggccg gcgggaaaat cgagcacgta 3600
   aaccccgagg tctacgcgat tttggagcgc tgggcacgcc tggaaaaagc gccagcttgg 3660
   atcggcgtga atccactgag cgggaaatgc cagctcatct ggctcattga tccggtgtat 3720
   gccgcagcag gcatgagcag cccgaatatg cgcctgctgg ctgcaacgac cgaggaaatg 3780
   acccgcgttt tcggcgctga ccaggctttt tcacataggc tgagccgtgg ccactgcact 3840
   ctccgacgat cccagccgta ccgctggcat gcccagcaca atcgcgtgga tcgcctagct 3900
   gatcttatgg aggttgctcg catgatctca ggcacagaaa aacctaaaaa acgctatgag 3960
   caggagtttt ctagcggacg ggcacgtatc gaagcggcaa gaaaagccac tgcggaagca 4020
   aaagcacttg ccacgcttga agcaagcctg ccgagcgccg ctgaagcgtc tggagagctg 4080
   atcgacggcg tccgtgtcct ctggactgct ccagggcgtg ccgcccgtga tgagacggct 4140
   tttcgccacg ctttgactgt gggataccag ttaaaagcgg ctggtgagcg cctaaaagac 4200
   accaagggtc atcgagccta cgagcgtgcc tacaccgtcg ctcaggcggt cggaggaggc 4260
   cgtgagcctg atctgccgcc ggactgtgac cgccagacgg attggccgcg acgtgtgcgc 4320
   ggctacgtcg ctaaaggcca gccagtcgtc cctgctcgtc agacagagac gcagagccag 4380
   ccgaggcgaa aagctctggc cactatggga agacgtggcg gtaaaaaggc cgcagaacgc 4440
   tggaaagacc caaacagtga gtacgcccga gcacagcgag aaaaactagc taagtccagt 4500
   caacgacaag ctaggaaagc taaaggaaat cgcttgacca ttgcaggttg gtttatgact 4560
   gttgagggag agactggctc gtggccgaca atcaatgaag ctatgtctga atttagcgtg 4620
   tcacgtcaga ccgtgaatag agcacttaag gtctgcgggc attgaacttc cacgaggacg 4680
   ccgaaagctt cccagtaaat gtgccatctc gtaggcagaa aacggttccc ccgtagggtc 4740
   tctctcttgg cctcctttct aggtcgggct gattgctctt gaagctctct aggggggctc 4800
   acaccatagg cagataacgt tccccaccgg ctcgcctcgt aagcgcacaa ggactgctcc 4860
   caaagatctt caaagccact gccgcgactg ccttcgcgaa gccttgcccc gcggaaattt 4920
   cctccaccga gttcgtgcac acccctatgc caagcttctt tcaccctaaa ttcgagagat 4980
   tggattctta ccgtggaaat tcttcgcaaa aatcgtcccc tgatcgccct tgcgacgttg 5040
   gcgtcggtgc cgctggttgc gcttggcttg accgacttga tcagcggccg c 5091
<210> 24
   <211> 28
   <212> DNA
   <213> Corynebacterium glutamicum
<220>
   <223> SEQ_ID_24
<400> 24
   gcgcggtacc tagactcacc ccagtgct 28
<210> 25
   <211> 30
   <212> DNA
   <213> Corynebacterium glutamicum
<220>
   <223> SEQ_ID_25
<400> 25
   ctctactagt ttagatgtag aactcgatgt 30
<210> 26
   <211> 6349
   <212> DNA
   <213> Corynebacterium glutamicum
<220>
   <223> SEQ_ID_26_PCLIK5MCS_PMETA_META
<220>
   <221> misc_feature
   <222> (42 )..(177 )
   <223> PmetA
<220>
   <221> misc_feature
   <222> (178 )..(1311 )
   <223> metA
<220>
   <221> misc_feature
   <222> (1727 )..(2518 )
   <223> KanR
<220>
   <221> misc_feature
   <222> (2785 )..(3645 )
   <223> orf1
<220>
   <221> misc_feature
   <222> (3791 )..(4465 )
   <223> Ori-Ec (pMB) complement
<220>
   <221> misc_feature
   <222> (4799 )..(5920 )
   <223> Rep Protein
<400> 26
   tcgatttaaa tctcgagagg cctgacgtcg ggcccggtac ctagactcac cccagtgctt 60
   aaagcgctgg gtttttcttt ttcagactcg tgagaatgca aactagacta gacagagctg 120
   tccatataca ctggacgaag ttttagtctt gtccacccag aacaggcggt tattttcatg 180
   cccaccctcg cgccttcagg tcaacttgaa atccaagcga tcggtgatgt ctccaccgaa 240
   gccggagcaa tcattacaaa cgctgaaatc gcctatcacc gctggggtga ataccgcgta 300
   gataaagaag gacgcagcaa tgtcgttctc atcgaacacg ccctcactgg agattccaac 360
   gcagccgatt ggtgggctga cttgctcggt cccggcaaag ccatcaacac tgatatttac 420
   tgcgtgatct gtaccaacgt catcggtggt tgcaacggtt ccaccggacc tggctccatg 480
   catccagatg gaaatttctg gggtaatcgc ttccccgcca cgtccattcg tgatcaggta 540
   aacgccgaaa aacaattcct cgacgcactc ggcatcacca cggtcgccgc agtacttggt 600
   ggttccatgg gtggtgcccg caccctagag tgggccgcaa tgtacccaga aactgttggc 660
   gcagctgctg ttcttgcagt ttctgcacgc gccagcgcct ggcaaatcgg cattcaatcc 720
   gcccaaatta aggcgattga aaacgaccac cactggcacg aaggcaacta ctacgaatcc 780
   ggctgcaacc cagccaccgg actcggcgcc gcccgacgca tcgcccacct cacctaccgt 840
   ggcgaactag aaatcgacga acgcttcggc accaaagccc aaaagaacga aaacccactc 900
   ggtccctacc gcaagcccga ccagcgcttc gccgtggaat cctacttgga ctaccaagca 960
   gacaagctag tacagcgttt cgacgccggc tcctacgtct tgctcaccga cgccctcaac 1020
   cgccacgaca ttggtcgcga ccgcggaggc ctcaacaagg cactcgaatc catcaaagtt 1080
   ccagtccttg tcgcaggcgt agataccgat attttgtacc cctaccacca gcaagaacac 1140
   ctctccagaa acctgggaaa tctactggca atggcaaaaa tcgtatcccc tgtcggccac 1200
   gatgctttcc tcaccgaaag ccgccaaatg gatcgcatcg tgaggaactt cttcagcctc 1260
   atctccccag acgaagacaa cccttcgacc tacatcgagt tctacatcta aactagttcg 1320
   gacctaggga tatcgtcgac atcgatgctc ttctgcgtta attaacaatt gggatcctct 1380
   agacccggga tttaaatcgc tagcgggctg ctaaaggaag cggaacacgt agaaagccag 1440
   tccgcagaaa cggtgctgac cccggatgaa tgtcagctac tgggctatct ggacaaggga 1500
   aaacgcaagc gcaaagagaa agcaggtagc ttgcagtggg cttacatggc gatagctaga 1560
   ctgggcggtt ttatggacag caagcgaacc ggaattgcca gctggggcgc cctctggtaa 1620
   ggttgggaag ccctgcaaag taaactggat ggctttcttg ccgccaagga tctgatggcg 1680
   caggggatca agatctgatc aagagacagg atgaggatcg tttcgcatga ttgaacaaga 1740
   tggattgcac gcaggttctc cggccgcttg ggtggagagg ctattcggct atgactgggc 1800
   acaacagaca atcggctgct ctgatgccgc cgtgttccgg ctgtcagcgc aggggcgccc 1860
   ggttcttttt gtcaagaccg acctgtccgg tgccctgaat gaactgcagg acgaggcagc 1920
   gcggctatcg tggctggcca cgacgggcgt tccttgcgca gctgtgctcg acgttgtcac 1980
   tgaagcggga agggactggc tgctattggg cgaagtgccg gggcaggatc tcctgtcatc 2040
   tcaccttgct cctgccgaga aagtatccat catggctgat gcaatgcggc ggctgcatac 2100
   gcttgatccg gctacctgcc cattcgacca ccaagcgaaa catcgcatcg agcgagcacg 2160
   tactcggatg gaagccggtc ttgtcgatca ggatgatctg gacgaagagc atcaggggct 2220
   cgcgccagcc gaactgttcg ccaggctcaa ggcgcgcatg cccgacggcg aggatctcgt 2280
   cgtgacccat ggcgatgcct gcttgccgaa tatcatggtg gaaaatggcc gcttttctgg 2340
   attcatcgac tgtggccggc tgggtgtggc ggaccgctat caggacatag cgttggctac 2400
   ccgtgatatt gctgaagagc ttggcggcga atgggctgac cgcttcctcg tgctttacgg 2460
   tatcgccgct cccgattcgc agcgcatcgc cttctatcgc cttcttgacg agttcttctg 2520
   agcgggactc tggggttcga aatgaccgac caagcgacgc ccaacctgcc atcacgagat 2580
   ttcgattcca ccgccgcctt ctatgaaagg ttgggcttcg gaatcgtttt ccgggacgcc 2640
   ggctggatga tcctccagcg cggggatctc atgctggagt tcttcgccca cgctagcggc 2700
   gcgccggccg gcccggtgtg aaataccgca cagatgcgta aggagaaaat accgcatcag 2760
   gcgctcttcc gcttcctcgc tcactgactc gctgcgctcg gtcgttcggc tgcggcgagc 2820
   ggtatcagct cactcaaagg cggtaatacg gttatccaca gaatcagggg ataacgcagg 2880
   aaagaacatg tgagcaaaag gccagcaaaa ggccaggaac cgtaaaaagg ccgcgttgct 2940
   ggcgtttttc cataggctcc gcccccctga cgagcatcac aaaaatcgac gctcaagtca 3000
   gaggtggcga aacccgacag gactataaag ataccaggcg tttccccctg gaagctccct 3060
   cgtgcgctct cctgttccga ccctgccgct taccggatac ctgtccgcct ttctcccttc 3120
   gggaagcgtg gcgctttctc atagctcacg ctgtaggtat ctcagttcgg tgtaggtcgt 3180
   tcgctccaag ctgggctgtg tgcacgaacc ccccgttcag cccgaccgct gcgccttatc 3240
   cggtaactat cgtcttgagt ccaacccggt aagacacgac ttatcgccac tggcagcagc 3300
   cactggtaac aggattagca gagcgaggta tgtaggcggt gctacagagt tcttgaagtg 3360
   gtggcctaac tacggctaca ctagaaggac agtatttggt atctgcgctc tgctgaagcc 3420
   agttaccttc ggaaaaagag ttggtagctc ttgatccggc aaacaaacca ccgctggtag 3480
   cggtggtttt tttgtttgca agcagcagat tacgcgcaga aaaaaaggat ctcaagaaga 3540
   tcctttgatc ttttctacgg ggtctgacgc tcagtggaac gaaaactcac gttaagggat 3600
   tttggtcatg agattatcaa aaaggatctt cacctagatc cttttaaagg ccggccgcgg 3660
   ccgcgcaaag tcccgcttcg tgaaaatttt cgtgccgcgt gattttccgc caaaaacttt 3720
   aacgaacgtt cgttataatg gtgtcatgac cttcacgacg aagtactaaa attggcccga 3780
   atcatcagct atggatctct ctgatgtcgc gctggagtcc gacgcgctcg atgctgccgt 3840
   cgatttaaaa acggtgatcg gatttttccg agctctcgat acgacggacg cgccagcatc 3900
   acgagactgg gccagtgccg cgagcgacct agaaactctc gtggcggatc ttgaggagct 3960
   ggctgacgag ctgcgtgctc ggccagcgcc aggaggacgc acagtagtgg aggatgcaat 4020
   cagttgcgcc tactgcggtg gcctgattcc tccccggcct gacccgcgag gacggcgcgc 4080
   aaaatattgc tcagatgcgt gtcgtgccgc agccagccgc gagcgcgcca acaaacgcca 4140
   cgccgaggag ctggaggcgg ctaggtcgca aatggcgctg gaagtgcgtc ccccgagcga 4200
   aattttggcc atggtcgtca cagagctgga agcggcagcg agaattatcg cgatcgtggc 4260
   ggtgcccgca ggcatgacaa acatcgtaaa tgccgcgttt cgtgtgccgt ggccgcccag 4320
   gacgtgtcag cgccgccacc acctgcaccg aatcggcagc agcgtcgcgc gtcgaaaaag 4380
   cgcacaggcg gcaagaagcg ataagctgca cgaatacctg aaaaatgttg aacgccccgt 4440
   gagcggtaac tcacagggcg tcggctaacc cccagtccaa acctgggaga aagcgctcaa 4500
   aaatgactct agcggattca cgagacattg acacaccggc ctggaaattt tccgctgatc 4560
   tgttcgacac ccatcccgag ctcgcgctgc gatcacgtgg ctggacgagc gaagaccgcc 4620
   gcgaattcct cgctcacctg ggcagagaaa atttccaggg cagcaagacc cgcgacttcg 4680
   ccagcgcttg gatcaaagac ccggacacgg agaaacacag ccgaagttat accgagttgg 4740
   ttcaaaatcg cttgcccggt gccagtatgt tgctctgacg cacgcgcagc acgcagccgt 4800
   gcttgtcctg gacattgatg tgccgagcca ccaggccggc gggaaaatcg agcacgtaaa 4860
   ccccgaggtc tacgcgattt tggagcgctg ggcacgcctg gaaaaagcgc cagcttggat 4920
   cggcgtgaat ccactgagcg ggaaatgcca gctcatctgg ctcattgatc cggtgtatgc 4980
   cgcagcaggc atgagcagcc cgaatatgcg cctgctggct gcaacgaccg aggaaatgac 5040
   ccgcgttttc ggcgctgacc aggctttttc acataggctg agccgtggcc actgcactct 5100
   ccgacgatcc cagccgtacc gctggcatgc ccagcacaat cgcgtggatc gcctagctga 5160
   tcttatggag gttgctcgca tgatctcagg cacagaaaaa cctaaaaaac gctatgagca 5220
   ggagttttct agcggacggg cacgtatcga agcggcaaga aaagccactg cggaagcaaa 5280
   agcacttgcc acgcttgaag caagcctgcc gagcgccgct gaagcgtctg gagagctgat 5340
   cgacggcgtc cgtgtcctct ggactgctcc agggcgtgcc gcccgtgatg agacggcttt 5400
   tcgccacgct ttgactgtgg gataccagtt aaaagcggct ggtgagcgcc taaaagacac 5460
   caagggtcat cgagcctacg agcgtgccta caccgtcgct caggcggtcg gaggaggccg 5520
   tgagcctgat ctgccgccgg actgtgaccg ccagacggat tggccgcgac gtgtgcgcgg 5580
   ctacgtcgct aaaggccagc cagtcgtccc tgctcgtcag acagagacgc agagccagcc 5640
   gaggcgaaaa gctctggcca ctatgggaag acgtggcggt aaaaaggccg cagaacgctg 5700
   gaaagaccca aacagtgagt acgcccgagc acagcgagaa aaactagcta agtccagtca 5760
   acgacaagct aggaaagcta aaggaaatcg cttgaccatt gcaggttggt ttatgactgt 5820
   tgagggagag actggctcgt ggccgacaat caatgaagct atgtctgaat ttagcgtgtc 5880
   acgtcagacc gtgaatagag cacttaaggt ctgcgggcat tgaacttcca cgaggacgcc 5940
   gaaagcttcc cagtaaatgt gccatctcgt aggcagaaaa cggttccccc gtagggtctc 6000
   tctcttggcc tcctttctag gtcgggctga ttgctcttga agctctctag gggggctcac 6060
   accataggca gataacgttc cccaccggct cgcctcgtaa gcgcacaagg actgctccca 6120
   aagatcttca aagccactgc cgcgactgcc ttcgcgaagc cttgccccgc ggaaatttcc 6180
   tccaccgagt tcgtgcacac ccctatgcca agcttctttc accctaaatt cgagagattg 6240
   gattcttacc gtggaaattc ttcgcaaaaa tcgtcccctg atcgcccttg cgacgttggc 6300
   gtcggtgccg ctggttgcgc ttggcttgac cgacttgatc agcggccgc              6349
<210> 27
   <211> 30
   <212> DNA
   <213> Corynebacterium glutamicum
<220>
   <223> SEQ_ID_27
<400> 27
   gagactcgag cggcttaaag tttggctgcc 30
<210> 28
   <211> 41
   <212> DNA
   <213> Corynebacterium glutamicum
<220>
   <223> SEQ_ID_28
<400> 28
   cctgaaggcg cgagggtggg catgatgaat ccctccatga g 41
<210> 29
   <211> 19
   <212> DNA
   <213> Corynebacterium glutamicum
<220>
   <223> SEQ_ID_29
<400> 29
   cccaccctcg cgccttcag 19
<210> 30
   <211> 18
   <212> DNA
   <213> Corynebacterium glutamicum
<220>
   <223> SEQ_ID_30
<400> 30
   ctgggtacat tgcggccc 18
<210> 31
   <211> 6372
   <212> DNA
   <213> Corynebacterium glutamicum
<220>
   <223> SEQ_ID_31_PCLIK5MCS_PGROESMETA
<220>
   <221> misc_feature
   <222> (3 )..(179 )
   <223> Pgro
<220>
   <221> misc_feature
   <222> (180 )..(1313 )
   <223> metA
<220>
   <221> misc_feature
   <222> (1735 )..(2526 )
   <223> KanR
<220>
   <221> misc_feature
   <222> (2793 )..(3653 )
   <223> Ori-Ec (pMB) complement
<220>
   <221> misc_feature
   <222> (3799 )..(4473 )
   <223> Orf1
<220>
   <221> misc_feature
   <222> (4807 )..(5928 )
   <223> Rep Protein
<400> 31
   agcggcttaa agtttggctg ccatgtgaat ttttagcacc ctcaacagtt gagtgctggc 60
   actctcgggg gtagagtgcc aaataggttg tttgacacac agttgttcac ccgcgacgac 120
   ggctgtgctg gaaacccaca accggcacac acaaaatttt tctcatggag ggattcatca 180
   tgcccaccct cgcgccttca ggtcaacttg aaatccaagc gatcggtgat gtctccaccg 240
   aagccggagc aatcattaca aacgctgaaa tcgcctatca ccgctggggt gaataccgcg 300
   tagataaaga aggacgcagc aatgtcgttc tcatcgaaca cgccctcact ggagattcca 360
   acgcagccga ttggtgggct gacttgctcg gtcccggcaa agccatcaac actgatattt 420
   actgcgtgat ctgtaccaac gtcatcggtg gttgcaacgg ttccaccgga cctggctcca 480
   tgcatccaga tggaaatttc tggggtaatc gcttccccgc cacgtccatt cgtgatcagg 540
   taaacgccga aaaacaattc ctcgacgcac tcggcatcac cacggtcgcc gcagtacttg 600
   gtggttccat gggtggtgcc cgcaccctag agtgggccgc aatgtaccca gaaactgttg 660
   gcgcagctgc tgttcttgca gtttctgcac gcgccagcgc ctggcaaatc ggcattcaat 720
   ccgcccaaat taaggcgatt gaaaacgacc accactggca cgaaggcaac tactacgaat 780
   ccggctgcaa cccagccacc ggactcggcg ccgcccgacg catcgcccac ctcacctacc 840
   gtggcgaact agaaatcgac gaacgcttcg gcaccaaagc ccaaaagaac gaaaacccac 900
   tcggtcccta ccgcaagccc gaccagcgct tcgccgtgga atcctacttg gactaccaag 960
   cagacaagct agtacagcgt ttcgacgccg gctcctacgt cttgctcacc gacgccctca 1020
   accgccacga cattggtcgc gaccgcggag gcctcaacaa ggcactcgaa tccatcaaag 1080
   ttccagtcct tgtcgcaggc gtagataccg atattttgta cccctaccac cagcaagaac 1140
   acctctccag aaacctggga aatctactgg caatggcaaa aatcgtatcc cctgtcggcc 1200
   acgatgcttt cctcaccgaa agccgccaaa tggatcgcat cgtgaggaac ttcttcagcc 1260
   tcatctcccc agacgaagac aacccttcga cctacatcga gttctacatc taacatatga 1320
   ctagttcgga cctagggata tcgtcgacat cgatgctctt ctgcgttaat taacaattgg 1380
   gatcctctag acccgggatt taaatcgcta gcgggctgct aaaggaagcg gaacacgtag 1440
   aaagccagtc cgcagaaacg gtgctgaccc cggatgaatg tcagctactg ggctatctgg 1500
   acaagggaaa acgcaagcgc aaagagaaag caggtagctt gcagtgggct tacatggcga 1560
   tagctagact gggcggtttt atggacagca agcgaaccgg aattgccagc tggggcgccc 1620
   tctggtaagg ttgggaagcc ctgcaaagta aactggatgg ctttcttgcc gccaaggatc 1680
   tgatggcgca ggggatcaag atctgatcaa gagacaggat gaggatcgtt tcgcatgatt 1740
   gaacaagatg gattgcacgc aggttctccg gccgcttggg tggagaggct attcggctat 1800
   gactgggcac aacagacaat cggctgctct gatgccgccg tgttccggct gtcagcgcag 1860
   gggcgcccgg ttctttttgt caagaccgac ctgtccggtg ccctgaatga actgcaggac 1920
   gaggcagcgc ggctatcgtg gctggccacg acgggcgttc cttgcgcagc tgtgctcgac 1980
   gttgtcactg aagcgggaag ggactggctg ctattgggcg aagtgccggg gcaggatctc 2040
   ctgtcatctc accttgctcc tgccgagaaa gtatccatca tggctgatgc aatgcggcgg 2100
   ctgcatacgc ttgatccggc tacctgccca ttcgaccacc aagcgaaaca tcgcatcgag 2160
   cgagcacgta ctcggatgga agccggtctt gtcgatcagg atgatctgga cgaagagcat 2220
   caggggctcg cgccagccga actgttcgcc aggctcaagg cgcgcatgcc cgacggcgag 2280
   gatctcgtcg tgacccatgg cgatgcctgc ttgccgaata tcatggtgga aaatggccgc 2340
   ttttctggat tcatcgactg tggccggctg ggtgtggcgg accgctatca ggacatagcg 2400
   ttggctaccc gtgatattgc tgaagagctt ggcggcgaat gggctgaccg cttcctcgtg 2460
   ctttacggta tcgccgctcc cgattcgcag cgcatcgcct tctatcgcct tcttgacgag 2520
   ttcttctgag cgggactctg gggttcgaaa tgaccgacca agcgacgccc aacctgccat 2580
   cacgagattt cgattccacc gccgccttct atgaaaggtt gggcttcgga atcgttttcc 2640
   gggacgccgg ctggatgatc ctccagcgcg gggatctcat gctggagttc ttcgcccacg 2700
   ctagcggcgc gccggccggc ccggtgtgaa ataccgcaca gatgcgtaag gagaaaatac 2760
   cgcatcaggc gctcttccgc ttcctcgctc actgactcgc tgcgctcggt cgttcggctg 2820
   cggcgagcgg tatcagctca ctcaaaggcg gtaatacggt tatccacaga atcaggggat 2880
   aacgcaggaa agaacatgtg agcaaaaggc cagcaaaagg ccaggaaccg taaaaaggcc 2940
   gcgttgctgg cgtttttcca taggctccgc ccccctgacg agcatcacaa aaatcgacgc 3000
   tcaagtcaga ggtggcgaaa cccgacagga ctataaagat accaggcgtt tccccctgga 3060
   agctccctcg tgcgctctcc tgttccgacc ctgccgctta ccggatacct gtccgccttt 3120
   ctcccttcgg gaagcgtggc gctttctcat agctcacgct gtaggtatct cagttcggtg 3180
   taggtcgttc gctccaagct gggctgtgtg cacgaacccc ccgttcagcc cgaccgctgc 3240
   gccttatccg gtaactatcg tcttgagtcc aacccggtaa gacacgactt atcgccactg 3300
   gcagcagcca ctggtaacag gattagcaga gcgaggtatg taggcggtgc tacagagttc 3360
   ttgaagtggt ggcctaacta cggctacact agaaggacag tatttggtat ctgcgctctg 3420
   ctgaagccag ttaccttcgg aaaaagagtt ggtagctctt gatccggcaa acaaaccacc 3480
   gctggtagcg gtggtttttt tgtttgcaag cagcagatta cgcgcagaaa aaaaggatct 3540
   caagaagatc ctttgatctt ttctacgggg tctgacgctc agtggaacga aaactcacgt 3600
   taagggattt tggtcatgag attatcaaaa aggatcttca cctagatcct tttaaaggcc 3660
   ggccgcggcc gcgcaaagtc ccgcttcgtg aaaattttcg tgccgcgtga ttttccgcca 3720
   aaaactttaa cgaacgttcg ttataatggt gtcatgacct tcacgacgaa gtactaaaat 3780
   tggcccgaat catcagctat ggatctctct gatgtcgcgc tggagtccga cgcgctcgat 3840
   gctgccgtcg atttaaaaac ggtgatcgga tttttccgag ctctcgatac gacggacgcg 3900
   ccagcatcac gagactgggc cagtgccgcg agcgacctag aaactctcgt ggcggatctt 3960
   gaggagctgg ctgacgagct gcgtgctcgg ccagcgccag gaggacgcac agtagtggag 4020
   gatgcaatca gttgcgccta ctgcggtggc ctgattcctc cccggcctga cccgcgagga 4080
   cggcgcgcaa aatattgctc agatgcgtgt cgtgccgcag ccagccgcga gcgcgccaac 4140
   aaacgccacg ccgaggagct ggaggcggct aggtcgcaaa tggcgctgga agtgcgtccc 4200
   ccgagcgaaa ttttggccat ggtcgtcaca gagctggaag cggcagcgag aattatcgcg 4260
   atcgtggcgg tgcccgcagg catgacaaac atcgtaaatg ccgcgtttcg tgtgccgtgg 4320
   ccgcccagga cgtgtcagcg ccgccaccac ctgcaccgaa tcggcagcag cgtcgcgcgt 4380
   cgaaaaagcg cacaggcggc aagaagcgat aagctgcacg aatacctgaa aaatgttgaa 4440
   cgccccgtga gcggtaactc acagggcgtc ggctaacccc cagtccaaac ctgggagaaa 4500
   gcgctcaaaa atgactctag cggattcacg agacattgac acaccggcct ggaaattttc 4560
   cgctgatctg ttcgacaccc atcccgagct cgcgctgcga tcacgtggct ggacgagcga 4620
   agaccgccgc gaattcctcg ctcacctggg cagagaaaat ttccagggca gcaagacccg 4680
   cgacttcgcc agcgcttgga tcaaagaccc ggacacggag aaacacagcc gaagttatac 4740
   cgagttggtt caaaatcgct tgcccggtgc cagtatgttg ctctgacgca cgcgcagcac 4800
   gcagccgtgc ttgtcctgga cattgatgtg ccgagccacc aggccggcgg gaaaatcgag 4860
   cacgtaaacc ccgaggtcta cgcgattttg gagcgctggg cacgcctgga aaaagcgcca 4920
   gcttggatcg gcgtgaatcc actgagcggg aaatgccagc tcatctggct cattgatccg 4980
   gtgtatgccg cagcaggcat gagcagcccg aatatgcgcc tgctggctgc aacgaccgag 5040
   gaaatgaccc gcgttttcgg cgctgaccag gctttttcac ataggctgag ccgtggccac 5100
   tgcactctcc gacgatccca gccgtaccgc tggcatgccc agcacaatcg cgtggatcgc 5160
   ctagctgatc ttatggaggt tgctcgcatg atctcaggca cagaaaaacc taaaaaacgc 5220
   tatgagcagg agttttctag cggacgggca cgtatcgaag cggcaagaaa agccactgcg 5280
   gaagcaaaag cacttgccac gcttgaagca agcctgccga gcgccgctga agcgtctgga 5340
   gagctgatcg acggcgtccg tgtcctctgg actgctccag ggcgtgccgc ccgtgatgag 5400
   acggcttttc gccacgcttt gactgtggga taccagttaa aagcggctgg tgagcgccta 5460
   aaagacacca agggtcatcg agcctacgag cgtgcctaca ccgtcgctca ggcggtcgga 5520
   ggaggccgtg agcctgatct gccgccggac tgtgaccgcc agacggattg gccgcgacgt 5580
   gtgcgcggct acgtcgctaa aggccagcca gtcgtccctg ctcgtcagac agagacgcag 5640
   agccagccga ggcgaaaagc tctggccact atgggaagac gtggcggtaa aaaggccgca 5700
   gaacgctgga aagacccaaa cagtgagtac gcccgagcac agcgagaaaa actagctaag 5760
   tccagtcaac gacaagctag gaaagctaaa ggaaatcgct tgaccattgc aggttggttt 5820
   atgactgttg agggagagac tggctcgtgg ccgacaatca atgaagctat gtctgaattt 5880
   agcgtgtcac gtcagaccgt gaatagagca cttaaggtct gcgggcattg aacttccacg 5940
   aggacgccga aagcttccca gtaaatgtgc catctcgtag gcagaaaacg gttcccccgt 6000
   agggtctctc tcttggcctc ctttctaggt cgggctgatt gctcttgaag ctctctaggg 6060
   gggctcacac cataggcaga taacgttccc caccggctcg cctcgtaagc gcacaaggac 6120
   tgctcccaaa gatcttcaaa gccactgccg cgactgcctt cgcgaagcct tgccccgcgg 6180
   aaatttcctc caccgagttc gtgcacaccc ctatgccaag cttctttcac cctaaattcg 6240
   agagattgga ttcttaccgt ggaaattctt cgcaaaaatc gtcccctgat cgcccttgcg 6300
   acgttggcgt cggtgccgct ggttgcgctt ggcttgaccg acttgatcag cggccgctcg 6360
   atttaaatct cg 6372
<210> 32
   <211> 29
   <212> DNA
   <213> Corynebacterium glutamicum
<220>
   <223> SEQ_ID_32
<400> 32
   ggatctagag ttctgtgaaa aacaccgtg 29
<210> 33
   <211> 29
   <212> DNA
   <213> Corynebacterium glutamicum
<220>
   <223> SEQ_ID_33
<400> 33
   gcgactagtg ccccacaaat aaaaaacac 29
<210> 34
   <211> 5156
   <212> DNA
   <213> Corynebacterium glutamicum
<220>
   <223> SEQ_ID_34
<220>
   <221> misc_feature
   <222> (121 )..(190 )
   <223> GroEL Terminator
<220>
   <221> misc_feature
   <222> (534 )..(1325 )
   <223> KanR
<220>
   <221> misc_feature
   <222> (1592 )..(2452 )
   <223> ori-EC(pMB) complement
<220>
   <221> misc_feature
   <222> (2598 )..(3272 )
   <223> Orf1
<220>
   <221> misc_feature
   <222> (3606 )..(4727 )
   <223> Rep Protein
<400> 34
   tcgatttaaa tctcgagagg cctgacgtcg ggcccggtac cacgcgtcat atgactagtt 60
   cggacctagg gatatcgtcg acatcgatgc tcttctgcgt taattaacaa ttgggatcct 120
   ctagagttct gtgaaaaaca ccgtggggca gtttctgctt cgcggtgttt tttatttgtg 180
   gggcactaga cccgggattt aaatcgctag cgggctgcta aaggaagcgg aacacgtaga 240
   aagccagtcc gcagaaacgg tgctgacccc ggatgaatgt cagctactgg gctatctgga 300
   caagggaaaa cgcaagcgca aagagaaagc aggtagcttg cagtgggctt acatggcgat 360
   agctagactg ggcggtttta tggacagcaa gcgaaccgga attgccagct ggggcgccct 420
   ctggtaaggt tgggaagccc tgcaaagtaa actggatggc tttcttgccg ccaaggatct 480
   gatggcgcag gggatcaaga tctgatcaag agacaggatg aggatcgttt cgcatgattg 540
   aacaagatgg attgcacgca ggttctccgg ccgcttgggt ggagaggcta ttcggctatg 600
   actgggcaca acagacaatc ggctgctctg atgccgccgt gttccggctg tcagcgcagg 660
   ggcgcccggt tctttttgtc aagaccgacc tgtccggtgc cctgaatgaa ctgcaggacg 720
   aggcagcgcg gctatcgtgg ctggccacga cgggcgttcc ttgcgcagct gtgctcgacg 780
   ttgtcactga agcgggaagg gactggctgc tattgggcga agtgccgggg caggatctcc 840
   tgtcatctca ccttgctcct gccgagaaag tatccatcat ggctgatgca atgcggcggc 900
   tgcatacgct tgatccggct acctgcccat tcgaccacca agcgaaacat cgcatcgagc 960
   gagcacgtac tcggatggaa gccggtcttg tcgatcagga tgatctggac gaagagcatc 1020
   aggggctcgc gccagccgaa ctgttcgcca ggctcaaggc gcgcatgccc gacggcgagg 1080
   atctcgtcgt gacccatggc gatgcctgct tgccgaatat catggtggaa aatggccgct 1140
   tttctggatt catcgactgt ggccggctgg gtgtggcgga ccgctatcag gacatagcgt 1200
   tggctacccg tgatattgct gaagagcttg gcggcgaatg ggctgaccgc ttcctcgtgc 1260
   tttacggtat cgccgctccc gattcgcagc gcatcgcctt ctatcgcctt cttgacgagt 1320
   tcttctgagc gggactctgg ggttcgaaat gaccgaccaa gcgacgccca acctgccatc 1380
   acgagatttc gattccaccg ccgccttcta tgaaaggttg ggcttcggaa tcgttttccg 1440
   ggacgccggc tggatgatcc tccagcgcgg ggatctcatg ctggagttct tcgcccacgc 1500
   tagcggcgcg ccggccggcc cggtgtgaaa taccgcacag atgcgtaagg agaaaatacc 1560
   gcatcaggcg ctcttccgct tcctcgctca ctgactcgct gcgctcggtc gttcggctgc 1620
   ggcgagcggt atcagctcac tcaaaggcgg taatacggtt atccacagaa tcaggggata 1680
   acgcaggaaa gaacatgtga gcaaaaggcc agcaaaaggc caggaaccgt aaaaaggccg 1740
   cgttgctggc gtttttccat aggctccgcc cccctgacga gcatcacaaa aatcgacgct 1800
   caagtcagag gtggcgaaac ccgacaggac tataaagata ccaggcgttt ccccctggaa 1860
   gctccctcgt gcgctctcct gttccgaccc tgccgcttac cggatacctg tccgcctttc 1920
   tcccttcggg aagcgtggcg ctttctcata gctcacgctg taggtatctc agttcggtgt 1980
   aggtcgttcg ctccaagctg ggctgtgtgc acgaaccccc cgttcagccc gaccgctgcg 2040
   ccttatccgg taactatcgt cttgagtcca acccggtaag acacgactta tcgccactgg 2100
   cagcagccac tggtaacagg attagcagag cgaggtatgt aggcggtgct acagagttct 2160
   tgaagtggtg gcctaactac ggctacacta gaaggacagt atttggtatc tgcgctctgc 2220
   tgaagccagt taccttcgga aaaagagttg gtagctcttg atccggcaaa caaaccaccg 2280
   ctggtagcgg tggttttttt gtttgcaagc agcagattac gcgcagaaaa aaaggatctc 2340
   aagaagatcc tttgatcttt tctacggggt ctgacgctca gtggaacgaa aactcacgtt 2400
   aagggatttt ggtcatgaga ttatcaaaaa ggatcttcac ctagatcctt ttaaaggccg 2460
   gccgcggccg cgcaaagtcc cgcttcgtga aaattttcgt gccgcgtgat tttccgccaa 2520
   aaactttaac gaacgttcgt tataatggtg tcatgacctt cacgacgaag tactaaaatt 2580
   ggcccgaatc atcagctatg gatctctctg atgtcgcgct ggagtccgac gcgctcgatg 2640
   ctgccgtcga tttaaaaacg gtgatcggat ttttccgagc tctcgatacg acggacgcgc 2700
   cagcatcacg agactgggcc agtgccgcga gcgacctaga aactctcgtg gcggatcttg 2760
   aggagctggc tgacgagctg cgtgctcggc cagcgccagg aggacgcaca gtagtggagg 2820
   atgcaatcag ttgcgcctac tgcggtggcc tgattcctcc ccggcctgac ccgcgaggac 2880
   ggcgcgcaaa atattgctca gatgcgtgtc gtgccgcagc cagccgcgag cgcgccaaca 2940
   aacgccacgc cgaggagctg gaggcggcta ggtcgcaaat ggcgctggaa gtgcgtcccc 3000
   cgagcgaaat tttggccatg gtcgtcacag agctggaagc ggcagcgaga attatcgcga 3060
   tcgtggcggt gcccgcaggc atgacaaaca tcgtaaatgc cgcgtttcgt gtgccgtggc 3120
   cgcccaggac gtgtcagcgc cgccaccacc tgcaccgaat cggcagcagc gtcgcgcgtc 3180
   gaaaaagcgc acaggcggca agaagcgata agctgcacga atacctgaaa aatgttgaac 3240
   gccccgtgag cggtaactca cagggcgtcg gctaaccccc agtccaaacc tgggagaaag 3300
   cgctcaaaaa tgactctagc ggattcacga gacattgaca caccggcctg gaaattttcc 3360
   gctgatctgt tcgacaccca tcccgagctc gcgctgcgat cacgtggctg gacgagcgaa 3420
   gaccgccgcg aattcctcgc tcacctgggc agagaaaatt tccagggcag caagacccgc 3480
   gacttcgcca gcgcttggat caaagacccg gacacggaga aacacagccg aagttatacc 3540
   gagttggttc aaaatcgctt gcccggtgcc agtatgttgc tctgacgcac gcgcagcacg 3600
   cagccgtgct tgtcctggac attgatgtgc cgagccacca ggccggcggg aaaatcgagc 3660
   acgtaaaccc cgaggtctac gcgattttgg agcgctgggc acgcctggaa aaagcgccag 3720
   cttggatcgg cgtgaatcca ctgagcggga aatgccagct catctggctc attgatccgg 3780
   tgtatgccgc agcaggcatg agcagcccga atatgcgcct gctggctgca acgaccgagg 3840
   aaatgacccg cgttttcggc gctgaccagg ctttttcaca taggctgagc cgtggccact 3900
   gcactctccg acgatcccag ccgtaccgct ggcatgccca gcacaatcgc gtggatcgcc 3960
   tagctgatct tatggaggtt gctcgcatga tctcaggcac agaaaaacct aaaaaacgct 4020
   atgagcagga gttttctagc ggacgggcac gtatcgaagc ggcaagaaaa gccactgcgg 4080
   aagcaaaagc acttgccacg cttgaagcaa gcctgccgag cgccgctgaa gcgtctggag 4140
   agctgatcga cggcgtccgt gtcctctgga ctgctccagg gcgtgccgcc cgtgatgaga 4200
   cggcttttcg ccacgctttg actgtgggat accagttaaa agcggctggt gagcgcctaa 4260
   aagacaccaa gggtcatcga gcctacgagc gtgcctacac cgtcgctcag gcggtcggag 4320
   gaggccgtga gcctgatctg ccgccggact gtgaccgcca gacggattgg ccgcgacgtg 4380
   tgcgcggcta cgtcgctaaa ggccagccag tcgtccctgc tcgtcagaca gagacgcaga 4440
   gccagccgag gcgaaaagct ctggccacta tgggaagacg tggcggtaaa aaggccgcag 4500
   aacgctggaa agacccaaac agtgagtacg cccgagcaca gcgagaaaaa ctagctaagt 4560
   ccagtcaacg acaagctagg aaagctaaag gaaatcgctt gaccattgca ggttggttta 4620
   tgactgttga gggagagact ggctcgtggc cgacaatcaa tgaagctatg tctgaattta 4680
   gcgtgtcacg tcagaccgtg aatagagcac ttaaggtctg cgggcattga acttccacga 4740
   ggacgccgaa agcttcccag taaatgtgcc atctcgtagg cagaaaacgg ttcccccgta 4800
   gggtctctct cttggcctcc tttctaggtc gggctgattg ctcttgaagc tctctagggg 4860
   ggctcacacc ataggcagat aacgttcccc accggctcgc ctcgtaagcg cacaaggact 4920
   gctcccaaag atcttcaaag ccactgccgc gactgccttc gcgaagcctt gccccgcgga 4980
   aatttcctcc accgagttcg tgcacacccc tatgccaagc ttctttcacc ctaaattcga 5040
   gagattggat tcttaccgtg gaaattcttc gcaaaaatcg tcccctgatc gcccttgcga 5100
   cgttggcgtc ggtgccgctg gttgcgcttg gcttgaccga cttgatcagc ggccgc 5156
<210> 35
   <211> 30
   <212> DNA
   <213> Corynebacterium glutamicum
<220>
   <223> SEQ_ID_35
<400> 35
   gagacatatg cccaccctcg cgccttcagg 30
<210> 36
   <211> 30
   <212> DNA
   <213> Corynebacterium glutamicum
<220>
   <223> SEQ_ID_36
<400> 36
   ctctactagt ttagatgtag aactcgatgt 30
<210> 37
   <211> 6287
   <212> DNA
   <213> Corynebacterium glutamicum
<220>
   <223> SEQ_ID_37_PCLIK5MCS_META_OHNE_STARTCODON
<220>
   <221> misc_feature
   <222> (54 )..(1184 )
   <223> MetA without startcodon
<220>
   <221> misc_feature
   <222> (1252 )..(1321 )
   <223> GroEL Terminator
<220>
   <221> misc_feature
   <222> (1665 )..(2456 )
   <223> KanR
<220>
   <221> misc_feature
   <222> (2723 )..(3583 )
   <223> Ori-EC(pMB) complement
<220>
   <221> misc_feature
   <222> (3729 )..(4403 )
   <223> Orf1
<220>
   <221> misc_feature
   <222> (4737 )..(5858 )
   <223> REP Protein
<400> 37
   tcgatttaaa tctcgagagg cctgacgtcg ggcccggtac cacgcgtcat atgcccaccc 60
   tcgcgccttc aggtcaactt gaaatccaag cgatcggtga tgtctccacc gaagccggag 120
   caatcattac aaacgctgaa atcgcctatc accgctgggg tgaataccgc gtagataaag 180
   aaggacgcag caatgtcgtt ctcatcgaac acgccctcac tggagattcc aacgcagccg 240
   attggtgggc tgacttgctc ggtcccggca aagccatcaa cactgatatt tactgcgtga 300
   tctgtaccaa cgtcatcggt ggttgcaacg gttccaccgg acctggctcc atgcatccag 360
   atggaaattt ctggggtaat cgcttccccg ccacgtccat tcgtgatcag gtaaacgccg 420
   aaaaacaatt cctcgacgca ctcggcatca ccacggtcgc cgcagtactt ggtggttcca 480
   tgggtggtgc ccgcacccta gagtgggccg caatgtaccc agaaactgtt ggcgcagctg 540
   ctgttcttgc agtttctgca cgcgccagcg cctggcaaat cggcattcaa tccgcccaaa 600
   ttaaggcgat tgaaaacgac caccactggc acgaaggcaa ctactacgaa tccggctgca 660
   acccagccac cggactcggc gccgcccgac gcatcgccca cctcacctac cgtggcgaac 720
   tagaaatcga cgaacgcttc ggcaccaaag cccaaaagaa cgaaaaccca ctcggtccct 780
   accgcaagcc cgaccagcgc ttcgccgtgg aatcctactt ggactaccaa gcagacaagc 840
   tagtacagcg tttcgacgcc ggctcctacg tcttgctcac cgacgccctc aaccgccacg 900
   acattggtcg cgaccgcgga ggcctcaaca aggcactcga atccatcaaa gttccagtcc 960
   ttgtcgcagg cgtagatacc gatattttgt acccctacca ccagcaagaa cacctctcca 1020
   gaaacctggg aaatctactg gcaatggcaa aaatcgtatc ccctgtcggc cacgatgctt 1080
   tcctcaccga aagccgccaa atggatcgca tcgtgaggaa cttcttcagc ctcatctccc 1140
   cagacgaaga caacccttcg acctacatcg agttctacat ctaaactagt tcggacctag 1200
   ggatatcgtc gacatcgatg ctcttctgcg ttaattaaca attgggatcc tctagagttc 1260
   tgtgaaaaac accgtggggc agtttctgct tcgcggtgtt ttttatttgt ggggcactag 1320
   acccgggatt taaatcgcta gcgggctgct aaaggaagcg gaacacgtag aaagccagtc 1380
   cgcagaaacg gtgctgaccc cggatgaatg tcagctactg ggctatctgg acaagggaaa 1440
   acgcaagcgc aaagagaaag caggtagctt gcagtgggct tacatggcga tagctagact 1500
   gggcggtttt atggacagca agcgaaccgg aattgccagc tggggcgccc tctggtaagg 1560
   ttgggaagcc ctgcaaagta aactggatgg ctttcttgcc gccaaggatc tgatggcgca 1620
   ggggatcaag atctgatcaa gagacaggat gaggatcgtt tcgcatgatt gaacaagatg 1680
   gattgcacgc aggttctccg gccgcttggg tggagaggct attcggctat gactgggcac 1740
   aacagacaat cggctgctct gatgccgccg tgttccggct gtcagcgcag gggcgcccgg 1800
   ttctttttgt caagaccgac ctgtccggtg ccctgaatga actgcaggac gaggcagcgc 1860
   ggctatcgtg gctggccacg acgggcgttc cttgcgcagc tgtgctcgac gttgtcactg 1920
   aagcgggaag ggactggctg ctattgggcg aagtgccggg gcaggatctc ctgtcatctc 1980
   accttgctcc tgccgagaaa gtatccatca tggctgatgc aatgcggcgg ctgcatacgc 2040
   ttgatccggc tacctgccca ttcgaccacc aagcgaaaca tcgcatcgag cgagcacgta 2100
   ctcggatgga agccggtctt gtcgatcagg atgatctgga cgaagagcat caggggctcg 2160
   cgccagccga actgttcgcc aggctcaagg cgcgcatgcc cgacggcgag gatctcgtcg 2220
   tgacccatgg cgatgcctgc ttgccgaata tcatggtgga aaatggccgc ttttctggat 2280
   tcatcgactg tggccggctg ggtgtggcgg accgctatca ggacatagcg ttggctaccc 2340
   gtgatattgc tgaagagctt ggcggcgaat gggctgaccg cttcctcgtg ctttacggta 2400
   tcgccgctcc cgattcgcag cgcatcgcct tctatcgcct tcttgacgag ttcttctgag 2460
   cgggactctg gggttcgaaa tgaccgacca agcgacgccc aacctgccat cacgagattt 2520
   cgattccacc gccgccttct atgaaaggtt gggcttcgga atcgttttcc gggacgccgg 2580
   ctggatgatc ctccagcgcg gggatctcat gctggagttc ttcgcccacg ctagcggcgc 2640
   gccggccggc ccggtgtgaa ataccgcaca gatgcgtaag gagaaaatac cgcatcaggc 2700
   gctcttccgc ttcctcgctc actgactcgc tgcgctcggt cgttcggctg cggcgagcgg 2760
   tatcagctca ctcaaaggcg gtaatacggt tatccacaga atcaggggat aacgcaggaa 2820
   agaacatgtg agcaaaaggc cagcaaaagg ccaggaaccg taaaaaggcc gcgttgctgg 2880
   cgtttttcca taggctccgc ccccctgacg agcatcacaa aaatcgacgc tcaagtcaga 2940
   ggtggcgaaa cccgacagga ctataaagat accaggcgtt tccccctgga agctccctcg 3000
   tgcgctctcc tgttccgacc ctgccgctta ccggatacct gtccgccttt ctcccttcgg 3060
   gaagcgtggc gctttctcat agctcacgct gtaggtatct cagttcggtg taggtcgttc 3120
   gctccaagct gggctgtgtg cacgaacccc ccgttcagcc cgaccgctgc gccttatccg 3180
   gtaactatcg tcttgagtcc aacccggtaa gacacgactt atcgccactg gcagcagcca 3240
   ctggtaacag gattagcaga gcgaggtatg taggcggtgc tacagagttc ttgaagtggt 3300
   ggcctaacta cggctacact agaaggacag tatttggtat ctgcgctctg ctgaagccag 3360
   ttaccttcgg aaaaagagtt ggtagctctt gatccggcaa acaaaccacc gctggtagcg 3420
   gtggtttttt tgtttgcaag cagcagatta cgcgcagaaa aaaaggatct caagaagatc 3480
   ctttgatctt ttctacgggg tctgacgctc agtggaacga aaactcacgt taagggattt 3540
   tggtcatgag attatcaaaa aggatcttca cctagatcct tttaaaggcc ggccgcggcc 3600
   gcgcaaagtc ccgcttcgtg aaaattttcg tgccgcgtga ttttccgcca aaaactttaa 3660
   cgaacgttcg ttataatggt gtcatgacct tcacgacgaa gtactaaaat tggcccgaat 3720
   catcagctat ggatctctct gatgtcgcgc tggagtccga cgcgctcgat gctgccgtcg 3780
   atttaaaaac ggtgatcgga tttttccgag ctctcgatac gacggacgcg ccagcatcac 3840
   gagactgggc cagtgccgcg agcgacctag aaactctcgt ggcggatctt gaggagctgg 3900
   ctgacgagct gcgtgctcgg ccagcgccag gaggacgcac agtagtggag gatgcaatca 3960
   gttgcgccta ctgcggtggc ctgattcctc cccggcctga cccgcgagga cggcgcgcaa 4020
   aatattgctc agatgcgtgt cgtgccgcag ccagccgcga gcgcgccaac aaacgccacg 4080
   ccgaggagct ggaggcggct aggtcgcaaa tggcgctgga agtgcgtccc ccgagcgaaa 4140
   ttttggccat ggtcgtcaca gagctggaag cggcagcgag aattatcgcg atcgtggcgg 4200
   tgcccgcagg catgacaaac atcgtaaatg ccgcgtttcg tgtgccgtgg ccgcccagga 4260
   cgtgtcagcg ccgccaccac ctgcaccgaa tcggcagcag cgtcgcgcgt cgaaaaagcg 4320
   cacaggcggc aagaagcgat aagctgcacg aatacctgaa aaatgttgaa cgccccgtga 4380
   gcggtaactc acagggcgtc ggctaacccc cagtccaaac ctgggagaaa gcgctcaaaa 4440
   atgactctag cggattcacg agacattgac acaccggcct ggaaattttc cgctgatctg 4500
   ttcgacaccc atcccgagct cgcgctgcga tcacgtggct ggacgagcga agaccgccgc 4560
   gaattcctcg ctcacctggg cagagaaaat ttccagggca gcaagacccg cgacttcgcc 4620
   agcgcttgga tcaaagaccc ggacacggag aaacacagcc gaagttatac cgagttggtt 4680
   caaaatcgct tgcccggtgc cagtatgttg ctctgacgca cgcgcagcac gcagccgtgc 4740
   ttgtcctgga cattgatgtg ccgagccacc aggccggcgg gaaaatcgag cacgtaaacc 4800
   ccgaggtcta cgcgattttg gagcgctggg cacgcctgga aaaagcgcca gcttggatcg 4860
   gcgtgaatcc actgagcggg aaatgccagc tcatctggct cattgatccg gtgtatgccg 4920
   cagcaggcat gagcagcccg aatatgcgcc tgctggctgc aacgaccgag gaaatgaccc 4980
   gcgttttcgg cgctgaccag gctttttcac ataggctgag ccgtggccac tgcactctcc 5040
   gacgatccca gccgtaccgc tggcatgccc agcacaatcg cgtggatcgc ctagctgatc 5100
   ttatggaggt tgctcgcatg atctcaggca cagaaaaacc taaaaaacgc tatgagcagg 5160
   agttttctag cggacgggca cgtatcgaag cggcaagaaa agccactgcg gaagcaaaag 5220
   cacttgccac gcttgaagca agcctgccga gcgccgctga agcgtctgga gagctgatcg 5280
   acggcgtccg tgtcctctgg actgctccag ggcgtgccgc ccgtgatgag acggcttttc 5340
   gccacgcttt gactgtggga taccagttaa aagcggctgg tgagcgccta aaagacacca 5400
   agggtcatcg agcctacgag cgtgcctaca ccgtcgctca ggcggtcgga ggaggccgtg 5460
   agcctgatct gccgccggac tgtgaccgcc agacggattg gccgcgacgt gtgcgcggct 5520
   acgtcgctaa aggccagcca gtcgtccctg ctcgtcagac agagacgcag agccagccga 5580
   ggcgaaaagc tctggccact atgggaagac gtggcggtaa aaaggccgca gaacgctgga 5640
   aagacccaaa cagtgagtac gcccgagcac agcgagaaaa actagctaag tccagtcaac 5700
   gacaagctag gaaagctaaa ggaaatcgct tgaccattgc aggttggttt atgactgttg 5760
   agggagagac tggctcgtgg ccgacaatca atgaagctat gtctgaattt agcgtgtcac 5820
   gtcagaccgt gaatagagca cttaaggtct gcgggcattg aacttccacg aggacgccga 5880
   aagcttccca gtaaatgtgc catctcgtag gcagaaaacg gttcccccgt agggtctctc 5940
   tcttggcctc ctttctaggt cgggctgatt gctcttgaag ctctctaggg gggctcacac 6000
   cataggcaga taacgttccc caccggctcg cctcgtaagc gcacaaggac tgctcccaaa 6060
   gatcttcaaa gccactgccg cgactgcctt cgcgaagcct tgccccgcgg aaatttcctc 6120
   caccgagttc gtgcacaccc ctatgccaag cttctttcac cctaaattcg agagattgga 6180
   ttcttaccgt ggaaattctt cgcaaaaatc gtcccctgat cgcccttgcg acgttggcgt 6240
   cggtgccgct ggttgcgctt ggcttgaccg acttgatcag cggccgc 6287
<210> 38
   <211> 30
   <212> DNA
   <213> Corynebacterium glutamicum
<220>
   <223> SEQ_ID_38_OLIGONUKLEOTIDPRIMER_1701
<400> 38
   gagactcgag cggcttaaag tttggctgcc 30
<210> 39
   <211> 32
   <212> DNA
   <213> Corynebacterium glutamicum
<220>
   <223> SEQ_ID_39_OLIGONUKLEOTIDPRIMER_1828
<400> 39
   ctctcatatg caatccctcc atgagaaaaa tt 32
<210> 40
   <211> 40
   <212> DNA
   <213> Corynebacterium glutamicum
<220>
   <223> SEQ_ID_40_OLIGONUKLEOTIDPRIMER_1831
<400> 40
   ctctcatatg cgcggccgca atccctccat gagaaaaatt 40
<210> 41
   <211> 39
   <212> DNA
   <213> Corynebacterium glutamicum
<220>
   <223> SEQ_ID_41_OLIGONUKLEOTIDPRIMER_1832
<400> 41
   ctctcatatg caatctctcc atgagaaaaa ttttgtgtg 39
<210> 42
   <211> 39
   <212> DNA
   <213> Corynebacterium glutamicum
<220>
   <223> SEQ_ID_42_OLIGONUKLEOTIDPRIMER_1833
<400> 42
   ctctcatatg caatctcctc atgagaaaaa ttttgtgtg 39
<210> 43
   <211> 39
   <212> DNA
   <213> Corynebacterium glutamicum
<220>
   <223> SEQ_ID_43_OLIGONUKLEOTIDPRIMER_1834
<400> 43
   ctctcatatg caatcccttc atgagaaaaa ttttgtgtg 39
<210> 44
   <211> 2961
   <212> DNA
   <213> Corynebacterium glutamicum
<220>
   <223> SEQ_ID_44_PBS_KS+
<400> 44
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tggagctcca 660
   ccgcggtggc ggccgctcta gaactagtgg atcccccggg ctgcaggaat tcgatatcaa 720
   gcttatcgat accgtcgacc tcgagggggg gcccggtacc cagcttttgt tccctttagt 780
   gagggttaat tgcgcgcttg gcgtaatcat ggtcatagct gtttcctgtg tgaaattgtt 840
   atccgctcac aattccacac aacatacgag ccggaagcat aaagtgtaaa gcctggggtg 900
   cctaatgagt gagctaactc acattaattg cgttgcgctc actgcccgct ttccagtcgg 960
   gaaacctgtc gtgccagctg cattaatgaa tcggccaacg cgcggggaga ggcggtttgc 1020
   gtattgggcg ctcttccgct tcctcgctca ctgactcgct gcgctcggtc gttcggctgc 1080
   ggcgagcggt atcagctcac tcaaaggcgg taatacggtt atccacagaa tcaggggata 1140
   acgcaggaaa gaacatgtga gcaaaaggcc agcaaaaggc caggaaccgt aaaaaggccg 1200
   cgttgctggc gtttttccat aggctccgcc cccctgacga gcatcacaaa aatcgacgct 1260
   caagtcagag gtggcgaaac ccgacaggac tataaagata ccaggcgttt ccccctggaa 1320
   gctccctcgt gcgctctcct gttccgaccc tgccgcttac cggatacctg tccgcctttc 1380
   tcccttcggg aagcgtggcg ctttctcata gctcacgctg taggtatctc agttcggtgt 1440
   aggtcgttcg ctccaagctg ggctgtgtgc acgaaccccc cgttcagccc gaccgctgcg 1500
   ccttatccgg taactatcgt cttgagtcca acccggtaag acacgactta tcgccactgg 1560
   cagcagccac tggtaacagg attagcagag cgaggtatgt aggcggtgct acagagttct 1620
   tgaagtggtg gcctaactac ggctacacta gaaggacagt atttggtatc tgcgctctgc 1680
   tgaagccagt taccttcgga aaaagagttg gtagctcttg atccggcaaa caaaccaccg 1740
   ctggtagcgg tggttttttt gtttgcaagc agcagattac gcgcagaaaa aaaggatctc 1800
   aagaagatcc tttgatcttt tctacggggt ctgacgctca gtggaacgaa aactcacgtt 1860
   aagggatttt ggtcatgaga ttatcaaaaa ggatcttcac ctagatcctt ttaaattaaa 1920
   aatgaagttt taaatcaatc taaagtatat atgagtaaac ttggtctgac agttaccaat 1980
   gcttaatcag tgaggcacct atctcagcga tctgtctatt tcgttcatcc atagttgcct 2040
   gactccccgt cgtgtagata actacgatac gggagggctt accatctggc cccagtgctg 2100
   caatgatacc gcgagaccca cgctcaccgg ctccagattt atcagcaata aaccagccag 2160
   ccggaagggc cgagcgcaga agtggtcctg caactttatc cgcctccatc cagtctatta 2220
   attgttgccg ggaagctaga gtaagtagtt cgccagttaa tagtttgcgc aacgttgttg 2280
   ccattgctac aggcatcgtg gtgtcacgct cgtcgtttgg tatggcttca ttcagctccg 2340
   gttcccaacg atcaaggcga gttacatgat cccccatgtt gtgcaaaaaa gcggttagct 2400
   ccttcggtcc tccgatcgtt gtcagaagta agttggccgc agtgttatca ctcatggtta 2460
   tggcagcact gcataattct cttactgtca tgccatccgt aagatgcttt tctgtgactg 2520
   gtgagtactc aaccaagtca ttctgagaat agtgtatgcg gcgaccgagt tgctcttgcc 2580
   cggcgtcaat acgggataat accgcgccac atagcagaac tttaaaagtg ctcatcattg 2640
   gaaaacgttc ttcggggcga aaactctcaa ggatcttacc gctgttgaga tccagttcga 2700
   tgtaacccac tcgtgcaccc aactgatctt cagcatcttt tactttcacc agcgtttctg 2760
   ggtgagcaaa aacaggaagg caaaatgccg caaaaaaggg aataagggcg acacggaaat 2820
   gttgaatact catactcttc ctttttcaat attattgaag catttatcag ggttattgtc 2880
   tcatgagcgg atacatattt gaatgtattt agaaaaataa acaaataggg gttccgcgca 2940
   catttccccg aaaagtgcca c 2961
<210> 45
   <211> 6431
   <212> DNA
   <213> Corynebacterium glutamicum
<220>
   <223> SEQ_ID_45_PCLIK5MCS_PGRO_1701_1828_META
<220>
   <221> misc_feature
   <222> (726 )..(899 )
   <223> P1701/1828
<220>
   <221> misc_feature
   <222> (906 )..(2036 )
   <223> MetA without startcodon
<220>
   <221> misc_feature
   <222> (2104 )..(2173 )
   <223> GroEL Terminator
<220>
   <221> misc_teature
   <222> (2517 )..(3308 )
   <223> KanR
<220>
   <221> misc_feature
   <222> (3575 )..(4435 )
   <223> Ori-EC(pMB) complement
<220>
   <221> misc_feature
   <222> (4581 )..(5255 )
   <223> Orf1
<220>
   <221> misc_feature
   <222> (5589 )..(279 )
   <223> REP Protein
<400> 45
   aggcgaaaag ctctggccac tatgggaaga cgtggcggta aaaaggccgc agaacgctgg 60
   aaagacccaa acagtgagta cgcccgagca cagcgagaaa aactagctaa gtccagtcaa 120
   cgacaagcta ggaaagctaa aggaaatcgc ttgaccattg caggttggtt tatgactgtt 180
   gagggagaga ctggctcgtg gccgacaatc aatgaagcta tgtctgaatt tagcgtgtca 240
   cgtcagaccg tgaatagagc acttaaggtc tgcgggcatt gaacttccac gaggacgccg 300
   aaagcttccc agtaaatgtg ccatctcgta ggcagaaaac ggttcccccg tagggtctct 360
   ctcttggcct cctttctagg tcgggctgat tgctcttgaa gctctctagg ggggctcaca 420
   ccataggcag ataacgttcc ccaccggctc gcctcgtaag cgcacaagga ctgctcccaa 480
   agatcttcaa agccactgcc gcgactgcct tcgcgaagcc ttgccccgcg gaaatttcct 540
   ccaccgagtt cgtgcacacc cctatgccaa gcttctttca ccctaaattc gagagattgg 600
   attcttaccg tggaaattct tcgcaaaaat cgtcccctga tcgcccttgc gacgttggcg 660
   tcggtgccgc tggttgcgct tggcttgacc gacttgatca gcggccgctc gatttaaatc 720
   tcgagcggct taaagtttgg ctgccatgtg aatttttagc accctcaaca gttgagtgct 780
   ggcactctcg ggggtagagt gccaaatagg ttgtttgaca cacagttgtt cacccgcgac 840
   gacggctgtg ctggaaaccc acaaccggca cacacaaaat ttttctcatg gagggattgc 900
   atatgcccac cctcgcgcct tcaggtcaac ttgaaatcca agcgatcggt gatgtctcca 960
   ccgaagccgg agcaatcatt acaaacgctg aaatcgccta tcaccgctgg ggtgaatacc 1020
   gcgtagataa agaaggacgc agcaatgtcg ttctcatcga acacgccctc actggagatt 1080
   ccaacgcagc cgattggtgg gctgacttgc tcggtcccgg caaagccatc aacactgata 1140
   tttactgcgt gatctgtacc aacgtcatcg gtggttgcaa cggttccacc ggacctggct 1200
   ccatgcatcc agatggaaat ttctggggta atcgcttccc cgccacgtcc attcgtgatc 1260
   aggtaaacgc cgaaaaacaa ttcctcgacg cactcggcat caccacggtc gccgcagtac 1320
   ttggtggttc catgggtggt gcccgcaccc tagagtgggc cgcaatgtac ccagaaactg 1380
   ttggcgcagc tgctgttctt gcagtttctg cacgcgccag cgcctggcaa atcggcattc 1440
   aatccgccca aattaaggcg attgaaaacg accaccactg gcacgaaggc aactactacg 1500
   aatccggctg caacccagcc accggactcg gcgccgcccg acgcatcgcc cacctcacct 1560
   accgtggcga actagaaatc gacgaacgct tcggcaccaa agcccaaaag aacgaaaacc 1620
   cactcggtcc ctaccgcaag cccgaccagc gcttcgccgt ggaatcctac ttggactacc 1680
   aagcagacaa gctagtacag cgtttcgacg ccggctccta cgtcttgctc accgacgccc 1740
   tcaaccgcca cgacattggt cgcgaccgcg gaggcctcaa caaggcactc gaatccatca 1800
   aagttccagt ccttgtcgca ggcgtagata ccgatatttt gtacccctac caccagcaag 1860
   aacacctctc cagaaacctg ggaaatctac tggcaatggc aaaaatcgta tcccctgtcg 1920
   gccacgatgc tttcctcacc gaaagccgcc aaatggatcg catcgtgagg aacttcttca 1980
   gcctcatctc cccagacgaa gacaaccctt cgacctacat cgagttctac atctaaacta 2040
   gttcggacct agggatatcg tcgacatcga tgctcttctg cgttaattaa caattgggat 2100
   cctctagagt tctgtgaaaa acaccgtggg gcagtttctg cttcgcggtg tttttatttt 2160
   gtggggcact agacccggga tttaaatcgc tagcgggctg ctaaaggaag cggaacacgt 2220
   agaaagccag tccgcagaaa cggtgctgac cccggatgaa tgtcagctac tgggctatct 2280
   ggacaaggga aaacgcaagc gcaaagagaa agcaggtagc ttgcagtggg cttacatggc 2340
   gatagctaga ctgggcggtt ttatggacag caagcgaacc ggaattgcca gctggggcgc 2400
   cctctggtaa ggttgggaag ccctgcaaag taaactggat ggctttcttg ccgccaagga 2460
   tctgatggcg caggggatca agatctgatc aagagacagg atgaggatcg tttcgcatga 2520
   ttgaacaaga tggattgcac gcaggttctc cggccgcttg ggtggagagg ctattcggct 2580
   atgactgggc acaacagaca atcggctgct ctgatgccgc cgtgttccgg ctgtcagcgc 2640
   aggggcgccc ggttcttttt gtcaagaccg acctgtccgg tgccctgaat gaactgcagg 2700
   acgaggcagc gcggctatcg tggctggcca cgacgggcgt tccttgcgca gctgtgctcg 2760
   acgttgtcac tgaagcggga agggactggc tgctattggg cgaagtgccg gggcaggatc 2820
   tcctgtcatc tcaccttgct cctgccgaga aagtatccat catggctgat gcaatgcggc 2880
   ggctgcatac gcttgatccg gctacctgcc cattcgacca ccaagcgaaa catcgcatcg 2940
   agcgagcacg tactcggatg gaagccggtc ttgtcgatca ggatgatctg gacgaagagc 3000
   atcaggggct cgcgccagcc gaactgttcg ccaggctcaa ggcgcgcatg cccgacggcg 3060
   aggatctcgt cgtgacccat ggcgatgcct gcttgccgaa tatcatggtg gaaaatggcc 3120
   gcttttctgg attcatcgac tgtggccggc tgggtgtggc ggaccgctat caggacatag 3180
   cgttggctac ccgtgatatt gctgaagagc ttggcggcga atgggctgac cgcttcctcg 3240
   tgctttacgg tatcgccgct cccgattcgc agcgcatcgc cttctatcgc cttcttgacg 3300
   agttcttctg agcgggactc tggggttcga aatgaccgac caagcgacgc ccaacctgcc 3360
   atcacgagat ttcgattcca ccgccgcctt ctatgaaagg ttgggcttcg gaatcgtttt 3420
   ccgggacgcc ggctggatga tcctccagcg cggggatctc atgctggagt tcttcgccca 3480
   cgctagcggc gcgccggccg gcccggtgtg aaataccgca cagatgcgta aggagaaaat 3540
   accgcatcag gcgctcttcc gcttcctcgc tcactgactc gctgcgctcg gtcgttcggc 3600
   tgcggcgagc ggtatcagct cactcaaagg cggtaatacg gttatccaca gaatcagggg 3660
   ataacgcagg aaagaacatg tgagcaaaag gccagcaaaa ggccaggaac cgtaaaaagg 3720
   ccgcgttgct ggcgtttttc cataggctcc gcccccctga cgagcatcac aaaaatcgac 3780
   gctcaagtca gaggtggcga aacccgacag gactataaag ataccaggcg tttccccctg 3840
   gaagctccct cgtgcgctct cctgttccga ccctgccgct taccggatac ctgtccgcct 3900
   ttctcccttc gggaagcgtg gcgctttctc atagctcacg ctgtaggtat ctcagttcgg 3960
   tgtaggtcgt tcgctccaag ctgggctgtg tgcacgaacc ccccgttcag cccgaccgct 4020
   gcgccttatc cggtaactat cgtcttgagt ccaacccggt aagacacgac ttatcgccac 4080
   tggcagcagc cactggtaac aggattagca gagcgaggta tgtaggcggt gctacagagt 4140
   tcttgaagtg gtggcctaac tacggctaca ctagaaggac agtatttggt atctgcgctc 4200
   tgctgaagcc agttaccttc ggaaaaagag ttggtagctc ttgatccggc aaacaaacca 4260
   ccgctggtag cggtggtttt tttgtttgca agcagcagat tacgcgcaga aaaaaaggat 4320
   ctcaagaaga tcctttgatc ttttctacgg ggtctgacgc tcagtggaac gaaaactcac 4380
   gttaagggat tttggtcatg agattatcaa aaaggatctt cacctagatc cttttaaagg 4440
   ccggccgcgg ccgcgcaaag tcccgcttcg tgaaaatttt cgtgccgcgt gattttccgc 4500
   caaaaacttt aacgaacgtt cgttataatg gtgtcatgac cttcacgacg aagtactaaa 4560
   attggcccga atcatcagct atggatctct ctgatgtcgc gctggagtcc gacgcgctcg 4620
   atgctgccgt cgatttaaaa acggtgatcg gatttttccg agctctcgat acgacggacg 4680
   cgccagcatc acgagactgg gccagtgccg cgagcgacct agaaactctc gtggcggatc 4740
   ttgaggagct ggctgacgag ctgcgtgctc ggccagcgcc aggaggacgc acagtagtgg 4800
   aggatgcaat cagttgcgcc tactgcggtg gcctgattcc tccccggcct gacccgcgag 4860
   gacggcgcgc aaaatattgc tcagatgcgt gtcgtgccgc agccagccgc gagcgcgcca 4920
   acaaacgcca cgccgaggag ctggaggcgg ctaggtcgca aatggcgctg gaagtgcgtc 4980
   ccccgagcga aattttggcc atggtcgtca cagagctgga agcggcagcg agaattatcg 5040
   cgatcgtggc ggtgcccgca ggcatgacaa acatcgtaaa tgccgcgttt cgtgtgccgt 5100
   ggccgcccag gacgtgtcag cgccgccacc acctgcaccg aatcggcagc agcgtcgcgc 5160
   gtcgaaaaag cgcacaggcg gcaagaagcg ataagctgca cgaatacctg aaaaatgttg 5220
   aacgccccgt gagcggtaac tcacagggcg tcggctaacc cccagtccaa acctgggaga 5280
   aagcgctcaa aaatgactct agcggattca cgagacattg acacaccggc ctggaaattt 5340
   tccgctgatc tgttcgacac ccatcccgag ctcgcgctgc gatcacgtgg ctggacgagc 5400
   gaagaccgcc gcgaattcct cgctcacctg ggcagagaaa atttccaggg cagcaagacc 5460
   cgcgacttcg ccagcgcttg gatcaaagac ccggacacgg agaaacacag ccgaagttat 5520
   accgagttgg ttcaaaatcg cttgcccggt gccagtatgt tgctctgacg cacgcgcagc 5580
   acgcagccgt gcttgtcctg gacattgatg tgccgagcca ccaggccggc gggaaaatcg 5640
   agcacgtaaa ccccgaggtc tacgcgattt tggagcgctg ggcacgcctg gaaaaagcgc 5700
   cagcttggat cggcgtgaat ccactgagcg ggaaatgcca gctcatctgg ctcattgatc 5760
   cggtgtatgc cgcagcaggc atgagcagcc cgaatatgcg cctgctggct gcaacgaccg 5820
   aggaaatgac ccgcgttttc ggcgctgacc aggctttttc acataggctg agccgtggcc 5880
   actgcactct ccgacgatcc cagccgtacc gctggcatgc ccagcacaat cgcgtggatc 5940
   gcctagctga tcttatggag gttgctcgca tgatctcagg cacagaaaaa cctaaaaaac 6000
   gctatgagca ggagttttct agcggacggg cacgtatcga agcggcaaga aaagccactg 6060
   cggaagcaaa agcacttgcc acgcttgaag caagcctgcc gagcgccgct gaagcgtctg 6120
   gagagctgat cgacggcgtc cgtgtcctct ggactgctcc agggcgtgcc gcccgtgatg 6180
   agacggcttt tcgccacgct ttgactgtgg gataccagtt aaaagcggct ggtgagcgcc 6240
   taaaagacac caagggtcat cgagcctacg agcgtgccta caccgtcgct caggcggtcg 6300
   gaggaggccg tgagcctgat ctgccgccgg actgtgaccg ccagacggat tggccgcgac 6360
   gtgtgcgcgg ctacgtcgct aaaggccagc cagtcgtccc tgctcgtcag acagagacgc 6420
   agagccagcc g 6431
<210> 46
   <211> 6439
   <212> DNA
   <213> Corynebacterium glutamicum
<220>
   <223> SEQ_ID_46_PCLIK5MCS_PGRO_1701_1831_META
<220>
   <221> misc_feature
   <222> (11 )..(183 )
   <223> P1701/1831
<220>
   <221> misc_feature
   <222> (199 )..(1329 )
   <223> MetA without startcodon
<220>
   <221> misc_feature
   <222> (1397 )..(1466 )
   <223> GroEL Terminator
<220>
   <221> misc_feature
   <222> (1810 )..(2601 )
   <223> KanR
<220>
   <221> misc_feature
   <222> (2868 )..(3728 )
   <223> Ori-EC(pMB) complement
<220>
   <221> misc_feature
   <222> (3874 )..(4548 )
   <223> Orf1
<220>
   <221> misc_feature
   <222> (4882 )..(6003 )
   <223> REP Protein
<400> 46
   aaatctcgag cggcttaaag tttggctgcc atgtgaattt ttagcaccct caacagttga 60
   gtgctggcac tctcgggggt agagtgccaa ataggttgtt tgacacacag ttgttcaccc 120
   gcgacgacgg ctgtgctgga aacccacaac cggcacacac aaaatttttc tcatggaggg 180
   attgcggccg cgcatatgcc caccctcgcg ccttcaggtc aacttgaaat ccaagcgatc 240
   ggtgatgtct ccaccgaagc cggagcaatc attacaaacg ctgaaatcgc ctatcaccgc 300
   tggggtgaat accgcgtaga taaagaagga cgcagcaatg tcgttctcat cgaacacgcc 360
   ctcactggag attccaacgc agccgattgg tgggctgact tgctcggtcc cggcaaagcc 420
   atcaacactg atatttactg cgtgatctgt accaacgtca tcggtggttg caacggttcc 480
   accggacctg gctccatgca tccagatgga aatttctggg gtaatcgctt ccccgccacg 540
   tccattcgtg atcaggtaaa cgccgaaaaa caattcctcg acgcactcgg catcaccacg 600
   gtcgccgcag tacttggtgg ttccatgggt ggtgcccgca ccctagagtg ggccgcaatg 660
   tacccagaaa ctgttggcgc agctgctgtt cttgcagttt ctgcacgcgc cagcgcctgg 720
   caaatcggca ttcaatccgc ccaaattaag gcgattgaaa acgaccacca ctggcacgaa 780
   ggcaactact acgaatccgg ctgcaaccca gccaccggac tcggcgccgc ccgacgcatc 840
   gcccacctca cctaccgtgg cgaactagaa atcgacgaac gcttcggcac caaagcccaa 900
   aagaacgaaa acccactcgg tccctaccgc aagcccgacc agcgcttcgc cgtggaatcc 960
   tacttggact accaagcaga caagctagta cagcgtttcg acgccggctc ctacgtcttg 1020
   ctcaccgacg ccctcaaccg ccacgacatt ggtcgcgacc gcggaggcct caacaaggca 1080
   ctcgaatcca tcaaagttcc agtccttgtc gcaggcgtag ataccgatat tttgtacccc 1140
   taccaccagc aagaacacct ctccagaaac ctgggaaatc tactggcaat ggcaaaaatc 1200
   gtatcccctg tcggccacga tgctttcctc accgaaagcc gccaaatgga tcgcatcgtg 1260
   aggaacttct tcagcctcat ctccccagac gaagacaacc cttcgaccta catcgagttc 1320
   tacatctaaa ctagttcgga cctagggata tcgtcgacat cgatgctctt ctgcgttaat 1380
   taacaattgg gatcctctag agttctgtga aaaacaccgt ggggcagttt ctgcttcgcg 1440
   gtgtttttta tttgtggggc actagacccg ggatttaaat cgctagcggg ctgctaaagg 1500
   aagcggaaca cgtagaaagc cagtccgcag aaacggtgct gaccccggat gaatgtcagc 1560
   tactgggcta tctggacaag ggaaaacgca agcgcaaaga gaaagcaggt agcttgcagt 1620
   gggcttacat ggcgatagct agactgggcg gttttatgga cagcaagcga accggaattg 1680
   ccagctgggg cgccctctgg taaggttggg aagccctgca aagtaaactg gatggctttc 1740
   ttgccgccaa ggatctgatg gcgcagggga tcaagatctg atcaagagac aggatgagga 1800
   tcgtttcgca tgattgaaca agatggattg cacgcaggtt ctccggccgc ttgggtggag 1860
   aggctattcg gctatgactg ggcacaacag acaatcggct gctctgatgc cgccgtgttc 1920
   cggctgtcag cgcaggggcg cccggttctt tttgtcaaga ccgacctgtc cggtgccctg 1980
   aatgaactgc aggacgaggc agcgcggcta tcgtggctgg ccacgacggg cgttccttgc 2040
   gcagctgtgc tcgacgttgt cactgaagcg ggaagggact ggctgctatt gggcgaagtg 2100
   ccggggcagg atctcctgtc atctcacctt gctcctgccg agaaagtatc catcatggct 2160
   gatgcaatgc ggcggctgca tacgcttgat ccggctacct gcccattcga ccaccaagcg 2220
   aaacatcgca tcgagcgagc acgtactcgg atggaagccg gtcttgtcga tcaggatgat 2280
   ctggacgaag agcatcaggg gctcgcgcca gccgaactgt tcgccaggct caaggcgcgc 2340
   atgcccgacg gcgaggatct cgtcgtgacc catggcgatg cctgcttgcc gaatatcatg 2400
   gtggaaaatg gccgcttttc tggattcatc gactgtggcc ggctgggtgt ggcggaccgc 2460
   tatcaggaca tagcgttggc tacccgtgat attgctgaag agcttggcgg cgaatgggct 2520
   gaccgcttcc tcgtgcttta cggtatcgcc gctcccgatt cgcagcgcat cgccttctat 2580
   cgccttcttg acgagttctt ctgagcggga ctctggggtt cgaaatgacc gaccaagcga 2640
   cgcccaacct gccatcacga gatttcgatt ccaccgccgc cttctatgaa aggttgggct 2700
   tcggaatcgt tttccgggac gccggctgga tgatcctcca gcgcggggat ctcatgctgg 2760
   agttcttcgc ccacgctagc ggcgcgccgg ccggcccggt gtgaaatacc gcacagatgc 2820
   gtaaggagaa aataccgcat caggcgctct tccgcttcct cgctcactga ctcgctgcgc 2880
   tcggtcgttc ggctgcggcg agcggtatca gctcactcaa aggcggtaat acggttatcc 2940
   acagaatcag gggataacgc aggaaagaac atgtgagcaa aaggccagca aaaggccagg 3000
   aaccgtaaaa aggccgcgtt gctggcgttt ttccataggc tccgcccccc tgacgagcat 3060
   cacaaaaatc gacgctcaag tcagaggtgg cgaaacccga caggactata aagataccag 3120
   gcgtttcccc ctggaagctc cctcgtgcgc tctcctgttc cgaccctgcc gcttaccgga 3180
   tacctgtccg cctttctccc ttcgggaagc gtggcgcttt ctcatagctc acgctgtagg 3240
   tatctcagtt cggtgtaggt cgttcgctcc aagctgggct gtgtgcacga accccccgtt 3300
   cagcccgacc gctgcgcctt atccggtaac tatcgtcttg agtccaaccc ggtaagacac 3360
   gacttatcgc cactggcagc agccactggt aacaggatta gcagagcgag gtatgtaggc 3420
   ggtgctacag agttcttgaa gtggtggcct aactacggct acactagaag gacagtattt 3480
   ggtatctgcg ctctgctgaa gccagttacc ttcggaaaaa gagttggtag ctcttgatcc 3540
   ggcaaacaaa ccaccgctgg tagcggtggt ttttttgttt gcaagcagca gattacgcgc 3600
   agaaaaaaag gatctcaaga agatcctttg atcttttcta cggggtctga cgctcagtgg 3660
   aacgaaaact cacgttaagg gattttggtc atgagattat caaaaaggat cttcacctag 3720
   atccttttaa aggccggccg cggccgcgca aagtcccgct tcgtgaaaat tttcgtgccg 3780
   cgtgattttc cgccaaaaac tttaacgaac gttcgttata atggtgtcat gaccttcacg 3840
   acgaagtact aaaattggcc cgaatcatca gctatggatc tctctgatgt cgcgctggag 3900
   tccgacgcgc tcgatgctgc cgtcgattta aaaacggtga tcggattttt ccgagctctc 3960
   gatacgacgg acgcgccagc atcacgagac tgggccagtg ccgcgagcga cctagaaact 4020
   ctcgtggcgg atcttgagga gctggctgac gagctgcgtg ctcggccagc gccaggagga 4080
   cgcacagtag tggaggatgc aatcagttgc gcctactgcg gtggcctgat tcctccccgg 4140
   cctgacccgc gaggacggcg cgcaaaatat tgctcagatg cgtgtcgtgc cgcagccagc 4200
   cgcgagcgcg ccaacaaacg ccacgccgag gagctggagg cggctaggtc gcaaatggcg 4260
   ctggaagtgc gtcccccgag cgaaattttg gccatggtcg tcacagagct ggaagcggca 4320
   gcgagaatta tcgcgatcgt ggcggtgccc gcaggcatga caaacatcgt aaatgccgcg 4380
   tttcgtgtgc cgtggccgcc caggacgtgt cagcgccgcc accacctgca ccgaatcggc 4440
   agcagcgtcg cgcgtcgaaa aagcgcacag gcggcaagaa gcgataagct gcacgaatac 4500
   ctgaaaaatg ttgaacgccc cgtgagcggt aactcacagg gcgtcggcta acccccagtc 4560
   caaacctggg agaaagcgct caaaaatgac tctagcggat tcacgagaca ttgacacacc 4620
   ggcctggaaa ttttccgctg atctgttcga cacccatccc gagctcgcgc tgcgatcacg 4680
   tggctggacg agcgaagacc gccgcgaatt cctcgctcac ctgggcagag aaaatttcca 4740
   gggcagcaag acccgcgact tcgccagcgc ttggatcaaa gacccggaca cggagaaaca 4800
   cagccgaagt tataccgagt tggttcaaaa tcgcttgccc ggtgccagta tgttgctctg 4860
   acgcacgcgc agcacgcagc cgtgcttgtc ctggacattg atgtgccgag ccaccaggcc 4920
   ggcgggaaaa tcgagcacgt aaaccccgag gtctacgcga ttttggagcg ctgggcacgc 4980
   ctggaaaaag cgccagcttg gatcggcgtg aatccactga gcgggaaatg ccagctcatc 5040
   tggctcattg atccggtgta tgccgcagca ggcatgagca gcccgaatat gcgcctgctg 5100
   gctgcaacga ccgaggaaat gacccgcgtt ttcggcgctg accaggcttt ttcacatagg 5160
   ctgagccgtg gccactgcac tctccgacga tcccagccgt accgctggca tgcccagcac 5220
   aatcgcgtgg atcgcctagc tgatcttatg gaggttgctc gcatgatctc aggcacagaa 5280
   aaacctaaaa aacgctatga gcaggagttt tctagcggac gggcacgtat cgaagcggca 5340
   agaaaagcca ctgcggaagc aaaagcactt gccacgcttg aagcaagcct gccgagcgcc 5400
   gctgaagcgt ctggagagct gatcgacggc gtccgtgtcc tctggactgc tccagggcgt 5460
   gccgcccgtg atgagacggc ttttcgccac gctttgactg tgggatacca gttaaaagcg 5520
   gctggtgagc gcctaaaaga caccaagggt catcgagcct acgagcgtgc ctacaccgtc 5580
   gctcaggcgg tcggaggagg ccgtgagcct gatctgccgc cggactgtga ccgccagacg 5640
   gattggccgc gacgtgtgcg cggctacgtc gctaaaggcc agccagtcgt ccctgctcgt 5700
   cagacagaga cgcagagcca gccgaggcga aaagctctgg ccactatggg aagacgtggc 5760
   ggtaaaaagg ccgcagaacg ctggaaagac ccaaacagtg agtacgcccg agcacagcga 5820
   gaaaaactag ctaagtccag tcaacgacaa gctaggaaag ctaaaggaaa tcgcttgacc 5880
   attgcaggtt ggtttatgac tgttgaggga gagactggct cgtggccgac aatcaatgaa 5940
   gctatgtctg aatttagcgt gtcacgtcag accgtgaata gagcacttaa ggtctgcggg 6000
   cattgaactt ccacgaggac gccgaaagct tcccagtaaa tgtgccatct cgtaggcaga 6060
   aaacggttcc cccgtagggt ctctctcttg gcctcctttc taggtcgggc tgattgctct 6120
   tgaagctctc taggggggct cacaccatag gcagataacg ttccccaccg gctcgcctcg 6180
   taagcgcaca aggactgctc ccaaagatct tcaaagccac tgccgcgact gccttcgcga 6240
   agccttgccc cgcggaaatt tcctccaccg agttcgtgca cacccctatg ccaagcttct 6300
   ttcaccctaa attcgagaga ttggattctt accgtggaaa ttcttcgcaa aaatcgtccc 6360
   ctgatcgccc ttgcgacgtt ggcgtcggtg ccgctggttg cgcttggctt gaccgacttg 6420
   atcagcggcc gctcgattt 6439
<210> 47
   <211> 6431
   <212> DNA
   <213> Corynebacterium glutamicum
<220>
   <223> SEQ_ID_47_PCLIK5MCS_PGRO_1701_1832_META
<220>
   <221> misc_feature
   <222> (726 )..(899 )
   <223> P1701/1832
<220>
   <221> misc_feature
   <222> (906 )..(2036 )
   <223> MetA without startcodon
<220>
   <221> misc_feature
   <222> (2104 )..(2173 )
   <223> GroEL Terminator
<220>
   <221> misc_feature
   <222> (2517 )..(3308 )
   <223> KanR
<220>
   <221> misc_feature
   <222> (3575 )..(4435 )
   <223> Ori-Ec(pMB) complement
<220>
   <221> misc_feature
   <222> (4581 )..(5255 )
   <223> Orf1
<220>
   <221> misc_feature
   <222> (5589 )..(279 )
   <223> REP Protein
<400> 47
   aggcgaaaag ctctggccac tatgggaaga cgtggcggta aaaaggccgc agaacgctgg 60
   aaagacccaa acagtgagta cgcccgagca cagcgagaaa aactagctaa gtccagtcaa 120
   cgacaagcta ggaaagctaa aggaaatcgc ttgaccattg caggttggtt tatgactgtt 180
   gagggagaga ctggctcgtg gccgacaatc aatgaagcta tgtctgaatt tagcgtgtca 240
   cgtcagaccg tgaatagagc acttaaggtc tgcgggcatt gaacttccac gaggacgccg 300
   aaagcttccc agtaaatgtg ccatctcgta ggcagaaaac ggttcccccg tagggtctct 360
   ctcttggcct cctttctagg tcgggctgat tgctcttgaa gctctctagg ggggctcaca 420
   ccataggcag ataacgttcc ccaccggctc gcctcgtaag cgcacaagga ctgctcccaa 480
   agatcttcaa agccactgcc gcgactgcct tcgcgaagcc ttgccccgcg gaaatttcct 540
   ccaccgagtt cgtgcacacc cctatgccaa gcttctttca ccctaaattc gagagattgg 600
   attcttaccg tggaaattct tcgcaaaaat cgtcccctga tcgcccttgc gacgttggcg 660
   tcggtgccgc tggttgcgct tggcttgacc gacttgatca gcggccgctc gatttaaatc 720
   tcgagcggct taaagtttgg ctgccatgtg aatttttagc accctcaaca gttgagtgct 780
   ggcactctcg ggggtagagt gccaaatagg ttgtttgaca cacagttgtt cacccgcgac 840
   gacggctgtg ctggaaaccc acaaccggca cacacaaaat ttttctcatg gagagattgc 900
   atatgcccac cctcgcgcct tcaggtcaac ttgaaatcca agcgatcggt gatgtctcca 960
   ccgaagccgg agcaatcatt acaaacgctg aaatcgccta tcaccgctgg ggtgaatacc 1020
   gcgtagataa agaaggacgc agcaatgtcg ttctcatcga acacgccctc actggagatt 1080
   ccaacgcagc cgattggtgg gctgacttgc tcggtcccgg caaagccatc aacactgata 1140
   tttactgcgt gatctgtacc aacgtcatcg gtggttgcaa cggttccacc ggacctggct 1200
   ccatgcatcc agatggaaat ttctggggta atcgcttccc cgccacgtcc attcgtgatc 1260
   aggtaaacgc cgaaaaacaa ttcctcgacg cactcggcat caccacggtc gccgcagtac 1320
   ttggtggttc catgggtggt gcccgcaccc tagagtgggc cgcaatgtac ccagaaactg 1380
   ttggcgcagc tgctgttctt gcagtttctg cacgcgccag cgcctggcaa atcggcattc 1440
   aatccgccca aattaaggcg attgaaaacg accaccactg gcacgaaggc aactactacg 1500
   aatccggctg caacccagcc accggactcg gcgccgcccg acgcatcgcc cacctcacct 1560
   accgtggcga actagaaatc gacgaacgct tcggcaccaa agcccaaaag aacgaaaacc 1620
   cactcggtcc ctaccgcaag cccgaccagc gcttcgccgt ggaatcctac ttggactacc 1680
   aagcagacaa gctagtacag cgtttcgacg ccggctccta cgtcttgctc accgacgccc 1740
   tcaaccgcca cgacattggt cgcgaccgcg gaggcctcaa caaggcactc gaatccatca 1800
   aagttccagt ccttgtcgca ggcgtagata ccgatatttt gtacccctac caccagcaag 1860
   aacacctctc cagaaacctg ggaaatctac tggcaatggc aaaaatcgta tcccctgtcg 1920
   gccacgatgc tttcctcacc gaaagccgcc aaatggatcg catcgtgagg aacttcttca 1980
   gcctcatctc cccagacgaa gacaaccctt cgacctacat cgagttctac atctaaacta 2040
   gttcggacct agggatatcg tcgacatcga tgctcttctg cgttaattaa caattgggat 2100
   cctctagagt tctgtgaaaa acaccgtggg gcagtttctg cttcgcggtg ttttttattt 2160
   gtggggcact agacccggga tttaaatcgc tagcgggctg ctaaaggaag cggaacacgt 2220
   agaaagccag tccgcagaaa cggtgctgac cccggatgaa tgtcagctac tgggctatct 2280
   ggacaaggga aaacgcaagc gcaaagagaa agcaggtagc ttgcagtggg cttacatggc 2340
   gatagctaga ctgggcggtt ttatggacag caagcgaacc ggaattgcca gctggggcgc 2400
   cctctggtaa ggttgggaag ccctgcaaag taaactggat ggctttcttg ccgccaagga 2460
   tctgatggcg caggggatca agatctgatc aagagacagg atgaggatcg tttcgcatga 2520
   ttgaacaaga tggattgcac gcaggttctc cggccgcttg ggtggagagg ctattcggct 2580
   atgactgggc acaacagaca atcggctgct ctgatgccgc cgtgttccgg ctgtcagcgc 2640
   aggggcgccc ggttcttttt gtcaagaccg acctgtccgg tgccctgaat gaactgcagg 2700
   acgaggcagc gcggctatcg tggctggcca cgacgggcgt tccttgcgca gctgtgctcg 2760
   acgttgtcac tgaagcggga agggactggc tgctattggg cgaagtgccg gggcaggatc 2820
   tcctgtcatc tcaccttgct cctgccgaga aagtatccat catggctgat gcaatgcggc 2880
   ggctgcatac gcttgatccg gctacctgcc cattcgacca ccaagcgaaa catcgcatcg 2940
   agcgagcacg tactcggatg gaagccggtc ttgtcgatca ggatgatctg gacgaagagc 3000
   atcaggggct cgcgccagcc gaactgttcg ccaggctcaa ggcgcgcatg cccgacggcg 3060
   aggatctcgt cgtgacccat ggcgatgcct gcttgccgaa tatcatggtg gaaaatggcc 3120
   gcttttctgg attcatcgac tgtggccggc tgggtgtggc ggaccgctat caggacatag 3180
   cgttggctac ccgtgatatt gctgaagagc ttggcggcga atgggctgac cgcttcctcg 3240
   tgctttacgg tatcgccgct cccgattcgc agcgcatcgc cttctatcgc cttcttgacg 3300
   agttcttctg agcgggactc tggggttcga aatgaccgac caagcgacgc ccaacctgcc 3360
   atcacgagat ttcgattcca ccgccgcctt ctatgaaagg ttgggcttcg gaatcgtttt 3420
   ccgggacgcc ggctggatga tcctccagcg cggggatctc atgctggagt tcttcgccca 3480
   cgctagcggc gcgccggccg gcccggtgtg aaataccgca cagatgcgta aggagaaaat 3540
   accgcatcag gcgctcttcc gcttcctcgc tcactgactc gctgcgctcg gtcgttcggc 3600
   tgcggcgagc ggtatcagct cactcaaagg cggtaatacg gttatccaca gaatcagggg 3660
   ataacgcagg aaagaacatg tgagcaaaag gccagcaaaa ggccaggaac cgtaaaaagg 3720
   ccgcgttgct ggcgtttttc cataggctcc gcccccctga cgagcatcac aaaaatcgac 3780
   gctcaagtca gaggtggcga aacccgacag gactataaag ataccaggcg tttccccctg 3840
   gaagctccct cgtgcgctct cctgttccga ccctgccgct taccggatac ctgtccgcct 3900
   ttctcccttc gggaagcgtg gcgctttctc atagctcacg ctgtaggtat ctcagttcgg 3960
   tgtaggtcgt tcgctccaag ctgggctgtg tgcacgaacc ccccgttcag cccgaccgct 4020
   gcgccttatc cggtaactat cgtcttgagt ccaacccggt aagacacgac ttatcgccac 4080
   tggcagcagc cactggtaac aggattagca gagcgaggta tgtaggcggt gctacagagt 4140
   tcttgaagtg gtggcctaac tacggctaca ctagaaggac agtatttggt atctgcgctc 4200
   tgctgaagcc agttaccttc ggaaaaagag ttggtagctc ttgatccggc aaacaaacca 4260
   ccgctggtag cggtggtttt tttgtttgca agcagcagat tacgcgcaga aaaaaaggat 4320
   ctcaagaaga tcctttgatc ttttctacgg ggtctgacgc tcagtggaac gaaaactcac 4380
   gttaagggat tttggtcatg agattatcaa aaaggatctt cacctagatc cttttaaagg 4440
   ccggccgcgg ccgcgcaaag tcccgcttcg tgaaaatttt cgtgccgcgt gattttccgc 4500
   caaaaacttt aacgaacgtt cgttataatg gtgtcatgac cttcacgacg aagtactaaa 4560
   attggcccga atcatcagct atggatctct ctgatgtcgc gctggagtcc gacgcgctcg 4620
   atgctgccgt cgatttaaaa acggtgatcg gatttttccg agctctcgat acgacggacg 4680
   cgccagcatc acgagactgg gccagtgccg cgagcgacct agaaactctc gtggcggatc 4740
   ttgaggagct ggctgacgag ctgcgtgctc ggccagcgcc aggaggacgc acagtagtgg 4800
   aggatgcaat cagttgcgcc tactgcggtg gcctgattcc tccccggcct gacccgcgag 4860
   gacggcgcgc aaaatattgc tcagatgcgt gtcgtgccgc agccagccgc gagcgcgcca 4920
   acaaacgcca cgccgaggag ctggaggcgg ctaggtcgca aatggcgctg gaagtgcgtc 4980
   ccccgagcga aattttggcc atggtcgtca cagagctgga agcggcagcg agaattatcg 5040
   cgatcgtggc ggtgcccgca ggcatgacaa acatcgtaaa tgccgcgttt cgtgtgccgt 5100
   ggccgcccag gacgtgtcag cgccgccacc acctgcaccg aatcggcagc agcgtcgcgc 5160
   gtcgaaaaag cgcacaggcg gcaagaagcg ataagctgca cgaatacctg aaaaatgttg 5220
   aacgccccgt gagcggtaac tcacagggcg tcggctaacc cccagtccaa acctgggaga 5280
   aagcgctcaa aaatgactct agcggattca cgagacattg acacaccggc ctggaaattt 5340
   tccgctgatc tgttcgacac ccatcccgag ctcgcgctgc gatcacgtgg ctggacgagc 5400
   gaagaccgcc gcgaattcct cgctcacctg ggcagagaaa atttccaggg cagcaagacc 5460
   cgcgacttcg ccagcgcttg gatcaaagac ccggacacgg agaaacacag ccgaagttat 5520
   accgagttgg ttcaaaatcg cttgcccggt gccagtatgt tgctctgacg cacgcgcagc 5580
   acgcagccgt gcttgtcctg gacattgatg tgccgagcca ccaggccggc gggaaaatcg 5640
   agcacgtaaa ccccgaggtc tacgcgattt tggagcgctg ggcacgcctg gaaaaagcgc 5700
   cagcttggat cggcgtgaat ccactgagcg ggaaatgcca gctcatctgg ctcattgatc 5760
   cggtgtatgc cgcagcaggc atgagcagcc cgaatatgcg cctgctggct gcaacgaccg 5820
   aggaaatgac ccgcgttttc ggcgctgacc aggctttttc acataggctg agccgtggcc 5880
   actgcactct ccgacgatcc cagccgtacc gctggcatgc ccagcacaat cgcgtggatc 5940
   gcctagctga tcttatggag gttgctcgca tgatctcagg cacagaaaaa cctaaaaaac 6000
   gctatgagca ggagttttct agcggacggg cacgtatcga agcggcaaga aaagccactg 6060
   cggaagcaaa agcacttgcc acgcttgaag caagcctgcc gagcgccgct gaagcgtctg 6120
   gagagctgat cgacggcgtc cgtgtcctct ggactgctcc agggcgtgcc gcccgtgatg 6180
   agacggcttt tcgccacgct ttgactgtgg gataccagtt aaaagcggct ggtgagcgcc 6240
   taaaagacac caagggtcat cgagcctacg agcgtgccta caccgtcgct caggcggtcg 6300
   gaggaggccg tgagcctgat ctgccgccgg actgtgaccg ccagacggat tggccgcgac 6360
   gtgtgcgcgg ctacgtcgct aaaggccagc cagtcgtccc tgctcgtcag acagagacgc 6420
   agagccagcc g                                                      6431
<210> 48
   <211> 6431
   <212> DNA
   <213> Corynebacterium glutamicum
<220>
   <223> SEQ_ID_48_PCLIK5MCS_PGRO_1701_1833_META
<220>
   <221> misc_feature
   <222> (726 )..(899 )
   <223> P1701/1833
<220>
   <221> misc_feature
   <222> (906 )..(2036 )
   <223> MetA without startcodon
<220>
   <221> misc_feature
   <222> (2104 )..(2173 )
   <223> GroEL Terminator
<220>
   <221> misc_feature
   <222> (2517 )..(3308 )
   <223> KanR
<220>
   <221> misc_feature
   <222> (3575 )..(4435 )
   <223> Ori-EC(pMB) complement
<220>
   <221> misc_feature
   <222> (4581 )..(5255 )
   <223> Orf1
<220>
   <221> misc_feature
   <222> (5589 )..(279 )
   <223> REP Protein
<400> 48
   aggcgaaaag ctctggccac tatgggaaga cgtggcggta aaaaggccgc agaacgctgg 60
   aaagacccaa acagtgagta cgcccgagca cagcgagaaa aactagctaa gtccagtcaa 120
   cgacaagcta ggaaagctaa aggaaatcgc ttgaccattg caggttggtt tatgactgtt 180
   gagggagaga ctggctcgtg gccgacaatc aatgaagcta tgtctgaatt tagcgtgtca 240
   cgtcagaccg tgaatagagc acttaaggtc tgcgggcatt gaacttccac gaggacgccg 300
   aaagcttccc agtaaatgtg ccatctcgta ggcagaaaac ggttcccccg tagggtctct 360
   ctcttggcct cctttctagg tcgggctgat tgctcttgaa gctctctagg ggggctcaca 420
   ccataggcag ataacgttcc ccaccggctc gcctcgtaag cgcacaagga ctgctcccaa 480
   agatcttcaa agccactgcc gcgactgcct tcgcgaagcc ttgccccgcg gaaatttcct 540
   ccaccgagtt cgtgcacacc cctatgccaa gcttctttca ccctaaattc gagagattgg 600
   attcttaccg tggaaattct tcgcaaaaat cgtcccctga tcgcccttgc gacgttggcg 660
   tcggtgccgc tggttgcgct tggcttgacc gacttgatca gcggccgctc gatttaaatc 720
   tcgagcggct taaagtttgg ctgccatgtg aatttttagc accctcaaca gttgagtgct 780
   ggcactctcg ggggtagagt gccaaatagg ttgtttgaca cacagttgtt cacccgcgac 840
   gacggctgtg ctggaaaccc acaaccggca cacacaaaat ttttctcatg aggagattgc 900
   atatgcccac cctcgcgcct tcaggtcaac ttgaaatcca agcgatcggt gatgtctcca 960
   ccgaagccgg agcaatcatt acaaacgctg aaatcgccta tcaccgctgg ggtgaatacc 1020
   gcgtagataa agaaggacgc agcaatgtcg ttctcatcga acacgccctc actggagatt 1080
   ccaacgcagc cgattggtgg gctgacttgc tcggtcccgg caaagccatc aacactgata 1140
   tttactgcgt gatctgtacc aacgtcatcg gtggttgcaa cggttccacc ggacctggct 1200
   ccatgcatcc agatggaaat ttctggggta atcgcttccc cgccacgtcc attcgtgatc 1260
   aggtaaacgc cgaaaaacaa ttcctcgacg cactcggcat caccacggtc gccgcagtac 1320
   ttggtggttc catgggtggt gcccgcaccc tagagtgggc cgcaatgtac ccagaaactg 1380
   ttggcgcagc tgctgttctt gcagtttctg cacgcgccag cgcctggcaa atcggcattc 1440
   aatccgccca aattaaggcg attgaaaacg accaccactg gcacgaaggc aactactacg 1500
   aatccggctg caacccagcc accggactcg gcgccgcccg acgcatcgcc cacctcacct 1560
   accgtggcga actagaaatc gacgaacgct tcggcaccaa agcccaaaag aacgaaaacc 1620
   cactcggtcc ctaccgcaag cccgaccagc gcttcgccgt ggaatcctac ttggactacc 1680
   aagcagacaa gctagtacag cgtttcgacg ccggctccta cgtcttgctc accgacgccc 1740
   tcaaccgcca cgacattggt cgcgaccgcg gaggcctcaa caaggcactc gaatccatca 1800
   aagttccagt ccttgtcgca ggcgtagata ccgatatttt gtacccctac caccagcaag 1860
   aacacctctc cagaaacctg ggaaatctac tggcaatggc aaaaatcgta tcccctgtcg 1920
   gccacgatgc tttcctcacc gaaagccgcc aaatggatcg catcgtgagg aacttcttca 1980
   gcctcatctc cccagacgaa gacaaccctt cgacctacat cgagttctac atctaaacta 2040
   gttcggacct agggatatcg tcgacatcga tgctcttctg cgttaattaa caattgggat 2100
   cctctagagt tctgtgaaaa acaccgtggg gcagtttctg cttcgcggtg ttttttattt 2160
   gtggggcact agacccggga tttaaatcgc tagcgggctg ctaaaggaag cggaacacgt 2220
   agaaagccag tccgcagaaa cggtgctgac cccggatgaa tgtcagctac tgggctatct 2280
   ggacaaggga aaacgcaagc gcaaagagaa agcaggtagc ttgcagtggg cttacatggc 2340
   gatagctaga ctgggcggtt ttatggacag caagcgaacc ggaattgcca gctggggcgc 2400
   cctctggtaa ggttgggaag ccctgcaaag taaactggat ggctttcttg ccgccaagga 2460
   tctgatggcg caggggatca agatctgatc aagagacagg atgaggatcg tttcgcatga 2520
   ttgaacaaga tggattgcac gcaggttctc cggccgcttg ggtggagagg ctattcggct 2580
   atgactgggc acaacagaca atcggctgct ctgatgccgc cgtgttccgg ctgtcagcgc 2640
   aggggcgccc ggttcttttt gtcaagaccg acctgtccgg tgccctgaat gaactgcagg 2700
   acgaggcagc gcggctatcg tggctggcca cgacgggcgt tccttgcgca gctgtgctcg 2760
   acgttgtcac tgaagcggga agggactggc tgctattggg cgaagtgccg gggcaggatc 2820
   tcctgtcatc tcaccttgct cctgccgaga aagtatccat catggctgat gcaatgcggc 2880
   ggctgcatac gcttgatccg gctacctgcc cattcgacca ccaagcgaaa catcgcatcg 2940
   agcgagcacg tactcggatg gaagccggtc ttgtcgatca ggatgatctg gacgaagagc 3000
   atcaggggct cgcgccagcc gaactgttcg ccaggctcaa ggcgcgcatg cccgacggcg 3060
   aggatctcgt cgtgacccat ggcgatgcct gcttgccgaa tatcatggtg gaaaatggcc 3120
   gcttttctgg attcatcgac tgtggccggc tgggtgtggc ggaccgctat caggacatag 3180
   cgttggctac ccgtgatatt gctgaagagc ttggcggcga atgggctgac cgcttcctcg 3240
   tgctttacgg tatcgccgct cccgattcgc agcgcatcgc cttctatcgc cttcttgacg 3300
   agttcttctg agcgggactc tggggttcga aatgaccgac caagcgacgc ccaacctgcc 3360
   atcacgagat ttcgattcca ccgccgcctt ctatgaaagg ttgggcttcg gaatcgtttt 3420
   ccgggacgcc ggctggatga tcctccagcg cggggatctc atgctggagt tcttcgccca 3480
   cgctagcggc gcgccggccg gcccggtgtg aaataccgca cagatgcgta aggagaaaat 3540
   accgcatcag gcgctcttcc gcttcctcgc tcactgactc gctgcgctcg gtcgttcggc 3600
   tgcggcgagc ggtatcagct cactcaaagg cggtaatacg gttatccaca gaatcagggg 3660
   ataacgcagg aaagaacatg tgagcaaaag gccagcaaaa ggccaggaac cgtaaaaagg 3720
   ccgcgttgct ggcgtttttc cataggctcc gcccccctga cgagcatcac aaaaatcgac 3780
   gctcaagtca gaggtggcga aacccgacag gactataaag ataccaggcg tttccccctg 3840
   gaagctccct cgtgcgctct cctgttccga ccctgccgct taccggatac ctgtccgcct 3900
   ttctcccttc gggaagcgtg gcgctttctc atagctcacg ctgtaggtat ctcagttcgg 3960
   tgtaggtcgt tcgctccaag ctgggctgtg tgcacgaacc ccccgttcag cccgaccgct 4020
   gcgccttatc cggtaactat cgtcttgagt ccaacccggt aagacacgac ttatcgccac 4080
   tggcagcagc cactggtaac aggattagca gagcgaggta tgtaggcggt gctacagagt 4140
   tcttgaagtg gtggcctaac tacggctaca ctagaaggac agtatttggt atctgcgctc 4200
   tgctgaagcc agttaccttc ggaaaaagag ttggtagctc ttgatccggc aaacaaacca 4260
   ccgctggtag cggtggtttt tttgtttgca agcagcagat tacgcgcaga aaaaaaggat 4320
   ctcaagaaga tcctttgatc ttttctacgg ggtctgacgc tcagtggaac gaaaactcac 4380
   gttaagggat tttggtcatg agattatcaa aaaggatctt cacctagatc cttttaaagg 4440
   ccggccgcgg ccgcgcaaag tcccgcttcg tgaaaatttt cgtgccgcgt gattttccgc 4500
   caaaaacttt aacgaacgtt cgttataatg gtgtcatgac cttcacgacg aagtactaaa 4560
   attggcccga atcatcagct atggatctct ctgatgtcgc gctggagtcc gacgcgctcg 4620
   atgctgccgt cgatttaaaa acggtgatcg gatttttccg agctctcgat acgacggacg 4680
   cgccagcatc acgagactgg gccagtgccg cgagcgacct agaaactctc gtggcggatc 4740
   ttgaggagct ggctgacgag ctgcgtgctc ggccagcgcc aggaggacgc acagtagtgg 4800
   aggatgcaat cagttgcgcc tactgcggtg gcctgattcc tccccggcct gacccgcgag 4860
   gacggcgcgc aaaatattgc tcagatgcgt gtcgtgccgc agccagccgc gagcgcgcca 4920
   acaaacgcca cgccgaggag ctggaggcgg ctaggtcgca aatggcgctg gaagtgcgtc 4980
   ccccgagcga aattttggcc atggtcgtca cagagctgga agcggcagcg agaattatcg 5040
   cgatcgtggc ggtgcccgca ggcatgacaa acatcgtaaa tgccgcgttt cgtgtgccgt 5100
   ggccgcccag gacgtgtcag cgccgccacc acctgcaccg aatcggcagc agcgtcgcgc 5160
   gtcgaaaaag cgcacaggcg gcaagaagcg ataagctgca cgaatacctg aaaaatgttg 5220
   aacgccccgt gagcggtaac tcacagggcg tcggctaacc cccagtccaa acctgggaga 5280
   aagcgctcaa aaatgactct agcggattca cgagacattg acacaccggc ctggaaattt 5340
   tccgctgatc tgttcgacac ccatcccgag ctcgcgctgc gatcacgtgg ctggacgagc 5400
   gaagaccgcc gcgaattcct cgctcacctg ggcagagaaa atttccaggg cagcaagacc 5460
   cgcgacttcg ccagcgcttg gatcaaagac ccggacacgg agaaacacag ccgaagttat 5520
   accgagttgg ttcaaaatcg cttgcccggt gccagtatgt tgctctgacg cacgcgcagc 5580
   acgcagccgt gcttgtcctg gacattgatg tgccgagcca ccaggccggc gggaaaatcg 5640
   agcacgtaaa ccccgaggtc tacgcgattt tggagcgctg ggcacgcctg gaaaaagcgc 5700
   cagcttggat cggcgtgaat ccactgagcg ggaaatgcca gctcatctgg ctcattgatc 5760
   cggtgtatgc cgcagcaggc atgagcagcc cgaatatgcg cctgctggct gcaacgaccg 5820
   aggaaatgac ccgcgttttc ggcgctgacc aggctttttc acataggctg agccgtggcc 5880
   actgcactct ccgacgatcc cagccgtacc gctggcatgc ccagcacaat cgcgtggatc 5940
   gcctagctga tcttatggag gttgctcgca tgatctcagg cacagaaaaa cctaaaaaac 6000
   gctatgagca ggagttttct agcggacggg cacgtatcga agcggcaaga aaagccactg 6060
   cggaagcaaa agcacttgcc acgcttgaag caagcctgcc gagcgccgct gaagcgtctg 6120
   gagagctgat cgacggcgtc cgtgtcctct ggactgctcc agggcgtgcc gcccgtgatg 6180
   agacggcttt tcgccacgct ttgactgtgg gataccagtt aaaagcggct ggtgagcgcc 6240
   taaaagacac caagggtcat cgagcctacg agcgtgccta caccgtcgct caggcggtcg 6300
   gaggaggccg tgagcctgat ctgccgccgg actgtgaccg ccagacggat tggccgcgac 6360
   gtgtgcgcgg ctacgtcgct aaaggccagc cagtcgtccc tgctcgtcag acagagacgc 6420
   agagccagcc g 6431
<210> 49
   <211> 6431
   <212> DNA
   <213> Corynebacterium glutamicum
<220>
   <223> SEQ_ID_49_PCLIK5MCS_PGRO_1701_1834_META
<220>
   <221> misc_feature
   <222> (726 )..(899 )
   <223> P1701/1834
<220>
   <221> misc_feature
   <222> (906 )..(2036 )
   <223> MetA without startcodon
<220>
   <221> misc_feature
   <222> (2104 )..(2173 )
   <223> GroEL Terminator
<220>
   <221> misc_feature
   <222> (2517 )..(3308 )
   <223> KanR
<220>
   <221> misc_feature
   <222> (3575 )..(4435 )
   <223> Ori-Ec(pMB) complement
<220>
   <221> misc_feature
   <222> (4581 )..(5255 )
   <223> Orf1
<220>
   <221> misc_feature
   <222> (5589 )..(279 )
   <223> REP Protein
<400> 49
   aggcgaaaag ctctggccac tatgggaaga cgtggcggta aaaaggccgc agaacgctgg 60
   aaagacccaa acagtgagta cgcccgagca cagcgagaaa aactagctaa gtccagtcaa 120
   cgacaagcta ggaaagctaa aggaaatcgc ttgaccattg caggttggtt tatgactgtt 180
   gagggagaga ctggctcgtg gccgacaatc aatgaagcta tgtctgaatt tagcgtgtca 240
   cgtcagaccg tgaatagagc acttaaggtc tgcgggcatt gaacttccac gaggacgccg 300
   aaagcttccc agtaaatgtg ccatctcgta ggcagaaaac ggttcccccg tagggtctct 360
   ctcttggcct cctttctagg tcgggctgat tgctcttgaa gctctctagg ggggctcaca 420
   ccataggcag ataacgttcc ccaccggctc gcctcgtaag cgcacaagga ctgctcccaa 480
   agatcttcaa agccactgcc gcgactgcct tcgcgaagcc ttgccccgcg gaaatttcct 540
   ccaccgagtt cgtgcacacc cctatgccaa gcttctttca ccctaaattc gagagattgg 600
   attcttaccg tggaaattct tcgcaaaaat cgtcccctga tcgcccttgc gacgttggcg 660
   tcggtgccgc tggttgcgct tggcttgacc gacttgatca gcggccgctc gatttaaatc 720
   tcgagcggct taaagtttgg ctgccatgtg aatttttagc accctcaaca gttgagtgct 780
   ggcactctcg ggggtagagt gccaaatagg ttgtttgaca cacagttgtt cacccgcgac 840
   gacggctgtg ctggaaaccc acaaccggca cacacaaaat ttttctcatg aagggattgc 900
   atatgcccac cctcgcgcct tcaggtcaac ttgaaatcca agcgatcggt gatgtctcca 960
   ccgaagccgg agcaatcatt acaaacgctg aaatcgccta tcaccgctgg ggtgaatacc 1020
   gcgtagataa agaaggacgc agcaatgtcg ttctcatcga acacgccctc actggagatt 1080
   ccaacgcagc cgattggtgg gctgacttgc tcggtcccgg caaagccatc aacactgata 1140
   tttactgcgt gatctgtacc aacgtcatcg gtggttgcaa cggttccacc ggacctggct 1200
   ccatgcatcc agatggaaat ttctggggta atcgcttccc cgccacgtcc attcgtgatc 1260
   aggtaaacgc cgaaaaacaa ttcctcgacg cactcggcat caccacggtc gccgcagtac 1320
   ttggtggttc catgggtggt gcccgcaccc tagagtgggc cgcaatgtac ccagaaactg 1380
   ttggcgcagc tgctgttctt gcagtttctg cacgcgccag cgcctggcaa atcggcattc 1440
   aatccgccca aattaaggcg attgaaaacg accaccactg gcacgaaggc aactactacg 1500
   aatccggctg caacccagcc accggactcg gcgccgcccg acgcatcgcc cacctcacct 1560
   accgtggcga actagaaatc gacgaacgct tcggcaccaa agcccaaaag aacgaaaacc 1620
   cactcggtcc ctaccgcaag cccgaccagc gcttcgccgt ggaatcctac ttggactacc 1680
   aagcagacaa gctagtacag cgtttcgacg ccggctccta cgtcttgctc accgacgccc 1740
   tcaaccgcca cgacattggt cgcgaccgcg gaggcctcaa caaggcactc gaatccatca 1800
   aagttccagt ccttgtcgca ggcgtagata ccgatatttt gtacccctac caccagcaag 1860
   aacacctctc cagaaacctg ggaaatctac tggcaatggc aaaaatcgta tcccctgtcg 1920
   gccacgatgc tttcctcacc gaaagccgcc aaatggatcg catcgtgagg aacttcttca 1980
   gcctcatctc cccagacgaa gacaaccctt cgacctacat cgagttctac atctaaacta 2040
   gttcggacct agggatatcg tcgacatcga tgctcttctg cgttaattaa caattgggat 2100
   cctctagagt tctgtgaaaa acaccgtggg gcagtttctg cttcgcggtg ttttttattt 2160
   gtggggcact agacccggga tttaaatcgc tagcgggctg ctaaaggaag cggaacacgt 2220
   agaaagccag tccgcagaaa cggtgctgac cccggatgaa tgtcagctac tgggctatct 2280
   ggacaaggga aaacgcaagc gcaaagagaa agcaggtagc ttgcagtggg cttacatggc 2340
   gatagctaga ctgggcggtt ttatggacag caagcgaacc ggaattgcca gctggggcgc 2400
   cctctggtaa ggttgggaag ccctgcaaag taaactggat ggctttcttg ccgccaagga 2460
   tctgatggcg caggggatca agatctgatc aagagacagg atgaggatcg tttcgcatga 2520
   ttgaacaaga tggattgcac gcaggttctc cggccgcttg ggtggagagg ctattcggct 2580
   atgactgggc acaacagaca atcggctgct ctgatgccgc cgtgttccgg ctgtcagcgc 2640
   aggggcgccc ggttcttttt gtcaagaccg acctgtccgg tgccctgaat gaactgcagg 2700
   acgaggcagc gcggctatcg tggctggcca cgacgggcgt tccttgcgca gctgtgctcg 2760
   acgttgtcac tgaagcggga agggactggc tgctattggg cgaagtgccg gggcaggatc 2820
   tcctgtcatc tcaccttgct cctgccgaga aagtatccat catggctgat gcaatgcggc 2880
   ggctgcatac gcttgatccg gctacctgcc cattcgacca ccaagcgaaa catcgcatcg 2940
   agcgagcacg tactcggatg gaagccggtc ttgtcgatca ggatgatctg gacgaagagc 3000
   atcaggggct cgcgccagcc gaactgttcg ccaggctcaa ggcgcgcatg cccgacggcg 3060
   aggatctcgt cgtgacccat ggcgatgcct gcttgccgaa tatcatggtg gaaaatggcc 3120
   gcttttctgg attcatcgac tgtggccggc tgggtgtggc ggaccgctat caggacatag 3180
   cgttggctac ccgtgatatt gctgaagagc ttggcggcga atgggctgac cgcttcctcg 3240
   tgctttacgg tatcgccgct cccgattcgc agcgcatcgc cttctatcgc cttcttgacg 3300
   agttcttctg agcgggactc tggggttcga aatgaccgac caagcgacgc ccaacctgcc 3360
   atcacgagat ttcgattcca ccgccgcctt ctatgaaagg ttgggcttcg gaatcgtttt 3420
   ccgggacgcc ggctggatga tcctccagcg cggggatctc atgctggagt tcttcgccca 3480
   cgctagcggc gcgccggccg gcccggtgtg aaataccgca cagatgcgta aggagaaaat 3540
   accgcatcag gcgctcttcc gcttcctcgc tcactgactc gctgcgctcg gtcgttcggc 3600
   tgcggcgagc ggtatcagct cactcaaagg cggtaatacg gttatccaca gaatcagggg 3660
   ataacgcagg aaagaacatg tgagcaaaag gccagcaaaa ggccaggaac cgtaaaaagg 3720
   ccgcgttgct ggcgtttttc cataggctcc gcccccctga cgagcatcac aaaaatcgac 3780
   gctcaagtca gaggtggcga aacccgacag gactataaag ataccaggcg tttccccctg 3840
   gaagctccct cgtgcgctct cctgttccga ccctgccgct taccggatac ctgtccgcct 3900
   ttctcccttc gggaagcgtg gcgctttctc atagctcacg ctgtaggtat ctcagttcgg 3960
   tgtaggtcgt tcgctccaag ctgggctgtg tgcacgaacc ccccgttcag cccgaccgct 4020
   gcgccttatc cggtaactat cgtcttgagt ccaacccggt aagacacgac ttatcgccac 4080
   tggcagcagc cactggtaac aggattagca gagcgaggta tgtaggcggt gctacagagt 4140
   tcttgaagtg gtggcctaac tacggctaca ctagaaggac agtatttggt atctgcgctc 4200
   tgctgaagcc agttaccttc ggaaaaagag ttggtagctc ttgatccggc aaacaaacca 4260
   ccgctggtag cggtggtttt tttgtttgca agcagcagat tacgcgcaga aaaaaaggat 4320
   ctcaagaaga tcctttgatc ttttctacgg ggtctgacgc tcagtggaac gaaaactcac 4380
   gttaagggat tttggtcatg agattatcaa aaaggatctt cacctagatc cttttaaagg 4440
   ccggccgcgg ccgcgcaaag tcccgcttcg tgaaaatttt cgtgccgcgt gattttccgc 4500
   caaaaacttt aacgaacgtt cgttataatg gtgtcatgac cttcacgacg aagtactaaa 4560
   attggcccga atcatcagct atggatctct ctgatgtcgc gctggagtcc gacgcgctcg 4620
   atgctgccgt cgatttaaaa acggtgatcg gatttttccg agctctcgat acgacggacg 4680
   cgccagcatc acgagactgg gccagtgccg cgagcgacct agaaactctc gtggcggatc 4740
   ttgaggagct ggctgacgag ctgcgtgctc ggccagcgcc aggaggacgc acagtagtgg 4800
   aggatgcaat cagttgcgcc tactgcggtg gcctgattcc tccccggcct gacccgcgag 4860
   gacggcgcgc aaaatattgc tcagatgcgt gtcgtgccgc agccagccgc gagcgcgcca 4920
   acaaacgcca cgccgaggag ctggaggcgg ctaggtcgca aatggcgctg gaagtgcgtc 4980
   ccccgagcga aattttggcc atggtcgtca cagagctgga agcggcagcg agaattatcg 5040
   cgatcgtggc ggtgcccgca ggcatgacaa acatcgtaaa tgccgcgttt cgtgtgccgt 5100
   ggccgcccag gacgtgtcag cgccgccacc acctgcaccg aatcggcagc agcgtcgcgc 5160
   gtcgaaaaag cgcacaggcg gcaagaagcg ataagctgca cgaatacctg aaaaatgttg 5220
   aacgccccgt gagcggtaac tcacagggcg tcggctaacc cccagtccaa acctgggaga 5280
   aagcgctcaa aaatgactct agcggattca cgagacattg acacaccggc ctggaaattt 5340
   tccgctgatc tgttcgacac ccatcccgag ctcgcgctgc gatcacgtgg ctggacgagc 5400
   gaagaccgcc gcgaattcct cgctcacctg ggcagagaaa atttccaggg cagcaagacc 5460
   cgcgacttcg ccagcgcttg gatcaaagac ccggacacgg agaaacacag ccgaagttat 5520
   accgagttgg ttcaaaatcg cttgcccggt gccagtatgt tgctctgacg cacgcgcagc 5580
   acgcagccgt gcttgtcctg gacattgatg tgccgagcca ccaggccggc gggaaaatcg 5640
   agcacgtaaa ccccgaggtc tacgcgattt tggagcgctg ggcacgcctg gaaaaagcgc 5700
   cagcttggat cggcgtgaat ccactgagcg ggaaatgcca gctcatctgg ctcattgatc 5760
   cggtgtatgc cgcagcaggc atgagcagcc cgaatatgcg cctgctggct gcaacgaccg 5820
   aggaaatgac ccgcgttttc ggcgctgacc aggctttttc acataggctg agccgtggcc 5880
   actgcactct ccgacgatcc cagccgtacc gctggcatgc ccagcacaat cgcgtggatc 5940
   gcctagctga tcttatggag gttgctcgca tgatctcagg cacagaaaaa cctaaaaaac 6000
   gctatgagca ggagttttct agcggacggg cacgtatcga agcggcaaga aaagccactg 6060
   cggaagcaaa agcacttgcc acgcttgaag caagcctgcc gagcgccgct gaagcgtctg 6120
   gagagctgat cgacggcgtc cgtgtcctct ggactgctcc agggcgtgcc gcccgtgatg 6180
   agacggcttt tcgccacgct ttgactgtgg gataccagtt aaaagcggct ggtgagcgcc 6240
   taaaagacac caagggtcat cgagcctacg agcgtgccta caccgtcgct caggcggtcg 6300
   gaggaggccg tgagcctgat ctgccgccgg actgtgaccg ccagacggat tggccgcgac 6360
   gtgtgcgcgg ctacgtcgct aaaggccagc cagtcgtccc tgctcgtcag acagagacgc 6420
   agagccagcc g 6431
<210> 50
   <211> 1005
   <212> DNA
   <213> Corynebacterium glutamicum
<220>
   <223> SEQ_ID_50_FRUCTOSE_1_6_BISPHOSPHATASE
<400> 50
   atgaacctaa agaaccccga aacgccagac cgtaaccttg ctatggagct ggtgcgagtt 60
   acggaagcag ctgcactggc ttctggacgt tgggttggac gtggcatgaa gaatgaaggc 120
   gacggtgccg ctgttgacgc catgcgccag ctcatcaact cagtgaccat gaagggcgtc 180
   gttgttatcg gcgagggcga aaaagacgaa gctccaatgc tgtacaacgg cgaagaggtc 240
   ggaaccggct ttggacctga ggttgatatc gcagttgacc cagttgacgg caccaccctg 300
   atggctgagg gtcgccccaa cgcaatttcc attctcgcag ctgcagagcg tggcaccatg 360
   tacgatccat cctccgtctt ctacatgaag aagatcgccg tgggacctga ggccgcaggc 420
   aagatcgaca tcgaagctcc agttgcccac aacatcaacg cggtggcaaa gtccaaggga 480
   atcaaccctt ccgacgtcac cgttgtcgtg cttgaccgtc ctcgccacat cgaactgatc 540
   gcagacattc gtcgtgcagg cgcaaaggtt cgtctcatct ccgacggcga cgttgcaggt 600
   gcagttgcag cagctcagga ttccaactcc gtggacatca tgatgggcac cggcggaacc 660
   ccagaaggca tcatcactgc gtgcgccatg aagtgcatgg gtggcgaaat ccagggcatc 720
   ctggccccaa tgaacgattt cgagcgccag aaggcacacg acgctggtct ggttcttgat 780
   caggttctgc acaccaacga tctggtgagc tccgacaact gctacttcgt ggcaaccggt 840
   gtgaccaacg gtgacatgct ccgtggcgtt tcctaccgcg caaacggcgc aaccacccgt 900
   tccctggtta tgcgcgcaaa gtcaggcacc atccgccaca tcgagtctgt ccaccagctg 960
   tccaagctgc aggaatactc cgtggttgac tacaccaccg cgacc 1005
<210> 51
   <211> 335
   <212> PRT
   <213> Corynebacterium glutamicum
<400> 51
   Met Asn Leu Lys Asn Pro Glu Thr Pro Asp Arg Asn Leu Ala Met Glu
   1               5                   10                  15
   Leu Val Arg Val Thr Glu Ala Ala Ala Leu Ala Ser Gly Arg Trp Val
               20                  25                  30
   Gly Arg Gly Met Lys Asn Glu Gly Asp Gly Ala Ala Val Asp Ala Met
           35                  40                  45
   Arg Gln Leu Ile Asn Ser Val Thr Met Lys Gly Val Val Val Ile Gly
       50                  55                  60
   Glu Gly Glu Lys Asp Glu Ala Pro Met Leu Tyr Asn Gly Glu Glu Val
   65                  70                  75                  80
   Gly Thr Gly Phe Gly Pro Glu Val Asp Ile Ala Val Asp Pro Val Asp
                   85                  90                  95
   Gly Thr Thr Leu Met Ala Glu Gly Arg Pro Asn Ala Ile Ser Ile Leu
               100                 105                 110
   Ala Ala Ala Glu Arg Gly Thr Met Tyr Asp Pro Ser Ser Val Phe Tyr
           115                 120                 125
   Met Lys Lys Ile Ala Val Gly Pro Glu Ala Ala Gly Lys Ile Asp Ile
       130                 135                 140
   Glu Ala Pro Val Ala His Asn Ile Asn Ala Val Ala Lys Ser Lys Gly
   145                 150                 155                 160
   Ile Asn Pro Ser Asp Val Thr Val Val Val Leu Asp Arg Pro Arg His
                   165                 170                 175
   Ile Glu Leu Ile Ala Asp Ile Arg Arg Ala Gly Ala Lys Val Arg Leu
               180                 185                 190
   Ile Ser Asp Gly Asp Val Ala Gly Ala Val Ala Ala Ala Gln Asp Ser
           195                 200                 205
   Asn Ser Val Asp Ile Met Met Gly Thr Gly Gly Thr Pro Glu Gly Ile
       210                 215                 220
   Ile Thr Ala Cys Ala Met Lys Cys Met Gly Gly Glu Ile Gln Gly Ile
   225                 230                 235                 240
   Leu Ala Pro Met Asn Asp Phe Glu Arg Gln Lys Ala His Asp Ala Gly
                   245                 250                 255
   Leu Val Leu Asp Gln Val Leu His Thr Asn Asp Leu Val Ser Ser Asp
               260                 265                 270
   Asn Cys Tyr Phe Val Ala Thr Gly Val Thr Asn Gly Asp Met Leu Arg
           275                 280                 285
   Gly Val Ser Tyr Arg Ala Asn Gly Ala Thr Thr Arg Ser Leu Val Met
       290                 295                 300
   Arg Ala Lys Ser Gly Thr Ile Arg His Ile Glu Ser Val His Gln Leu
   305                 310                 315                 320
   Ser Lys Leu Gln Glu Tyr Ser Val Val Asp Tyr Thr Thr Ala Thr
                   325                 330                 335
<210> 52
   <211> 6
   <212> DNA
   <213> Corynebacterium glutamicum
<220>
   <223> SEQ_ID_52_POTENTIELLE_10_REGION_1
<400> 52
   tagagt 6
<210> 53
   <211> 7
   <212> DNA
   <213> Corynebacterium glutamicum
<220>
   <223> SEQ_ID_53_RIBOSOMALE_BINDUNGSSTELLE\SHINE_DALGARNO
<400> 53
   ggaggga 7
PF 55184
43
PF 55184

## Beschreibung

Die vorliegende Erfindung betrifft die Verwendung von Nukleinsäuresequenzen zur Regulation der Transkription und Expression von Genen, die neuen Promotoren und Expressionseinheiten selbst, Verfahren zur Veränderung oder Verursachung der Transkriptionsrate und/oder Expressionsrate von Genen, Expressionskasetten, enthaltend die Expressionseinheiten, genetisch veränderte Mikroorganismen mit veränderter oder verursachter Trankriptionsrate und/oder Expressionsrate sowie Verfahren zur Herstellung von biosynthetischen Produkten durch Kultivierung der genetisch veränderten Mikroorganismen.

Verschiedene biosynthetische Produkte, wie beispielsweise Feinchemikalien, wie unter anderem Aminosäuren, Vitamine aber auch Proteine werden über natürliche Stoffwechselprozesse in Zellen hergestellt und werden in vielen Industriezweigen verwendet, einschließlich der Nahrungsmittel-, Futtermittel-, Kosmetik-, Feed-, Food- und pharmazeutischen Industrie. Diese Substanzen, die zusammen als Feinchemikalien/Proteine bezeichnet werden, umfassen unter anderem organische Säuren, sowohl proteinogene als auch nicht-proteinogene Aminosäuren, Nukleotide und Nukleoside, Lipide und Fettsäuren, Diole, Kohlenhydrate, aromatische Verbindungen, Vitamine und Cofaktoren, sowie Proteine und Enzyme. Ihre Produktion erfolgt am zweckmäßigsten im Großmaßstab mittels Anzucht von Bakterien, die entwickelt wurden, um große Mengen der jeweils gewünschten Substanz zu produzieren und sezernieren. Für diesen Zweck besonders geeignete Organismen sind coryneforme Bakterien, grampositive nicht-pathogene Bakterien.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration während der Fermentation, oder die Aufarbeitung zum Produkt, beispielsweise durch lonenaustauschchromatographie aber auch Sprühtrocknung, oder die intrinsischen Leistungseigenschaften des Mikroorganismus selbst betreffen.

Seit einigen Jahren werden ebenfalls Methoden der rekombinanten DNA-Technik zur Stammverbesserung von Feinchemikalien/Proteine produzierender Stämme von Corynebacterium eingesetzt, indem man einzelne Gene amplifiziert und die Auswirkung auf die Produktion von Feinchemikalien/Proteine untersucht.

Andere Wege, um ein Verfahren für die Herstellung Feinchemikalien, Aminosäuren oder Proteine zu entwickeln, oder die Produktivität eines bereits existierenden Verfahrens für die Herstellung Feinchemikalien, Aminosäuren oder Proteine zu erhöhen bzw. zu verbessern, sind die Expression eines oder mehrerer Gene zu erhöhen bzw. zu verändern und oder die Translation einer mRNA durch geeignete Polynukleotidsequenzen zu beeinflussen. Beeinflussung kann in diesem Zusammenhang die Erhöhung, Verringerung, oder auch andere Parameter der Expression von Genen wie zeitliche Expressionsmuster umfassen.

Dem Fachmann sind unterschiedliche Bestandteile von bakteriellen Regulationssequenzen bekannt. Man unterscheidet die Bindungstellen von Regulatoren, auch Operatoren genannt, die Bindungstellen von RNA-Polymerase-Holoenzymen, auch -35 und - 10 Regionen genannt, und die Bindungsstelle von Ribosomaler 16S-RNA, auch Ribosomale Bindungsstelle oder auch Shine-Dalgarno-Sequenz genannt.

Als Sequenz einer Ribosomalen Bindungsstelle, auch Shine-Dalgarno-Sequenz genannt, im Sinne dieser Erfindung werden Polynukleotidsequenzen verstanden, die sich bis zu 20 Basen stromauf des Initiationskodon der Translation befinden.

In der Literatur (E. coli und S. typhimurium, Neidhardt F.C. 1995 ASM Press) wird beschrieben, dass sowohl die Zusammensetzung der Polynukletidsequenz der Shine-Delgarno-Sequenz, die Sequenzabfolge der Basen, aber auch der Abstand einer in der Shine-Delgarno-Sequenz enthaltenen Polynukletidsequenz zum einen wesentlichen Einfluss auf die Initiationstionsrate der Translation hat.

Nukleinsäuresequenzen mit Promotoraktivität können die Bildung von mRNA auf unterschiedliche Weise beeinflussen. Promotoren, deren Aktivität unabhängig von der physiologischen Wachstumsphase des Organismus sind, nennt man konstitutiv. Wiederum andere Promotoren reagieren auf externe chemische, wie physikalische Stimuli wie Sauerstoff, Metabolite, Hitze, pH, etc.. Wiederum andere zeigen in unterschiedlichen Wachstumsphasen eine starke Abhängigkeit ihrer Aktivität. Beispielsweise sind in der Literatur Promotoren beschrieben, die während der exponentiellen Wachstumsphase von Mikroorganismen eine besonders ausgeprägte Aktivität zeigen, oder aber auch genau in der stationären Phase des mikrobiellen Wachstums. Beide Charakteristika von Promotoren können für eine Produktion von Feinchemikalien und Proteine je nach Stoffwechselweg einen günstigen Einfluss auf die Produktivität haben.

Zum Beispiel kann man Promotoren die während des Wachstums die Expresssion eines Gens ausschalten, diese aber nach einem optimalen Wachstum anschalten dazu nutzen, ein Gen zu regulieren, das die Produktion eines Metaboliten kontrolliert. Der veränderte Stamm weist dann die gleichen Wachstumsparameter wie der Ausgangsstamm auf, produziert aber mehr Produkt pro Zelle. Diese Art der Modifizierung kann sowohl den Titer (g Produkt/Liter) als auch die C-Ausbeute (g Produkt/g-C-Quelle) erhöhen.

In Corynebacterium Spezies konnten bereits solche Nukleotidsequenzen isoliert werden, die für eine Erhöhung bzw. eine Abschwächung der Genexpression genutzt werden können. Diese regulierten Promotoren können die Rate, mit der ein Gen transkribiert wird, abhängig von den internen und/oder externen Bedingungen der Zelle erhöhen oder erniedrigen. Zum Teil kann die Anwesenheit eines bestimmten Faktors, bekannt als Inducer, die Rate der Transkrition vom Promotor stimulieren. Inducer können direkt oder aber indirekt die Transkription vom Promotor beeinflussen. Eine andere Klasse von Faktoren, bekannt als Suppressoren ist in der Lage, die Transkription vom Promotor zu reduzieren oder aber zu inhibieren. Wie auch die Inducer, können auch die Suppressoren direkt oder indirekt wirken. Es sind jedoch auch Promotoren bekannt, die über die Temperatur reguliert werden. So kann der Level der Transkription solcher Promotoren zum Beispiel durch eine Erhöhung der Wachstumstemperatur über die normale Wachstumstemperatur der Zelle erhöht oder aber abgeschwächt werden.

Eine geringe Anzahl von Promotoren aus C. glutamicum wurden bis zum heutigen Tag beschrieben. Der Promotor des Malatsynthase-Gens aus C. glutamicum wurde im DE 4440118 beschrieben. Dieser Promotor wurde einem für ein Protein kodierendes Strukturgen vorgeschaltet. Nach Transformation eines solchen Konstrukts in ein coryneformes Bakterium wird die Expression des dem Promotor nachgeschalteten Strukturgen reguliert. Die Expression des Strukturgens wird induziert sobald dem Medium ein ensprechender Induktor zugesetzt wird.

Reinscheid et al., Microbiology 145:503 (1999) haben eine trankriptionelle Fusion zwischen dem pta-ack Promotor aus C. glutamicum und einem Reportergen (Chloramphenicol Acetyltransferase) beschrieben. Zellen von C. glutamicum, die eine solche transkriptionelle Fusion enthalten, wiesen eine erhöhte Expression des Reportergenes bei Wachstum auf Acetat haltigem Medium auf. Im Vergleich dazu zeigten transformierte Zellen, die auf Glucose wuchsen, keine erhöhte Expression dieses Reportergens.

In Pa'tek et al., Microbiology 142:1297 (1996) wurden einige DNA Sequenzen aus C. glutamicum beschrieben, die die Expression eines Reportergens in C. glutamicum Zellen verstärken können, beschrieben. Diese Sequenzen wurden miteinander verglichen, um Consensus-Sequenzen für C. glutamicum Promotoren zu definieren.

Weitere DNA-Sequenzen aus C. glutamicum, die zur Regulation der Genexpression genutzt werden können, sind im Patent WO 02/40679 beschrieben worden. Diese isolierten Polynukleotide stellen Expressionseinheiten aus Corynebakterium glutamicum dar, die entweder zur Erhöhung oder aber zur Verringerung einer Genexpression genutzt werden können. Weiterhin sind in diesem Patent rekombinante Plasmide beschrieben, auf denen die Expressionseinheiten aus Corynebakterium glutamicum mit heterologen Genen assoziiert sind. Die hier beschrieben Methode , Fusion von einem Promotor aus Corynebakterium glutamicum mit einem heterlogen Gen, kann unter anderem zur Regulation der Gene der Aminosäurebiosynthese eingesetzt werden.

Der Erfindung lag die Aufgabe zugrunde weitere Promotoren und/oder Expressionseinheiten mit vorteilhaften Eigenschaften zur Verfügung zustellen.

Demgemäß wurde gefunden, dass man Nukleinsäuren mit Promotoraktivität, enthaltend
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
   oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C)
zur Transkription von Genen verwenden kann.

Unter "Transkription" wird erfindungsgemäß der Prozess verstanden, durch den ausgehend von einer DNA-Matrize ein komplementäres RNA-Molekül hergestellt wird. An diesem Prozess sind Proteine wie die RNA-Polymerase sogenannte Sigma-Faktoren und transkriptionelle Regulatorproteine beteiligt. Die synthetisierte RNA dient dann als Matrize im Prozess der Translation, der dann zum biosynthetisch aktiven Protein führt.

Die Bildungsrate, mit der ein biosynthetsich aktives Protein hergestellt wird, ist ein Produkt aus der Rate der Transkription und der Translation. Beide Raten können erfindungsgemäß beeinflusst werden und damit die Rate der Bildung von Produkten in einem Mikroorganismus beeinflussen.

Unter einem "Promotor" oder einer "Nukleinsäure mit Promotoraktivität" wird erfindungsgemäß eine Nukleinsäure verstanden, die in funktioneller Verknüpfung mit einer zu trankripierenden Nukleinsäure, die Transkription dieser Nukleinsäure reguliert.

Unter einer "funktionellen Verknüpfung" versteht man in diesem Zusammenhang beispielsweise die sequentielle Anordnung einer der erfindungsgemäßen Nukleinsäuren mit Promotoraktivität und einer zu transkribierenden Nukleinsäuresequenz und ggf. weiterer regulativer Elemente wie zum Beispiel Nukleinsäureseuqenzen, die die Transkription von Nukleinsäuren gewährleisten, sowie zum Beipsiel einen Terminator derart, dass jedes der regulativen Elemente seine Funktion bei der Transkription der der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu trankribierende Nukleinsäuresequenz hinter (d.h. am 3'-Ende) der erfindungsgemäßen Promotorsequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Promotoraktivität" wird erfindungsgemäß die in einer bestimmten Zeit durch den Promotor gebildete Menge RNA, also die Trankriptionsrate verstanden.

Unter "spezifischer Promotoraktivität" wird erfindungsgemäß die in einer bestimmten Zeit durch den Promotor gebildete Menge RNA pro Promotor verstanden.

Unter dem Begriff "Wildtyp" wird erfindungsgemäß der entsprechende Ausgangsmikroorganismus verstanden.

Je nach Zusammenhang kann unter dem Begriff "Mikroorganismus" der Ausgangsmikroorganismus (Wildtyp) oder ein erfindungsgemäßer, genetisch veränderter Mikroorganismus oder beides verstanden werden.

Vorzugsweise und insbesondere in Fällen, in denen der Mikroorganismus oder der Wildtyp nicht eindeutig zugeordnet werden kann, wird unter "Wildtyp" für die Veränderung oder Verursachung der Promotoraktivität oder Trankriptionsrate, für die Veränderung oder Verursachung der Expressionsaktivität oder Expressionsrate und für die Erhöhung des Gehalts an biosynthetischen Produkten jeweils ein Referenzorganismus verstanden.

In einer bevorzugten Ausführungsform ist dieser Referenzorganismus Corynebakterium glutamicum ATCC 13032.

In einer bevorzugten Ausführungsform werden Ausgangsmikroorganismen verwendet die bereits in der Lage sind, die gewünschte Feinchemikalie herzustellen. Besonders bevorzugt sind dabei unter den besonders bevorzugten Mikroorganismen der Bakterien der Gattung Corynebacterien und den besonders bevorzugten Feinchemikalien L-Lysin, L-Methionin und L-Threonin, diejenigen Ausgangsmikroorganismen die bereits in der Lage sind, L-Lysin, L-Methionin und/oder L-Threonin herzustellen. Dies sind besonderes bevorzugt Corynebakterien bei denen beispielsweise das Gen kodierend für eine Aspartokinase (ask-Gen) dereguliert ist oder die feed-back-Inhibierung aufgehoben oder reduziert ist. Beispielsweise weisen solche Bakterien im ask-Gen eine Mutation auf, die zu einer Reduzierung oder Aufhebung der feed-back-Inhibierung führen, wie beispielsweise die Mutation T311I.

Bei einer "verursachten Promotoraktivität" oder Transkriptionsrate im Bezug auf ein Gen im Vergleich zum Wildtyp wird somit im Vergleich zum Wildtyp die Bildung einer RNA verursacht, die im Wildtyp so nicht vorhanden war.

Bei einer veränderten Promotoraktivität oder Transkriptionsrate im Bezug auf ein Gen im Vergleich zum Wildtyp wird somit im Vergleich zum Wildtyp in einer bestimmten Zeit die gebildete Menge der RNA verändert.

Unter "verändert" wird in diesem Zusammenhang bevorzugt erhöht oder erniedrigt verstanden.

Dies kann beispielsweise durch Erhöhung oder Reduzierung der spezifischen Promotoraktivität des endogenen erfindungsgemäßen Promotors, beispielsweise durch Mutation des Promotors oder durch Stimmulierung oder Hemmung des Promotors erfolgen.

Weiterhin kann die erhöhte Promotoraktivität oder Transkriptionsrate beispielsweise durch Regulation der Transkription von Genen im Mikroorganismus durch erfindungsgemäße Nukleinsäuren mit Promotoraktivität oder durch Nukleinsäuren mit erhöhter spezifischer Promotoraktivität erreicht werden, wobei die Gene in Bezug auf die Nukleinsäuren mit Promotoraktivität heterolog sind.

Vorzusgweise wird die Regulation der Transkription von Genen im Mikroorganismus durch erfindungsgemäße Nukleinsäuren mit Promotoraktivität oder durch Nukleinsäuren mit erhöhter spezifischer Promotoraktivität dadurch erreicht, dass man

eine oder mehrere erfindungsgemäße Nukleinsäuren mit Promotoraktivität, gegebenenfalls mit veränderter spezifischer Promotoraktivität, in das Genom des Mikroorganismus einbringt, so dass die Transkription eines oder mehrerer endogenen Gene unter der Kontrolle der eingebrachten erfindungsgemäßen Nukleinsäure mit Promotoraktivität gegebenenfalls mit veränderter spezifischer Promotoraktivität, erfolgt oder
ein oder mehrere Gene in das Genom des Mikrorganismus einbringt, so dass die Transkription eines oder mehrerer der eingebrachten Gene unter der Kontrolle der endogenen erfindungsgemäßen Nukleinsäuren mit Promotoraktivität, gegebenenfalls mit veränderter spezifischer Promotoraktivität, erfolgt oder
ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende Nukleinsäuren, in den Mikroorganismus einbringt.

Die erfindungsgemäßen Nukleinsäuren mit Promotoraktivität enthalten
A) die Nukleinsäuresequenz SEQ. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
   oder
C) eine Nukleinsäuresequenz, die mit der Nukleinsäuresequenz SEQ. ID. NO. 1 unter stringenten Bedingungen hybridisiert oder
D) funktionell äquivalente Fragmente der Sequenzen unter A), B) oder C)

Die Nukleinsäuresequenz SEQ. ID. NO. 1 stellt die Promotorsequenz des GroES Chaperonin (Pgro) aus Corynebakterium glutamicum dar. SEQ. ID.NO. 1 entspricht der Promotorsequenz des Wildtyps..

Die Erfindung betrifft weiterhin Nukleinsäuren mit Promotoraktivität enthaltend eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist.

Weitere natürliche erfindungsgemäße Beispiele für erfindungsgemäße Promotoren lassen sich beispielsweise aus verschiedenen Organismen, deren genomische Sequenz bekannt ist, durch Identitätsvergleiche der Nukleinsäuresequenzen aus Datenbanken mit der vorstehend beschriebenen Sequenzen SEQ ID NO: 1 leicht auffinden.

Künstliche erfindungsgemäße Promotor-Sequenzen lassen sich ausgehend von der Sequenz SEQ ID NO: 1 durch künstliche Variation und Mutation, beispielsweise durch Substitution, Insertion oder Deletion von Nukleotiden leicht auffinden.

Unter dem Begriff "Substitution" ist in der Beschreibung der Austausch einer oder mehrerer Nukleotide durch ein oder mehrere Nukleotide zu verstehen. "Deletion" ist das Ersetzen eines Nukleotides durch eine direkte Bindung. Insertionen sind Einfügungen von Nukleotiden in die Nukleinsäuresequenz, wobei formal eine direkte Bindung durch ein oder mehrere Nukleotide ersetzt wird.

Unter Identität zwischen zwei Nukleinsäuren wird die Identität der Nukleotide über die jeweils gesamte Nukleinsäurelänge verstanden, insbesondere die Identität die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:
FAST algorithm on

| | |
|---|---|
| K-tuplesize | 1 |
| Gap penalty | 3 |
| Window size | 5 |
| Number of best diagonals | 5 |

Unter einer Nukleinsäuresequenz, die eine Identität von mindestens 90 % mit der Sequenz SEQ ID NO: 1 aufweist, wird dementsprechend eine Nukleinsäuresequenz verstanden, die bei einem Vergleich seiner Sequenz mit der Sequenz SEQ ID NO: 1, insbesondere nach obigen Programmlogarithmus mit obigem Parametersatz eine Identität von mindestens 90 % aufweist.

Besonders bevorzugte Promotoren weisen mit der Nukleinsäuresequenz SEQ. ID. NO. 1 eine Identität von 91 %, bevorzugter 92%, 93%, 94%, 95%, 96%, 97%, 98%, besonders bevorzugt 99% auf.

Weitere natürliche Beispiele für Promotoren lassen sich weiterhin ausgehend von den vorstehend beschriebenen Nukleinsäuresequenzen, insbesondere ausgehend von der Sequenz SEQ ID NO: 1 aus verschiedenen Organismen, deren genomische Sequenz nicht bekannt ist, durch Hybridisierungstechniken in an sich bekannter Weise leicht auffinden.

Ein weiterer Gegenstand der Erfindung betrifft daher Nukleinsäuren mit Promotoraktivität, enthaltend eine Nukleinsäuresequenz, die mit der Nukleinsäuresequenz SEQ. ID. No. 1 unter stringenten Bedingungen hybridisiert. Diese Nukleinsäuresequenz umfasst mindestens 10, bevorzugter mehr als12,15,30,50 oder besonders bevorzugt mehr als 150 Nukleotide.

Die Hybridisierung erfolgt erfinungsgemäß unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben:

Unter stringenten Hybridisierungs-Bedingungen werden insbesondere verstanden: Die über Nacht Inkubation bei 42°C in einer Lösung bestehend aus 50 % Formamid, 5 x SSC (750 mM NaCl, 75 mM Tri-Natrium Citrat), 50 mM Natrium Phosphat (ph7,6), 5x Denhardt Lösung, 10% Dextransulfat und 20 g/ml denaturierte, gescheerte Lachsspermien-DNA, gefolgt von einem Waschen der Filter mit 0,1x SSC bei 65°C.

Unter einem "funktionell äquivalenten Fragment" werden für Nukleinsäuresequenzen mit Promotoraktivität, Fragmente verstanden die im wesentlichen die gleiche oder eine höhere spezifische Promotoraktivität aufweisen wie die Ausgangssequenz.

Unter "im wesentlichen gleich" wird eine spezifische Promotoraktivität verstanden die mindestens 50%, vorzugsweise 60%, bevorzugter 70%, bevorzugter 80%, bevorzugter 90%, besonders bevorzugt 95% der spezifischen Promotoraktivität der Ausgangssequenz aufweist.

Unter "Fragmente" werden Teilsequenzen der durch Ausführungsform A), B) oder C) beschriebenen Nukleinsäuren mit Promotoraktivität verstanden. Vorzusgweise weisen diese Fragmente mehr als 10, bevorzugter aber mehr als 12,15, 30, 50 oder besonders bevorzugts mehr als 150 zusammenhängende Nukleotide der Nukleinsäuresequenz SEQ. ID. NO. 1 auf.

Besonders bevorzugt ist die Verwendung der Nukleinsäuresequenz SEQ. ID. NO. 1 als Promotor, d.h. zur Transkriptiion von Genen.

Die SEO. ID. NO. 1 ist ohne Funktionszuordnung im Genbank-Eintrag AP005283 beschrieben worden. Daher betrifft die Erfindung ferner die neuen, erfindungsgemäßen Nukleinsäuresequenzen mit Promotoraktivität.

Insbesondere betrifft die Erfindung eine Nukleinsäure mit Promotoraktivität, enthaltend
A) die Nukleinsäuresequenz SEO. ID. NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 1 aufweist
   oder
C) eine Nukleinsäuresequenz, die mit der Nukleinsäuresequenz SEO. ID. NO. 1 unter stringenten Bedingungen hybridisiert oder
D) funktionell äquivalente Fragmente der Sequenzen unter A), B) oder C),
mit der Maßgabe, dass die Nukleinsäure mit der Sequenz SEQ. ID. NO. 1 ausgenommen ist.

Alle vorstehend erwähnten Nukleinsäuren mit Promotoraktivität sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Die Erfindung betrifft ferner die Verwendung einer Expressionseinheit, enthaltend eine der erfindungsgemäßen Nukleinsäuren mit Promotoraktivität und zusätzlich funktionell verknüpft eine Nukleinsäuresequenz, die die Translation von Ribonukleinsäuren gewährleistet, zur Expression von Genen.

Unter einer Expressioneinheit wird erfindungsgemäß eine Nukleinsäure mit Expressionsaktivität verstanden, also eine Nukleinsäure verstanden, die in funktioneller Verknüpfung mit einer zu exprimierenden Nukleinsäure oder Gens, die Expression, also die Transkription und die Translation dieser Nukleinsäure oder dieses Gens reguliert.

Unter einer "funktionellen Verknüpfung" versteht man in diesem Zusammenhang beispielsweise die sequentielle Anordnung einer der erfindungsgemäßen Expressionseinheit und einer transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen,
in denen die transgen zu exprimierende Nukleinsäuresequenz hinter (d.h. am 3'-Ende) der erfindungsgemäßen Expressionseinheitssequenz positioniert wird, so dass beide Sequenzen kovalent miteinander verbunden sind. Bevorzugt ist dabei der Abstand zwischen der Expressionseinheitssequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Expressionsaktivität" wird erfindungsgemäß die in einer bestimmten Zeit durch die Expressionseinheit gebildete Menge Protein, also die Expressionsrate verstanden.

Unter "spezifischer Expressionsaktivität" wird erfindungsgemäß die in einer bestimmten Zeit durch die Expressionseinheit gebildete Menge Protein pro Expressionseinheit verstanden.

Bei einer "verursachten Expressionsaktivität" oder Expressionsrate im Bezug auf ein Gen im Vergleich zum Wildtyp wird somit im Vergleich zum Wildtyp die Bildung eines Proteins verursacht, das im Wildtyp so nicht vorhanden war.

Bei einer "veränderten Expressionsaktivität" oder Expressionsrate im Bezug auf ein Gen im Vergleich zum Wildtyp wird somit im Vergleich zum Wildtyp in einer bestimmten Zeit die gebildete Menge des Proteins verändert.

Unter "verändert" wird in diesem Zusammenhang bevorzugt erhöht oder erniedrigt verstanden.

Dies kann beispielsweise durch Erhöhung oder Reduzierung der spezifischen Aktivität der endogenen Expressionseinheit, beispielsweise durch Mutation der Expressionseinheit oder durch Stimmulierung oder Hemmung der Expressionseinheit erfolgen.

Weiterhin kann die erhöhte Expressionsaktivität oder Expressionsrate beispielsweise durch Regulation der Expression von Genen im Mikroorganismus durch erfindungsgemäße Expressionseinheiten oder durch Expressionseinheiten mit Erhöhter spezifischer Expressionsaktivität erreicht werden, wobei die Gene im Bezug auf die Expressionseinheiten heterolog sind.

Vorzugsweise wird die Regulation der Expression von Genen im Mikroorganismus durch erfindungsgemäße Expressionseinheiten oder durch erfindungsgemäße Expressionseinheiten mit Erhöhter spezifischer Expressionsaktivitätdadurch erreicht, dass man
eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten erfindungsgemäßen Expressionseinheiten, gegebenenfalls mit veränderter spezifischer Expressionsaktivität, erfolgt oder
ein oder mehrere Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit veränderter spezifischer Expressionsaktivität, erfolgt oder
ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Die erfindungsgemäßen Expressionseinheiten, enthalten eine erfindungsgemäße, vorstehend bechriebene Nukleinsäure mit Promotoraktivität und zusätzlich funktionell verknüpft eine Nukleinsäuresequenz, die die Translation von Ribonukleinsäuren gewährleistet.

Vorzugsweise enthält diese Nukleinsäuresequenz, die die Translation von Ribonukleinsäuren gewährleistet, die Nukleinsäuresequenz SEQ. ID. NO. 42 als ribosomale Bindungsstelle.

In einer bevorzugten Ausführungsform enthält die erfindungsgemäße Expressionseinheit:
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist oder
G) eine Nukleinsäuresequenz, die mit der Nukleinsäuresequenz SEQ. ID. NO. 2 unter stringenten Bedingungen hybridisiert oder
H) funktionell äquivalente Fragmente der Sequenzen unter E), F) oder G).

Die Nukleinsäuresequenz SEQ. ID. NO. 2 stellt die Nukleinsäuresequenz der Expressionseinheit des GroES Chaperonin (Pgro) aus Corynebakterium glutamicum dar. SEQ. ID.NO. 2 entspricht der Sequenz der Expressionseinheit des Wildtyps.

Die Erfindung betrifft weiterhin Expressionseinheiten, enthaltend eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist.

Weitere natürliche erfindungsgemäße Beispiele für erfindungsgemäße Expressionseinheiten lassen sich beispielsweise aus verschiedenen Organismen, deren genomische Sequenz bekannt ist, durch Identitätsvergleiche der Nukleinsäuresequenzen aus Datenbanken mit der vorstehend beschriebenen Sequenzen SEQ ID NO: 2 leicht auffinden.

Künstliche erfindungsgemäße Sequenzen der Expressionseinheiten lassen sich ausgehend von der Sequenz SEQ ID NO: 2 durch künstliche Variation und Mutation, beispielsweise durch Substitution, Insertion oder Deletion von Nukleotiden leicht auffinden.

Unter einer Nukleinsäuresequenz, die eine Identität von mindestens 90 % mit der Sequenz SEQ ID NO: 2 aufweist, wird dementsprechend eine Nukleinsäuresequenz verstanden, die bei einem Vergleich seiner Sequenz mit der Sequenz SEQ ID NO: 2, insbesondere nach obigen Programmlogarithmus mit obigem Parametersatz eine Identität von mindestens 90 % aufweist.

Besonders bevorzugte Expressionseinheiten weisen mit der Nukleinsäuresequenz SEQ. ID. NO. 2 eine Identität von 91 %, bevorzugter 92%, 93%, 94%, 95%, 96%, 97%, 98%, besonders bevorzugt 99% auf.

Weitere natürliche Beispiele für Expressionseinheiten lassen sich weiterhin ausgehend von den vorstehend beschriebenen Nukleinsäuresequenzen, insbesondere ausgehend von der Sequenz SEQ ID NO: 2 aus verschiedenen Organismen, deren genomische Sequenz nicht bekannt ist, durch Hybridisierungstechniken in an sich bekannter Weise leicht auffinden.

Ein weiterer Gegenstand der Erfindung betrifft daher Expressionseinheiten, enthaltend eine Nukleinsäuresequenz, die mit der Nukleinsäuresequenz SEQ. ID. No. 2 unter stringenten Bedingungen hybridisiert. Diese Nukleinsäuresequenz umfasst mindestens 10, bevorzugter mehr alls12,15,30;50 oder besonders bevorzugt mehr als 150 Nukleotide.

Unter "hybridisieren" versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Die Hybridisierung erfolgt erfinungsgemäß unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben:

Unter stringenten Hybridisierungs-Bedingungen werden insbesondere verstanden: Die über Nacht Inkubation bei 42°C in einer Lösung bestehend aus 50 % Formamid, 5 x SSC (750 mM NaCl, 75 mM Tri-Natrium Citrat), 50 mM Natrium Phosphat (ph7,6), 5x Denhardt Lösung, 10% Dextransulfat und 20 g/ml denaturierte, gescheerte Lachsspermien-DNA, gefolgt von einem Waschen der Filter mit 0,1x SSC bei 65°C.

Die erfindungsgemäß Nukleotidsequenzen ermöglichen ferner die Erzeugung von Sonden und Primern, die zur Identifizierung und/oder Klonierung von homologer Sequenzen in anderen Zelltypen und Mikroorganismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Unter einem "funktionell äquivalenten Fragment" werden für Expressionseinheiten, Fragmente verstanden die im wesentlichen die gleiche oder eine höhere spezifische Expressionsaktivität aufweisen wie die Ausgangssequenz.

Unter "im wesentlichen gleich" wird eine spezifische Expressionsaktivität verstanden die mindestens 50%, vorzugsweise 60%, bevorzugter 70%, bevorzugter 80%, bevorzugter 90%, besonders bevorzugt 95% der spezifischen Expressionsaktivität der Ausgangssequenz aufweist.

Unter "Fragmente" werden Teilsequenzen der durch Ausführungsform E), F) oder G) beschriebenen Expressionseinheiten verstanden. Vorzusgweise weisen diese Fragmente mehr als 10, bevorzugter aber mehr als 12,15, 30, 50 oder besonders bevorzugts mehr als 150 zusammenhängende Nukleotide der Nukleinsäuresequenz SEO. ID. NO. 1 auf.

Besonders bevorzugt ist die Verwendung der Nukleinsäuresequenz SEQ. ID. NO. 2 als Expressionseinheit, d.h. zur Expression von Genen.

Die SEO. ID. NO. 2 ist ohne Funktionszuordnung im Genbank-Eintrag AP005283 beschrieben worden. Daher betrifft die Erfindung ferner die neuen, erfindungsgemäßen Expressionseinheiten.

Insbesondere betrifft die Erfindung eine Expressionseinheit, enthaltend eine erfindungsgemäße Nukleinsäure mit Promotoraktivität zusätzlich funktionell verknüpft eine Nukleinsäuresequenz, die die Translation von Ribonukleinsäuren gewährleistet.

Besonders bevorzugt betrifft die Erfindung eine Expressionseinheit, enthaltend
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEO. ID. NO. 2 aufweist oder
G) eine Nukleinsäuresequenz, die mit der Nukleinsäuresequenz SEO. ID. NO. 2 unter stringenten Bedingungen hybridisiert oder
H) funktionell äquivalente Fragmente der Sequenzen unter E), F) oder G),
mit der Maßgabe, dass die Nukleinsäure mit der Sequenz SEQ. ID. NO. 2 ausgenommen ist.

Die erfindungsgemäßen Expressionseinheiten umfassen ein oder mehrere der folgenden genetischen Elemente: eine Minus 10 ("-10") Sequenz; eine Minus 35 ("-35") Sequenz; einen Transkriptionsstart, eine Enhancer Region; und eine Operator Region.

Vorzugsweise sind diese genetischen Elemente spezifisch für die Spezies Corynebakterien, speziell für Corynbacterium glutamicum.

Alle vorstehend erwähnten Expressionseinheiten sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Für die Erfindungen in diesem Patent wurden Methoden und Techniken genutzt, die dem Fachmann, der in mikrobiologischen und rekombinanten DNA-Techniken geübt ist, bekannt sind. Methoden und Techniken für das Wachstum von Bakterienzellen, das Einschleusen von isolierten DNA-Molekülen in die Wirtszelle, und die Isolierung, Klonierung und Sequenzierung von isolierten Nukleinsäuremolekülen usw. sind Beispiele für solche Techniken und Methoden. Diese Methoden sind in vielen Standardliteraturstellen beschrieben: Davis et al., Basic Methods In Molecular Biology (1986); J. H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes & Genomes, University Science Books, Mill Valley, Carlifornia (1991); J. Sambrook, E.F. Fritsch and T. Maniatis, Molecular Cloning: A Laboratory Manual, 2nd ed., Cold Spring Harbor Laboratory Press, Cold Spring Harbor, New York (1989); P.B. Kaufmann et al., Handbook of Molcular and Cellular Methods in Biology and Medicine, CRC Press, Boca Raton, Florida (1995); Methods in Plant Molecular Biology and Biotechnology, B.R. Glick and J.E. Thompson, eds., CRC Press, Boca Raton, Florida (1993); and P.F. Smith-Keary, Molecular Genetics of Escherichia coli, The Guilford Press, New York, NY (1989).

Alle Nukleinsäuremoleküle der vorliegenden Erfindung liegen bevorzugt in Form eines isolierten Nukleinsäuremoleküls vor. Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Die Erfindung umfasst weiterhin die zu den konkret beschriebenen Nukleotidsequenzen komplementären Nukleinsäuremoleküle oder einen Abschnitt davon.

Die erfindungsgemäßen Promotoren und/oder Expressionseinheiten lassen sich beispielsweise besonders vorteilhaft in verbesserten Verfahren zur fermentativen Herstellung von biosynthetischen Produkten wie nachstehend beschrieben verwenden.

Die erfindungsgemäßen Promotoren und/oder Expressionseinheiten weisen insbesondere den Vorteil auf, dass sie in Mikroorganismen durch Stress induziert werden. Durch geeignete Steuerung des Fermentationsprozesses lässt sich diese Stress-Induktion gezieit für eine Erhöhung der Trankritptions/Expressionsrate gewünschter Gene steuern. Insbesondere bei der Produktion von L-Lysin wird diese Stressphase sehr früh erreicht, so das hier sehr früh eine erhöhte Transkriptions/Expressionsrate von gewünschten Genen erreicht werden kann.

Die erfindungesgemäßen Nukleinsäuren mit Promotoraktivität können zur Veränderung, also zur Erhöhung oder Reduzierung, oder zur Verursachung der Transkriptionsrate von Genen in Mikroorganismen im Vergleich zum Wildtyp verwendet werden.

Die erfindungsgemäßen Expressionseinheiten können zur Veränderung, also zur Erhöhung oder Reduzierung, oder zur Verursachung der Expressionsrate von Genen in Mikroorganismen im Vergleich zum Wildtyp verwendet werden.

Ferner können die erfindungsgemäßen Nukleinsäuren mit Promotoraktivität und die erfindungesgemäßen Expressionseinheiten zur Regulation und Verstärkung der Bildung von verschiedenen biosynthetischen Produkten, wie beispielsweise Feinchemikalien, Proteinen, inbesondere Aminosäuren, in Mikroorganismen, insbsondere in Corynebacterium species dienen.

Die Erfindung betrifft daher ein Verfahren zur Veränderung oder Verursachung der Transkriptionsrate von Genen in Mikroorganismen im Vergleich zum Wildtyp durch
a) Veränderung der spezifischen Promotoraktivität im Mikroorganismus von endogenen, erfindungsgemäßen Nukleinsäuren mit Promotoraktivität, die die Transkription von endogenen Genen regulieren, im Vergleich zum Wildtyp oder
b) Regulation der Transkription von Genen im Mikroorganismus durch erfindungsgemäße Nukleinsäuren mit Promotoraktivität oder durch Nukleinsäuren mit veränderter spezifischer Promotoraktivität gemäß Ausführungsform a), wobei die Gene in Bezug auf die Nukleinsäuren mit Promotoraktivität heterolog sind.

Gemäß Ausführungsform a) kann die Veränderung oder Verursachung der Transkriptionsrate von Genen in Mikroorganismen im Vergleich zum Wildtyp dadurch erfolgen, dass im Mikroorganismus die spezifische Promotoraktivität verändert, also erhöht oder erniedrigt wird. Dies kann beispielsweise durch gezielte Mutation der erfindungsgemäßen Nukleinsäuresequenz mit Promotoraktivität, also durch gezielte Substitution, Deletion oder Insertion von Nukleotiden erfolgen. Eine erhöhte bzw. erniedrigte Promotoraktivität kann dadurch erreicht werden,dass Nukleotide in der Bindungsstelle des RNA-Polymerase-Holoenzym-Bindungsstellen (dem Fachmann auch als -10-Region und - 35 Region bekannt) ausgetauscht werden. Weiterhin dadurch dass der Abstand der beschriebenen RNA-Polymerase-Holoenzym-Bindungsstellen zueinander durch Deletionen von Nukleotiden oder Insertionen von Nukleotiden verkleinert oder vergrößert werden. Weiterhin dadurch dass Bindungsstellen (dem Fachmann auch als - Operatoren bekannt) für Regulatiosproteine (dem Fachmann bekannt als Repressoren und Aktiviatoren) in räumliche Nähe an die Bindungsstellen des RNA-Polymerase-Holoenzyms gebracht werden, dass diese Regulatoren nach Bindung an eine Promotor-Sequenz die Bindung des und Transkriptionsaktivität des RNA-Polymerase-Holoenzyms abschwächen oder verstärken, oder auch unter einen neuen regulatorischen Einfluss stellen.

Die Nukleinsäuresequenz SEQ. ID. NO. 53 stellt vorzugsweise die ribosomale Bindungsstelle der erfindungsgemäßen Expressionseinheiten, die Sequenz SEQ. ID. NO. 52 die -10-Region der erfindungsgemäßen Expressionseinheiten dar. Veränderungen der Nukleinsäureaequenz in diesen Regionen führen zu einer Veränderung der spezifischen Expressionsaktivität.

Die Erfindung betrifft daher die Verwendung der Nukleinsäuresequenz SEQ. ID. NO. 53 als ribosomale Bindungsstelle in Expressionseinheiten, die die Expression von Genen in Bakterien der Gattung Corynebacterium oder Brevibacterium ermöglichen.

Weiterhin betrifft die Erfindung die Verwendung der Nukleinsäuresequenz SEQ. ID. NO 52 als -10-Region in Expressionseinheiten, die die Expression von Genen in Bakterien der Gattung Corynebacterium oder Brevibacterium ermöglichen.

Insbesondere betrifft die Erfindung eine Expressionseinheit, die die Expression von Genen in Bakterien der Gattung Corynebacterium oder Brevibacterium ermöglicht, enthaltend die Nukleinsäuresequenz SEQ. ID. NO. 53. Vorzugsweise wird dabei die Nukleinsäuresequenz SEQ. ID. NO. 53. als ribosomale Bindungsstelle verwendet.

Weiterhin betrifft die Erfindung eine Expressionseinheit, die die Expression von Genen in Bakterien der Gattung Corynebacterium oder Brevibacterium ermöglicht, enthaltend die Nukleinsäuresequenz SEQ. ID. NO. 52. Vorzugsweise wird dabei die Nukleinsäuresequenz SEQ. ID. NO. 52 als -10-Region verwendet.

In Bezug auf die "spezifische Promotoraktivität" wird unter Erhöhung oder Reduzierung im Vergleich zum Wildtyp eine Erhöhung oder Reduzierung der spezifischen Aktivität gegenüber der erfindungsgemäßen Nukleinsäure mit Promotoraktivität des Wildtyps, also beispielsweise gegenüber der SEQ. ID. NO. 1 verstanden.

Gemäß Ausführungsform b) kann die Veränderung oder Verursachung der Transkriptionsrate von Genen in Mikroorganismen im Vergleich zum Wildtyp dadurch erfolgen, dass man die Transkription von Genen im Mikroorganismus durch erfindungsgemäße Nukleinsäuren mit Promotoraktivität oder durch Nukleinsäuren mit veränderter spezifischer Promotoraktivität gemäß Ausführungsform a) reguliert, wobei die Gene in Bezug auf die Nukleinsäuren mit Promotoraktivität heterolog sind.

Dies wird bevorzugt dadurch erreicht, dass man
b1) eine oder mehrere erfindungsgemäße Nukleinsäuren mit Promotoraktivität, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen, erfindungsgemäßen Nukleinsäuren mit Promotoraktivität, gegebenenfalls mit veränderter spezifischer Promotoraktivität, erfolgt oder
b3) ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende Nukleinsäuren, in den Mikroorganismus einbringt.

Es ist somit möglich, die Transkriptionsrate eines endogenen Gens des Wildtyps zu verändern, also zu erhöhen oder zu erniedrigen indem man
gemäß Ausführungsform b1) eine oder mehrere erfindungsgemäße Nukleinsäuren mit Promotoraktivität, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit veränderter spezifischer Promotoraktivität, erfolgt oder
gemäß Ausführungsform b2) ein oder mehrere endogene Gene in das Genom des Mikrorganismus einbringt, so dass die Transkription eines oder mehrerer der eingebrachten, endogenen Gene unter der Kontrolle der endogenen, erfindungsgemäßen Nukleinsäuren mit Promotoraktivität, gegebenenfalls mit veränderter spezifischer Promotoraktivität, erfolgt oder

Gemäß Ausführungsform b3) ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende endogene Nukleinsäuren, in den Mikroorganismus einbringt.

Ferner ist es somit möglich, die Transkriptionsrate eines exogenen Gens im Vergleich zum Wildtyps zu verursachen, indem man
gemäß Ausführungsform b2) ein oder mehrere exogene Gene in das Genom des Mikrorganismus einbringt, so dass die Transkription eines oder mehrerer der eingebrachten, exogenen Gene unter der Kontrolle der endogenen, erfindungsgemäßen Nukleinsäuren mit Promotoraktivität, gegebenenfalls mit veränderter spezifischer Promotoraktivität, erfolgt oder

Gemäß Ausführungsform b3) ein oder mehrere Nukleinsäurekonstrukte, enthaltend eine erfindungsgemäße Nukleinsäure mit Promotoraktivität, gegebenenfalls mit veränderter spezifischer Promotoraktivität, und funktionell verknüpft eine oder mehrere, zu transkribierende exogene Nukleinsäuren, in den Mikroorganismus einbringt.

Die Insertion von Genen gemäß Ausführungsform b2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform b3) kann dabei chromosomal oder extrachromosomal erfolgen. Vorzugsweise erfolgt die Insertion der Nukleinsäurekonstrukte chromosomal. Eine "chromosomale" Integration ist die Insertion eines exogenen DNA-Fragmentes in das Chromosom einer Wirtszelle. Dieser Begriff wird auch für die homologe Rekombination zwischen einem exogenen DNA-Fragment und der entsprechenden Region auf dem Chromosom der Wirtszelle genutzt.

In Ausführungsform b) werden bevorzugt auch erfindungsgemäße Nukleinsäuren mit veränderter spezifischer Promotoraktivität gemäß Ausführungsform a) eingesetzt. Diese können in Ausführungsform b), wie in Ausführungsform a) beschrieben im Mikroorganismus vorliegen und hergestellt werden oder in isolierter Form in den Mikroorganismus eingebracht werden.

Unter "endogen" werden genetische Informationen, wie beispielsweise Gene, verstanden, die bereits im Wildtypgenom enthalten sind.

Unter "exogen" werden genetische Informationen, wie beispielsweise Gene, verstanden, die im Wildtypgenom nicht enthalten sind.

Unter dem Begriff "Gene" in Bezug auf Regulation der Transkription durch die erfindungsgemäßen Nukleinsäuren mit Promotoraktivität werden vorzugsweise Nukleinsäuren verstanden, die einen zu transkripierenden Bereich, also beispielsweise einen Bereich der die Translation reguliert, einen kodierenden Bereich, sowie gegebenenfalls weitere Regulationselemente, wie beispielsweise einen Terminator, enthalten.

Unter dem Begriff "Gene" in Bezug auf die nachstehend beschriebene Regulation der Expression durch die erfindungsgemäßen Expressionseinheiten werden vorzugsweise Nukleinsäuren verstanden, die einen kodierenden Bereich, sowie gegebenenfalls weitere Regulationselemente, wie beispielsweise einen Terminator, enthalten.

Unter einem "kodierenden Bereich" wird eine Nukleinsäuresequenz verstanden, die ein Protein kodiert.

Unter "heterolog" in Bezug auf Nukleinsären mit Promotoraktivität und Gene wird verstanden, dass die verwendeten Gene im Wildtyp nicht unter Regulation der erfindungsgemäßen Nukleinsäuren mit Promotoraktivität transkribiert werden, sondern das eine neue, im Wildtyp nicht vorkommende funktionelle Verknüpfung entsteht und die funktionelle Kombination aus erfindungsgemäßer Nukleinsäure mit Promotoraktivität und spezifisches Gen im Wildtyp nicht vorkommt.

Unter "heterolog" in Bezug auf Expressionseinheiten und Gene wird verstanden, dass die verwendeten Gene im Wildtyp nicht unter Regulation der erfindungsgemäßen Expressionseinheiten exprimiert werden, sondern das eine neue, im Wildtyp nicht vorkommende funktionelle Verknüpfung entsteht und die funktionelle Kombination aus erfindungsgemäßer Expressionseinheit und spezifisches Gen im Wildtyp nicht vorkommt.

Die Erfindung betrifft ferner in einer bevorzugten Ausführungsform ein Verfahren zur Erhöhung oder Verursachung der Transkriptionsrate von Genen in Mikroorganismen im Vergleich zum Wildtyp indem man
ah) die spezifische Promotoraktivität im Mikroorganismus von erfindungsgemäßen endogenen Nukleinsäuren mit Promotoraktivität , die die Transkription von endogenen Genen regulieren, im Vergleich zum Wildtyp erhöht oder
bh) die Transkription von Genen im Mikroorganismus durch erfindungsgemäße Nukleinsäuren mit Promotoraktivität oder durch Nukleinsäuren mit erhöhter spezifischer Promotoraktivität gemäß Ausführungsform a) reguliert, wobei die Gene in Bezug auf die Nukleinsäuren mit Promotoraktivität heterolog sind.

Vorzugsweise wird die Regulation der Transkription von Genen im Mikroorganismus durch erfindungsgemäße Nukleinsäuren mit Promotoraktivität oder durch erfindungsgemäße Nukleinsäuren mit erhöhter spezifischer Promotoraktivität gemäß Ausführungsform ah) dadurch erreicht wird, dass man
bh1) eine oder mehrere erfindungsgemäße Nukleinsäuren mit Promotoraktivität, gegebenenfalls mit erhöhter spezifischer Promotoraktivität, in das Genom des Mikroorganismus einbringt, so dass die Transkription eines oder mehrerer endogenen Gene unter der Kontrolle der eingebrachten erfindungsgemäßen Nukleinsäure mit Promotoraktivität, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der erfindungsgemäßen, endogenen Nukleinsäuren mit Promotoraktivität, gegebenenfalls mit erhöhter spezifischer Promotoraktivität, erfolgt oder
bh3) ein oder mehrere Nukleinsäurekonstrukte, enthaltend eine erfindungsgemäße Nukleinsäure mit Promotoraktivität, gegebenenfalls mit erhöhter spezifischer Promotoraktivität, und funktionell verknüpft eine oder mehrere, zu transkribierende Nukleinsäuren, in den Mikroorganismus einbringt.

Die Erfindung betrifft ferner in einer bevorzugten Ausführungsform ein Verfahren zur Reduzierung der Transkriptionsrate von Genen in Mikroorganismen im Vergleich zum Wildtyp, indem man
ar) die spezifische Promotoraktivität im Mikroorganismus von endogenen erfindungsgemäßen Nukleinsäuren mit Promotoraktivität, die die Transkription der endogenen Gene regulieren, im Vergleich zum Wildtyp reduziert oder
br) Nukleinsäuren mit reduzierter spezifischer Promotoraktivität gemäß Ausführungsform a) in das Genom des Mikroorganismus einbringt, so dass die Transkription endogene Gene unter der Kontrolle der eingebrachten Nukleinsäure mit reduzierter Promotoraktivität erfolgt.

Die Erfindung betrifft ferner ein Verfahren zur Veränderung oder Verursachung der Expressionsrate eines Gens in Mikroorganismen im Vergleich zum Wildtyp durch
c) Veränderung der spezifischen Expressionsaktivität im Mikroorganismus von erfindungsgemäßen, endogenen Expressionseinheiten, die die Expression der endogenen Gene regulieren, im Vergleich zum Wildtyp oder
d) Regulation der Expression von Genen im Mikroorganismus durch erfindungsgemäße Expressionseinheiten oder durch erfindungsgemäße Expressionseinheiten mit veränderter spezifischer Expressionsaktivität gemäß Ausführungsform c), wobei die Gene im Bezug auf die Expressionseinheiten heterolog sind.

Gemäß Ausführungsform c) kann die Veränderung oder Verursachung der Expressionsrate von Genen in Mikroorganismen im Vergleich zum Wildtyp dadurch erfolgen, dass im Mikroorganismus die spezifische Expressionsaktivität verändert, also erhöht oder erniedrigt wird. Dies kann beispielsweise durch gezielte Mutation der erfindungsgemäßen Nukleinsäuresequenz mit Promotoraktivität, also durch gezielte Substitution, Deletion oder Insertion von Nukleotiden erfolgen. Beispielsweise führt die Verlängerung des Abstandes zwischen Shine-Dalgarno-Sequenz und dem translationellen Startcodon in der Regel zu einer Änderung, einer Verkleinerung oder aber auch einer Verstärkung der spezifischen Expressionsaktivität. Eine Veränderung der der spezifischen Expressionsaktivität kann auch dadurch erreicht werden, dass die Sequenz der Shine-Dalgarno-Region (Ribosomale Bindungsstelle) in seinem Abstand zum translationellen Startcodon durch Deletionen oder Insertionen von Nukleotiden entweder verkürzt oder verlängert.wird. Aber auch dadurch dass die Sequenz der Shine-Dalgarno-Region so verändert wird, dass die Homologie zu komplementären 3' Seite 16S rRNA entweder verstärkt oder aber auch verringert wird.

In Bezug auf die "spezifische Expressionsaktivität" wird unter Erhöhung oder Reduzierung im Vergleich zum Wildtyp eine Erhöhung oder Reduzierung der spezifischen Aktivität gegenüber der erfindungsgemäßen Expressionseinheit des Wildtyps, also beispielsweise gegenüber der SEQ. ID. NO. 2 verstanden.

Gemäß Ausführungsform d) kann die Veränderung oder Verursachung der Expressionsrate von Genen in Mikroorganismen im Vergleich zum Wildtyp dadurch erfolgen, dass man die Expression von Genen im Mikroorganismus durch erfindungsgemäße Expressionseinheiten oder durch erfindungsgemäße Expressionseinheiten mit veränderter spezifischer Expressionsaktivität gemäß Ausführungsform c) reguliert, wobei die Gene in Bezug auf die Expressionseinheiten heterolog sind.

Dies wird bevorzugt dadurch erreicht, dass man
d1) eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
d2) ein oder mehrere Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit veränderter spezifischer Expressionsaktivität, erfolgt oder
d3) ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Es ist somit möglich, die Expressionsrate eines endogenen Gens des Wildtyps zu verändern, also zu erhöhen oder zu erniedrigen indem man
gemäß Ausführungsform d1) eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
gemäß Ausführungsform d2) ein oder mehrere Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit veränderter spezifischer Expressionsaktivität, erfolgt oder
gemäß Ausführungsform d3) ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Ferner ist es somit möglich, die Expressionssrate eines exogenen Gens im Vergleich zum Wildtyps zu verursachen, indem man
gemäß Ausführungsform d2) ein oder mehrere exogene Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit veränderter spezifischer Expressionsaktivität, erfolgt oder
gemäß Ausführungsform d3) ein oder mehrere Nukleinsäurekonstrukte, enthaltend eine erfindungsgemäße Expressionseinheit, gegebenenfalls mit veränderter spezifischer Expressionsaktivität, und funktionell verknüpft eine oder mehrere, zu exprimierende, exogene Nukleinsäuren, in den Mikroorganismus einbringt.

Die Insertion von Genen gemäß Ausführungsform d2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform d3) kann dabei chromosomal oder extrachromosomal erfolgen. Vorzugsweise erfolgt die Insertion der Nukleinsäurekonstrukte chromosomal.

Die Nukleinsäurekonstrukte werden im folgenden auch als Expressionskasetten bezeichnet.

In Ausführungsform d) werden bevorzugt auch erfindungsgemäße Expressionseinheiten mit veränderter spezifischer Expressionsaktivität gemäß Ausführungsform c) eingesetzt. Diese können in Ausführungsform d), wie in Ausführungsform d) beschrieben im Mikroorganismus vorliegen und hergestellt werden oder in isolierter Form in den Mikroorganismus eingebracht werden.

Die Erfindung betrifft ferner in einer bevorzugten Ausführungsform ein Verfahren zur Erhöhung oder Verursachung der Expressionsrate eines Gens in Mikroorganismen im Vergleich zum Wildtyp indem man
ch) die spezifische Expressionsaktivität im Mikroorganismus von erfindungsgemäßen, endogenen Expressionseinheiten, die die Expression der endogenen Gene regulieren, im Vergleich zum Wildtyp erhöht oder
dh) die Expression von Genen im Mikroorganismus durch erfindungsgemäße Expressionseinheiten oder durch Expressionseinheiten mit erhöhter spezifischer Expressionsaktivität gemäß Ausführungsform c) reguliert, wobei die Gene im Bezug auf die Expressionseinheiten heterolog sind.

Vorzugsweise wird die Regulation der Expression von Genen im Mikroorganismus durch erfindungsgemäße Expressionseinheiten oder durch Expressionseinheiten mit erhöhter spezifischer Expressionsaktivität gemäß Ausführungsform c) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der erfindungsgemäßen, endogenen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Die Erfindung betrifft ferner ein Verfahren zur Reduzierung der Expressionsrate von Genen in Mikroorganismen im Vergleich zum Wildtyp, indem man
cr) die spezifische Expressionsaktivität im Mikroorganismus von endogenen, erfindungsgemäßen Expressionseinheiten, die die Expression der endogenen Gene regulieren, im Vergleich zum Wildtyp reduziert oder
dr) Expressionseinheiten mit reduzierter spezifischer Expressionsaktivität gemäß Ausführungsform cr) in das Genom des Mikroorganismus einbringt, so dass die Expression endogener Gene unter der Kontrolle der eingebrachten Expressionseinheiten mit reduzierter Expressionsaktivität erfolgt.

In einer bevorzugten Ausführungsform der vorstehend beschriebenen erfindungsgemäßen Verfahren zur Veränderung oder Verursachung der Transkriptionsrate und/oder Expressionsrate von Genen in Mikroorganismen sind die Gene ausgewählt aus der Gruppe Nukleinsäuren kodierend ein Protein aus den Biosyntheseweg von Feinchemikalien, wobei die Gene gegebenenfalls weitere Regulationselemente enthalten können.

In einer besonders bevorzugten Ausführungsform der vorstehend beschriebenen erfindungsgemäßen Verfahren zur Veränderung oder Verursachung der Transkriptionsrate und/oder Expressionsrate von Genen in Mikroorganismen sind die Gene ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

In einer besondere bevorzugten Ausführungsform sind die Proteine aus dem Biosyntheseweg von Aminosäuren ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

Bevorzugte Proteine und Nukleinsäuren kodierend diese Proteine der vorstehend beschriebenen Proteine aus dem Biosyntheseweg von Aminosäuren sind Proteinsequenzen bzw. Nukleinsäuresequenzen mikrobiellen Ursprungs, vorzusgweise aus Bakterien der Gattung Corynebacterium oder Brevibacterium, bevorzugt aus coryneformen Bakterien, besonders bevorzugt aus Corynebakterium glutamicum.

Beispiele für besonders bevorzugte Proteinsequenzen und die entsprechenden Nukleinsäuresequenzen kodierend diese Proteine aus dem Biosyntheseweg von Aminosäuren, deren Bezugsdokument, sowie deren Bezeichnung im Bezugsdokument sind in Tabelle 1 aufgelistet:

**Tabelle 1**

| Protein | Nukleinsäure kodierend Protein | Bezugsdokument | SEQ. ID. NO. im Bezugsdokument |
|---|---|---|---|
| Aspartatkinase | ask oder lysC | EP1108790 | DNA: 281 |
| | | | Protein: 3781 |
| Aspartat-Semialdehyd-Dehydrogenase | asd | EP1108790 | DNA: 331 |
| | | | Protein: 3831 |
| Dihydrodipicolinate-Synthetase | dapA | WO 0100843 | DNA: 55 |
| | | | Protein: 56 |
| | | | |
| Dihydrodipicolinate-Reduktase | dapB | WO 0100843 | DNA: 35 |
| | | | Protein: 36 |
| | | | |
| Meso-Diaminopimelat-D-Dehydrogenase | ddh | EP1108790 | DNA : 3494 |
| | | | Protein : 6944 |
| Diaminopicolinat-Decarboxylase | lysA | EP1108790 | DNA: 3451 |
| | | | Prot.:6951 |
| Lysin-Exporter | lysE | EP1108790 | DNA: 3455 |
| | | | Prot.: 6955 |
| Arginyl-t-RNA Synthetase | argS | EP1108790 | DNA: 3450 |
| | | | Prot.: 6950 |
| Glucose-6-Phosphat-Dehydrognease | zwf | WO 0100844 | DNA: 243 |
| | | | Prot.: 244 |
| Glycerinaldehyd-3-Phosphat-Dehydrogenase | gap | WO 0100844 | DNA: 187 |
| | | | Prot.: 188 |
| | | | |
| 3-Phosphoglycerat-kinase | pgk | WO 0100844 | DNA: 69 |
| | | | Prot.: 70 |
| Pyruvat-Carboxylase | pycA | EP1108790 | DNA: 765 |
| | | | Prot.: 4265 |
| Triosephosphat-Isomerase | tpi | WO 0100844 | DNA: 61 |
| | | | Prot.: 62 |
| Biotin-Ligase | birA | EP1108790 | DNA: 786 |
| | | | Prot.: 4286 |
| PEP-Carboxylase | pck | EP1108790 | DNA: 3470 |
| | | | Prot.: 6970 |
| Homoserin Kinase | thrB | WO 0100843 | DNA: 173 |
| | | | Prot.: 174 |
| Threonin Synthase | thrC | WO 0100843 | DNA: 175 |
| | | | Prot.: 176 |
| Threonin Export Carrier | thrE | WO 0251231 | DNA: 41 |
| | | | Prot.: 42 |
| Threonin Efflux Protein | RXA2390 | WO 0100843 | DNA: 7 |
| | | | Prot.: 8 |
| Threonin Dehydratase | ilvA | EP 1108790 | DNA: 2328 |
| | | | Prot.: 5828 |
| Homoserin-O-Acetyltransferase | metA | EP 1108790 | DNA:727 |
| | | | Prot: 4227 |
| Cystathionin-gamma-synthase | metB | EP 1108790 | DNA:3491 |
| | | | Prot: 6991 |
| Cystathionin-beta-Lyase | metC | EP 1108790 | DNA:2535 |
| | | | Prot: 6035 |
| Coenzym B12-abhängige Methionin-Synthase,- | metH | EP 1108790 | DNA:1663 |
| | | | Prot: 5163 |
| | | | |
| O-Acetylhomoserin-Sulfhydrylase | metY | EP 1108790 | DNA:726 |
| | | | Prot: 4226 |
| Methylentetrahydro-folat-Reduktase | metF | EP 1108790 | DNA:2379 |
| | | | Prot: 5879 |
| D-3-Phosphoglycerat-Dehydrogenase | serA | EP 1108790 | DNA:1415 |
| | | | Prot: 4915 |
| Phosphoserin-Phosphatase 1 | serB | WO 0100843 | DNA: 153 |
| | | | Prot.:154 |
| Phosphoserin-Phosphatase 2 | serB | EP 1108790 | DNA: 467 |
| | | | Prot: 3967 |
| Phosphoserin-Phosphatase 3 | serB | EP 1108790 | DNA: 334 |
| | | | Prot.: 3834 |
| Phosphoserin-Aminotransferase | serC | WO 0100843 | DNA: 151 |
| | | | Prot.: 152 |
| Serin AcetylTransferase | cysE | WO 0100843 | DNA: 243 |
| | | | Prot.: 244 |
| Cystein-Synthase I | cysK | EP 1108790 | DNA: 2817 |
| | | | Prot.: 6317 |
| Cystein Synthase II | CysM | EP 1108790 | DNA: 2338 |
| | | | Prot.: 5838 |
| Homoserin-Dehydrogenase | hom | EP 1108790 | DNA: 3452 |
| | | | Prot.: 6952 |
| | | | |
| Coenzym B12-unabhängige Methionin-Synthase | metE | WO 0100843 | DNA:755 |
| | | | Prot.: 756 |
| | | | |
| Serin-Hydroxymethyltransferase | glyA | WO 0100843 | DNA: 143 |
| | | | Prot.: 144 |
| | | | |
| Protein in Sulfat-Reduktion | RXA247 | EP 1108790 | DNA: 3089 |
| | | | Prot.: 6589 |
| Protein in Sulfat-Reduktion | RXA248 | EP 1108790 | DNA: 3090 |
| | | | Prot.: 6590 |
| Sulfatadenyltransferase Untereinheit 1 | CysN | EP 1108790 | DNA: 3092 |
| | | | Prot.: 6592 |
| Sulfatadenyltransferase Untereinheit 2 | CysD | EP 1108790 | DNA: 3093 |
| | | | Prot.: 6593 |
| Phosphoadenosin Phosphosulfat Reduktase | CysH | WO 02729029 | DNA: 7 |
| | | | Prot.: 8 |
| | | | |
| Ferredoxin-Sulfit-Reduktase | RXA073 | WO 0100842 | DNA: 329 |
| | | | Prot.: 330 |
| Ferredoxin NADP Reduktase | RXA076 | WO 0100843 | DNA: 79 |
| | | | Prot.: 80 |
| Transkriptioneller Re-gulator LuxR | luxR | WO 0100842 | DNA: 297 |
| | | | Protein: 298 |
| Transkriptioneller Regulator LysR1 | lysR1 | EP 1108790 | DNA: 676 |
| | | | Protein: 4176 |
| Transkriptioneller Regulator LysR2 | lysR2 | EP 1108790 | DNA: 3228 |
| | | | Protein: 6728 |
| Transkriptioneller Regulator LysR3 | lysR3 | EP 1108790 | DNA: 2200 |
| | | | Protein: 5700 |
| Malat-Quinon-Oxodoreduktase | mqo | WO 0100844 | DNA: 569 |
| | | | Protein: 570 |
| Transketolase | RXA2739 | EP 1108790 | DNA: 1740 |
| | | | Prot: 5240 |
| Transaldolase | RXA2738 | WO 0100844 | DNA: 245 |
| | | | Prot: 246 |
| OPCA | opcA | WO 0100804 | DNA: 79 |
| | | | Prot: 80 |
| 1-Phosphofructokinase 1 | pfk1 | WO010084 | DNA: 55 |
| | | | Protein: 56 |
| 1-Phosphofructokinase 2 | pfk2 | WO0100844 | DNA: 57 |
| | | | Protein: 58 |
| 6-Phosphofructokinase 1 | 6-pfk1 | EP 1108790 | DNA: 1383 |
| | | | Protein: 4883 |
| 6-Phosphofructokinase 2 | 6-pfk2 | DE 10112992 | DNA: 1 |
| | | | Protein: 2 |
| Fructose-1,6-bisphosphatase 1 | fbr1 | EP1108790 | DNA: 1136 |
| | | | Protein: 4636 |
| Pyruvat Oxidase | poxB | WO 0100844 | DNA : 85 |
| | | | Protein: 86 |
| RXA00655-Regulator | RXA655 | US2003162267 | DNA: 1 |
| | | .2 | Prot.: 2 |
| RXN02910-Regulator | RXN2910 | US2003162267 | DNA: 5 |
| | | .2 | Prot.: 6 |
| 6-phosphogluconolactonase | RXA2735 | WO 0100844 | DNA: 1 |
| | | | Prot.: 2 |
| | | | |

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz der Fructose-1,6-bisphosphatase 2, oder auch fbr2 genannt, (SEQ. ID. NO. 51) und die entsprechenden Nukleinsäuresequenz kodierend eine Fructose-1,6-bisphosphatase 2 (SEQ. ID. NO. 50).

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz des Proteins in Sulfat-Reduktion, oder auch RXA077 genannt, (SEQ. ID. NO. 4) und die entsprechenden Nukleinsäuresequenz kodierend ein Protein in Sulfat-Reduktion (SEQ. ID. NO. 3)

Weitere besonders bevorzugte Proteinsequenzen aus dem Biosyntheseweg von Aminosäuren, weisen jeweils die in Tabelle 1 für dieses Protein angegebene Aminosäuresequenz auf, wobei das jeweilige Protein jeweils an mindestens einer der in Tabelle 2/Spalte2 für diese Aminosäuresequenz angegebenen Aminosäurepositionen eine andere proteinogene Aminosäure aufweist als die jeweilige in Tabelle2/Spalte3 in der gleichen Zeile angegebene Aminosäure. In einer weiter bevorzugten Ausführungsform, weisen die Proteine an mindestens einer der in Tabelle 2/Spalte 2 für die Aminosäuresequenz angegebenenen Aminosäureposition die in Tabelle2/Spalte4 in der gleichen Zeile angegebene Aminosäure auf. Es handelt sich bei den in Tabelle 2 angegebenen Proteine um mutierte Proteine des Biosyntheseweges von Aminosäuren, die besonders vorteilhafte Eigenschaften aufweisen und sich deshalb insbesondere zur Expression der entsprechenden Nukleinsäuren durch den erfinungsgemäßen Promotor und zur Herstellung von Aminosäuren eignen. Beispielsweise führt die Mutation T311I zu einem Ausschalten der feedback-Inhibierung von ask.

Die entsprechenden Nukleinsäuren, die ein vorstehend beschriebenes mutiertes Protein aus Tabelle 2 kodieren, lassen sich durch übliche verfahren herstellen.

Als Ausgangspunkt zur Herstellung der Nukleinsäuresequenzen kodierend ein mutiertes Protein eignet sich beispielsweise das Genom eines Corynebacterium glutamicum-Stammes, der von der American Type Culture Collection unter der Bezeichnung ATCC 13032 erhältlich ist oder die in Tabelle 1 in Bezug genommenen Nukleinsäuresequenzen. Für die Rückübersetzung der Aminosäuresequenz der mutierten Proteine in die Nukleinsäuresequenzen kodierend diese Proteine ist es vorteilhaft, die codon usage desjenigen Organismus zu verwenden, in den die Nukleinsäuresequenz eingebracht werden soll oder in der die Nukleinsäuresequenz vorliegt. Beispielsweise ist es vorteihaft für Corynebakterium glutamicum die codon usage von Corynebakterium glutamicum zu verwenden. Die codon usage des jeweiligen Organismus lässt sich in an sich bekannter Weise aus Datenbanken oder Patentanmeldungen ermitteln, die zumindest ein Protein und ein Gen, das dieses Protein kodiert, aus dem gewünschten Organismus beschreiben.

Die Angaben in Tabelle 2 sind folgendermassen zu verstehen:

In Spalte 1 "Identifikation" wird eine eindeutige Bezeichnung für jede Sequenz in Bezug auf Tabelle 1 aufgeführt.

In Spalte 2 "AS-POS" bezieht sich die jeweilige Zahl auf die Aminosäureposition der entsprechenden Polypeptidsequenz aus Tabelle 1. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 3 "AS-Wildtyp" bezeichnet der jeweilige Buchstabe die Aminosäure - dargestellt im Ein-Buchstaben-Code- an der in Spalte 2 angegebenen Position beim entsprechenden Wildtyp-Stamm der Sequenz aus Tabelle 1.

In Spalte 4 "AS-Mutante" bezeichnet der jeweilige Buchstabe die Aminosäure - dargestellt im Ein-Buchstaben-Code- an der in Spalte 2 angegebenen Position beim entsprechenden Mutanten-Stamm.

In Spalte 5 "Funktion" wird die physiologische Funktion der entsprechenden Polypeptidsequenz aufgeführt.

Für ein mutiertes Protein mit einer bestimmten Funktion (Spalte 5) und einer bestimmten Ausgangsaminosäuresequenz (Tabelle 1) werden in den Spalten 2,3 und 4 mindestens eine Mutation, bei einigen Sequenzen auch mehrere Mutationen beschrieben. Diese mehreren Mutationen beziehen sich immer auf die jeweils obenstehende, nächstliegendste Ausgangsaminosäuresequenz (Tabelle 1). Unter dem Begriff "mindestens eine der Aminsäurepositionen" einer bestimmten Aminosäuresequenz wird vorzugsweise mindestens eine der für diese Aminosäuresequenz in Spalte 2, 3 und 4 beschriebenen Mutationen verstanden.

Ein-Buchstaben-Code der proteinogenen Aminosäuren:
A Alanin
C Cystein
D Aspartat
E Glutamat
FPhenylalanin
G Glycin
H His
I Isoleucin
K Lysin
L Leucin
M Methionin
N Asparagin
P Prolin
Q Glutamin
R Arginin
S Serin
TThreonin
V Valin
W Tryptophan
Y Tyrosin

**Tabelle 2**

| Spalte 1 | Spalte 2 | Spalte 3 | Spalte 4 | Spalte 5 |
|---|---|---|---|---|
| Identifikation | AS Position | AS Wildtyp | AS Mutante | Funktion |
| ask | 317 | S | A | Aspartatkinase |
| | 311 | T | I | |
| | 279 | A | T | |
| asd | 66 | D | G | Aspartat-Semialdehyd-Dehydrogenase |
| | 234 | R | H | |
| | 272 | D | E | |
| | 285 | K | E | |
| | 20 | L | F | |
| dapA | 2 | S | A | Dihydrodipicolinat Synthetase |
| | 84 | K | N | |
| | 85 | L | V | |
| dapB | 91 | D | A | Dihydrodipicolinat-Reduktase |
| | 83 | D | N | |
| ddh | 174 | D | E | Meso-Diaminopimelat-D-Dehydrogenase |
| | 235 | F | L | |
| | 237 | S | A | |
| lysA | 265 | A | D | Diaminopicolinat-Decarboxylase |
| | 320 | D | N | |
| | 332 | I | V | |
| argS | 355 | G | D | Arginyl-t-RNA-Synthetase |
| | 156 | A | S | |
| | 513 | V | A | |
| | 540 | H | R | |
| zwf | 8 | S | T | Glucose-6-Phosphat-Dehydrogenase |
| | 150 | T | A | |
| | 321 | G | S | |
| gap | 264 | G | S | Glycerinaldehyd-3-Phosphat-Dehydrogenase |
| pycA | 7 | S | L | Pyruvat-Carboxylase |
| | 153 | E | D | |
| | 182 | A | S | |
| | 206 | A | S | |
| | 227 | H | R | |
| | 455 | A | G | |
| | 458 | P | S | |
| | 639 | S | T | |
| | 1008 | R | H | |
| | 1059 | S | P | |
| | 1120 | D | E | |
| pck | 162 | H | Y | PEP-Carboxylase |
| | 241 | G | D | |
| | 829 | T | R | |
| thrB | 103 | S | A | Homoserin Kinase |
| | 190 | T | A | |
| | 133 | A | V | |
| | 138 | P | S | |
| thrC | 69 | G | R | Threonin Synthase |
| | 478 | T | I | |
| RXA330 | 85 | I | M | Threonin-Efflux Protein |
| | 161 | F | I | |
| | 195 | G | D | |
| hom | 104 | V | I | Homoserin-Deydrogenase |
| | 116 | T | I | |
| | 148 | G | A | |
| | 59 | V | A | |
| | 270 | T | S | |
| | 345 | R | P | |
| | 268 | K | N | |
| | 61 | D | H | |
| | 72 | E | Q | |
| lysR1 | 80 | R | H | transkriptioneller Regulator LysR1 |
| lysR3 | 142 | R | W | transkriptioneller Regulator LysR3 |
| | 179 | A | T | |
| RXA2739 | 75 | N | D | Transketolase |
| | 329 | A | T | |
| | 332 | A | T | |
| | 556 | V | I | |
| RXA2738 | 242 | K | M | Transaldolase |
| opcA | 107 | Y | H | OpcA |
| | 219 | K | N | |
| | 233 | P | S | |
| | 261 | Y | H | |
| | 312 | S | F | |
| | 65 | G | R | Aspartat-1-Decarboxylase |
| | 33 | G | S | 6- Phosphogluconolactonase |

In den vorstehend beschriebenen erfindungsgemäßen Verfahren zur Veränderung oder Verursachung der Transkriptionsrate und/oder Expressionsrate von Genen in Mikroorganismen sowie den nachstehend beschriebenen Verfahren zur Herstellung von genetisch veränderten Mikroorganismen, den nachstehend beschriebenen genetisch veränderten Mikroorganoismen und den nachstehend beschriebenen Verfahren zur Herstellung von biosynthetischen Produkten erfolgt das Einbringen der erfindungsgemäßen Nukleinsäuren mit Promotoraktivität, der erfindungsgemäßen Expressionseinheiten, der vorstehend beschriebenen Gene und der vorstehend beschriebnen Nukleinsäurekonstrukte oder Expressionskasetten in den Mikroorganismus, insbeondere in corynefome Bakterien, vorzugsweise durch die SacB-Methode.

Die SacB-Methode ist dem Fachmann bekannt und beispielsweise in Schäfer A, Tauch A, Jäger W, Kalinowski J, Thierbach G, Pühler A.; Small mobilizable multi-purpose cloning vectors derived from the Escherichia coli plasmids pK18 and pK19: selection of defined deletions in the chromosome of Corynebacterium glutamicum, Gene. 1994 Jul 22;145(1):69-73 und Blomfield IC, Vaughn V, Rest RF, Eisenstein BI.; Allelic exchange in Escherichia coli using the Bacillus subtilis sacB gene and a temperature-sensitive pSC101 replicon; Mol Microbiol. 1991 Jun;5(6):1447-57 beschrieben.

In einer bevorzugten Ausführungsform der vorstehend beschriebenen erfindungsgemäßen Verfahren erfolgt die Veränderung oder Verursachung der Transkriptionsrate und/oder Expressionsrate von Genen in Mikroorganismen durch Einbringen von erfindungsgemäßen Nukleinsäuren mit Promotoraktivität bzw. erfindungsgemäßen Expressionseinheiten in den Mikroorganismus.

In einer weiteren bevorzugten Ausführungsform der vorstehend beschriebenen erfindungsgemäßen Verfahren erfolgt die Veränderung oder Verursachung der Transkriptionsrate und/oder Expressionsrate von Genen in Mikroorganismen durch Einbringen der vorstehend beschriebenen Nukleinsäurekonstrukte oder Expressionskasetten in den Mikroorganismus.

Die Erfindung betrifft daher ferner eine Expressionskassette, umfassend
mindestens eine erfindungsgemäße Expressionseinheit
mindestens eine weitere, zu exprimierende Nukleinsäuresequenz, also ein zu exprimierendes Gen und
gegebenenfalls weitere genetische Kontrollelemente, wie beispielsweise einen Terminator,
wobei mindestens eine Expressionseinheit und eine weitere, zu exprimierende, Nukleinsäuresequenz funktionell miteinander verknüpft sind und die weitere, zu exprimierende, Nukleinsäuresequenz in Bezug auf die Expressionseinheit heterolog ist.

Vorzugsweise ist die zu exprimierende Nukleinsäuresequenz mindestens eine Nukleinsäuren kodierend ein Protein aus den Biosyntheseweg von Feinchemikalien.

Besonders bevorzugt ist die zu exprimierende Nukleinsäuresequenz ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen.

Bevorzugte Proteine aus dem Biosyntheseweg von Aminosäuren sind vorstehend und deren Beispiele in Tabelle 1 und 2 beschrieben.

In den erfindungsgemäßen Expressionskassetten ist die physikalische Lage der Expressionseinheit relativ zum zu exprimierenden Gen so gewählt, daß die Expressioneinheit die Transkription und vorzugsweise auch die Translation des zu exprimierenden Gens reguliert und damit die Bildung eines oder mehrerer Proteine ermöglicht. Die "Bildung ermöglichen" beinhaltet dabei die konstitutive Steigerung der Bildung, Abschwächung bzw. Blockierung der Bildung unter spezifischen Bedingungen und oder die Steigerung der Bildung unter spezifischen Bedingungen. Die "Bedingungen" umfassen dabei: (1) Zugabe einer Komponente zum Kulturmedium, (2) Entfernen einer Komponente vom Kulturmedium, (3) Austausch einer Komponente im Kulturmedium durch eine zweite Komponente, (4) Erhöhung der Temperatur des Kulturmediums, (5) Erniedrigung der Temperatur des Kulturmediums, und (6) Regulierung der atmosphärischen Bedingungen, wie z.B. die Sauerstoff- oder Stickstoffkonzentration, in der das Kulturmedium gehalten wird.

Die Erfindung betrifft ferner einen Expressionsvektor enthaltend eine vorstehend beschriebene, erfindungsgemäße Expressionskassette.

Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Plasmide eignen sich solche besonders bevorzugt, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS, oder solche, die auf pCG4 (US-A 4,489,160) oder pNG2 (Serwold-Davis et al., FEMS Microbiology Letters 66,119-124 (1990)) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Sirnon et al., Bio/ Technology 1,784-791 (1983)), pK18mob oder pK19mob (Schäfer et al., Gene 145,69-73 (1994)), Bernard et al., Journal ofMolecular Biology, 234: 534-541 (1993)), pEM1 (Schrumpf et al. 1991, Journal of Bacteriology 173: 4510--4516) oder pBGS8 (Spratt et al., 1986, Gene 41: 337-342) in Frage. Der Plasmidvektor, der das zu amplifizierende Gen enthält, wird anschließend durch Transformation in den gewünschten Stamm von C. glutamicum überführt. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

Die Erfindung betrifft ferner einen genetisch verändertern Mikroorganismus, wobei die genetische Veränderung zu einer Veränderung oder Verursachung der Transkriptionsrate von mindestens einem Gen im Vergleich zum Wildtyp führt und bedingt ist durch
a) Veränderung der spezifischen Promotoraktivität im Mikroorganismus von mindestens einer endogenen Nukleinsäure mit Promotoraktivität gemäß Anspruch 1, die die Transkription mindestens eines endogenen Gens reguliert oder
b) Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a), wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht, dass man
b1) eine oder mehrere Nukleinsäuren mit Promotoraktivität gemäß Anspruch 1, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 1, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäß Anspruch 1, gegebenenfalls mit veränderter spezifischer Promotoraktivität, und funktionell verknüpft eine oder mehrere, zu transkribierende Nukleinsäuren, in den Mikroorganismus einbringt.

Die Erfindung betrifft ferner eine genetisch verändertern Mikroorganismus mit erhöhter oder verursachter Transkriptionsrate von mindestens einem Gen im Vergleich zum Wildtyp, wobei
ah) die spezifische Promotoraktivität im Mikroorganismus von endogenen Nukleinsäuren mit Promotoraktivität gemäß Anspruch 1, die die Transkription von endogenen Genen regulieren, im Vergleich zum Wildtyp erhöht ist oder
bh) die Transkription von Genen im Mikroorganismus durch Nukleinsäuren mit Promotoraktivität gemäß Anspruch 1 oder durch Nukleinsäuren mit erhöhter spezifischer Promotoraktivität gemäß Ausführungsform ah) reguliert wird, wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch Nukleinsäuren mit Promotoraktivität gemäß Anspruch 1 oder durch Nukleinsäuren mit Promotoraktivität gemäß Anspruch 1 mit veränderter spezifischer Promotoraktivität gemäß Ausführungsform a) dadurch erreicht, dass man
bh1) eine oder mehrere Nukleinsäuren mit Promotoraktivität gemäß Anspruch 1, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäß Anspruch 1, gegebenenfalls mit erhöhter spezifischer Promotoraktivität, und funktionell verknüpft eine oder mehrere, zu transkribierende Nukleinsäuren, in den Mikroorganismus einbringt.

Die Erfindung betrifft ferner einen genetisch verändertern Mikroorganismus mit reduzierter Transkriptionsrate von mindetstens einem Gen im Vergleich zum Wildtyp, wobei
ar)die spezifische Promotoraktivität im Mikroorganismus von mindestens einer endogenen Nukleinsäure mit Promotoraktivität gemäß Anspruch 1, die die Transkription von mindestens einem, endogenen Gen reguliert, im Vergleich zum Wildtyp reduziert ist oder
br)eine oder mehrere Nukleinsäuren mit reduzierter Promotoraktivität gemäßAusführungsform a) in das Genom des Mikrorganismus eingebracht wurden, so dass die Transkription mindestens eines endogenen Gens unter der Kontrolle der eingebrachten Nukleinsäure mit reduzierter Promotoraktivität erfolgt.

Die Erfindung betrifft ferner einen genetisch verändertern Mikroorganismus, wobei die genetische Veränderung zu einer Veränderung oder Verursachung der Expressionsrate mindestens eines Gens im Vergleich zum Wildtyp führt und bedingt ist durch
c) Veränderung der spezifischen Expressionsaktivität im Mikroorganismus von mindestens einer endogenen Expressionseinheiten gemäß Anspruch 2 oder 3, die die Expression mindestens eines endogenen Gens reguliert, im Vergleich zum Wildtyp oder
d) Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a), wobei die Gene im Bezug auf die Expressionseinheiten heterolog sind.

Wie vorstehend bei den Verfahren beschrieben, wird die Regulation der Expression von Genen im Mikroorganismus durch Expressionseinheiten gemäß Anspruch 2 oder 3 oder durch Expressionseinheiten gemäß Anspruch 2 oder 3 mit veränderter spezifischer Expressionsaktivität gemäß Ausführungsform a) dadurch erreicht, dass man
d1) eine oder mehrere Expressionseinheiten gemäß Anspruch 2 oder 3, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten gemäß Anspruch 2 oder 3, gegebenenfalls mit veränderter spezifischer Expressionsaktivität, erfolgt oder
d2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit veränderter spezifischer Expressionsaktivität, erfolgt oder
d3) ein oder mehrere Nukleinsäurekonstrukte, enthaltend eine Expressionseinheit gemäß Anspruch 2 oder 3, gegebenenfalls mit veränderter spezifischer Expressionsaktivität, und funktionell verknüpft eine oder mehrere, zu exprimierende Nukleinsäuren, in den Mikroorganismus einbringt.

Die Erfindung betrifft ferner einen genetisch verändertern Mikroorganismus mit erhöhter oder verursachter Expressionsrate mindestens eines Gens im Vergleich zum Wildtyp, wobei man
ch) die spezifische Expressionsaktivität im Mikroorganismus von mindestens einer endogenen Expressionseinheiten gemäß Anspruch 2 oder 3, die die Expression der endogenen Gene reguliert, im Vergleich zum Wildtyp erhöht oder
dh) die Expression von Genen im Mikroorganismus durch Expressionseinheiten gemäß Anspruch 2 oder 3 oder durch Expressionseinheiten gemäß Anspruch 2 oder 3 mit erhöhter spezifischer Expressionsaktivität gemäß Ausführungsform a) reguliert, wobei die Gene im Bezug auf die Expressionseinheiten heterolog sind.

Wie vorstehend bei den Verfahren beschrieben, wird die Regulation der Expression von Genen im Mikroorganismus durch Expressionseinheiten gemäß Anspruch 2 oder 3 oder durch Expressioriseinheiten gemäß Anspruch 2 oder 3 mit erhöhter spezifischer Expressionsaktivität gemäß Ausführungsform a) dadurch erreicht, dass man
dh1) eine oder mehrere Expressionseinheiten gemäß Anspruch 2 oder 3, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten gemäß Anspruch 2 oder 3, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der endogenen Expressionseinheiten gemäß Anspruch 2 oder 3, gegebenenfalls mit erhöhter spezifischer Expressionsaktivität, erfolgt oder
dh3) ein oder mehrere Nukleinsäurekonstrukte, enthaltend eine Expressionseinheit gemäß Anspruch 2 oder 3, gegebenenfalls mit erhöhter spezifischer Expressionsaktivität, und funktionell verknüpft eine oder mehrere, zu exprimierende Nukleinsäuren, in den Mikroorganismus einbringt.

Die Erfidnung betrifft ferner einen genetisch verändertern Mikroorganismus mit reduzierter Expressionsrate von mindetstens einem Gen im Vergleich zum Wildtyp, wobei,
cr) die spezifische Expressionsaktivität im Mikroorganismus von mindestens einer endogenen Expressionseinheit gemäß Anspruch 2 oder 3, die die Expression von mindestens einem edogenen Gen reguliert, im Vergleich zum Wildtyp reduziert ist oder
dr)eine oder mehrere Expressionseinheiten gemäß Anspruch 2 oder 3 mit reduzierter Expressionsaktivität in das Genom des Mikrorganismus eingebracht wurden, so dass die Expression mindestens eines Gens unter der Kontrolle der eingebrachten Expressionseinheit gemäß Anspruch 2 oder 3 mit reduzierter Expressionsaktivität erfolgt.

Ferner betrifft die Erfindung einen genetisch veränderter Mikroorganismus, enthaltend eine Expressionseinheit gemäß Anspruch 2 oder 3 und funktionell verknüpft ein zu eprimierendes Gen, wobei das Gen im Bezug auf die Expressionseinheit heterolog ist.

Besonders bevorzugt enthält dieser genetisch veränderte Mikroorganismus eine erfindungsgemäße Expressionskasette.

Besonders bevorzugt betrifft die vorliegende Erfindung gentisch veränderte Mikroorgansismen, insbesondere coryneforme Bakterien, die einen Vektor, insbesondere Pendelvektor oder Plasmidvektor, der wenigstens ein rekombinantes Nukleinsäurekonstrukt erfindungsgemäßer Definition trägt, enthalten.

In einer bevorzugten Ausführungsform der genetisch veränderten Mikroorganismen sind die vorstehend beschriebenen Gene mindestens eine Nukleinsäuren kodierend ein Protein aus den Biosyntheseweg von Feinchemikalien.

In einer besonders bevorzugten Ausführungsform der genetisch veränderten Mikroorganismen sind die vorstehend beschriebenen Gene ausgewählt sind aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

Bevorzugte Proteine aus dem Biosyntheseweg von Aminosäuren sind ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase

Besonders bevorzugte Beispiele der Proteine und Gene aus dem Biosyntheseweg von Aminosäuren sind vorstehend in Tabelle 1 und Tabelle 2 beschrieben.

Bevorzugte Mikroorganismen oder genetisch veränderte Mikroorganismen sind Bakterien, Algen, Pilze oder Hefen.

Besonders bevorzugte Mikroorgansimen sind insbeondere coryneforme Bakterien.

Bevorzugte coryneforme Bakterien sind Bakterien der Gattung Corynebacterium, insbesondere der Arten Corynebacterium glutamicum, Corynebacterium acetoglutamicum, Corynebacterium acetoacidophilum, Corynebacterium thermoaminogenes, Corynebacterium melassecola und Corynebacterium efficiens oder der Gattung Brevibacterium, insbesondere der Arten Brevibacterium flavum, Brevibacterium lactofermentum und Brevibacterium divaricatum.

Besonders bevorzugte Bakterien der Gattungen Corynebacterium und Brevibacterium sind ausgewählt aus der Gruppe Corynebacterium glutamicum ATCC 13032, Corynebacterium acetoglutamicum ATCC 15806, Corynebacterium acetoacidophilum ATCC 13870, Corynebacterium thermoaminogenes FERM BP-1539, Corynebacterium melassecola ATCC 17965, Corynebacterium efficiens DSM 44547, Corynebacterium efficiens DSM 44548. Corynebacterium efficiens DSM 44549, Brevibacterium flavum ATCC 14067, Brevibacterium lactofermentum ATCC 13869, Brevibacterium divaricatum ATCC 14020, Corynebacterium glutamicum KFCC10065 und Corynebacterium glutamicum ATCC21608.

Mit der Abkürzung KFCC ist die Korean Federation of Culture Collection gemeint, mit der Abkürzung ATCC die American type strain culture collection, mit der Abkürzung DSM die Deutsche Sammlung von Mikroorganismen.

Weitere besonders bevorzugte Bakterien der Gattungen Corynebacterium und Brevibacterium sind in Tabelle 3 aufgelistet:

| Bakterium | | Hinterlegungsnummer | | | | | | | |
|---|---|---|---|---|---|---|---|---|---|
| Genus | species | ATCC | FERM | NRRL | CEC T | NCIMB | CBS | NCT C | DSM Z |
| Brevibacterium | ammoniagenes | 21054 | | | | | | | |
| Brevibacterium | ammoniagenes | 19350 | | | | | | | |
| Brevibacterium | ammoniagenes | 19351 | | | | | | | |
| Brevibacterium | ammoniagenes | 19352 | | | | | | | |
| Brevibacterium | ammoniagenes | 19353 | | | | | | | |
| Brevibacterium | ammoniagenes | 19354 | | | | | | | |
| Brevibacterium | ammoniagenes | 19355 | | | | | | | |
| Brevibacterium | ammoniagenes | 19356 | | | | | | | |
| Brevibacterium | ammoniagenes | 21055 | | | | | | | |
| Brevibacterium | ammoniagenes | 21077 | | | | | | | |
| Brevibacterium | ammoniagenes | 21553 | | | | | | | |
| Brevibacterium | ammoniagenes | 21580 | | | | | | | |
| Brevibacterium | ammoniagenes | 39101 | | | | | | | |
| Brevibacterium | butanicum | 21196 | | | | | | | |
| Brevibacterium | divaricatum | 21792 | P928 | | | | | | |
| Brevibacterium | flavum | 21474 | | | | | | | |
| Brevibacterium | flavum | 21129 | | | | | | | |
| Brevibacterium | flavum | 21518 | | | | | | | |
| Brevibacterium | flavum | | | B1147 4 | | | | | |
| Brevibacterium | flavum | | | B1147 2 | | | | | |
| Brevibacterium | flavum | 21127 | | | | | | | |
| Brevibacterium | flavum | 21128 | | | | | | | |
| Brevibacterium | flavum | 21427 | | | | | | | |
| Brevibacterium | flavum | 21475 | | | | | | | |
| Brevibacterium | flavum | 21517 | | | | | | | |
| Brevibacterium | flavum | 21528 | | | | | | | |
| Brevibacterium | flavum | 21529 | | | | | | | |
| Brevibacterium | flavum | | | B1147 7 | | | | | |
| Brevibacterium | flavum | | | B1147 8 | | | | | |
| Brevibacterium | flavum | 21127 | | | | | | | |
| Brevibacterium | flavum | | | B1147 4 | | | | | |
| Brevibacterium | healii | 15527 | | | | | | | |
| Brevibacterium | ketoglutamicum | 21004 | | | | | | | |
| Brevibacterium | ketoglutamicum | 21089 | | | | | | | |
| Brevibacterium | ketosoreductum | 21914 | | | | | | | |
| Brevibacterium | lactofermentum | | | | 70 | | | | |
| Brevibacterium | lactofermentum | | | | 74 | | | | |
| Brevibacterium | lactofermentum | | | | 77 | | | | |
| Brevibacterium | lactofermentum | 21798 | | | | | | | |
| Brevibacterium | lactofermentum | 21799 | | | | | | | |
| Brevibacterium | lactofermentum | 21800 | | | | | | | |
| Brevibacterium | lactofermentum | 21801 | | | | | | | |
| Brevibacterium | lactofermentum | | | B1147 0 | | | | | |
| Brevibacterium | lactofermentum | | | B1147 1 | | | | | |
| Brevibacterium | lactofermentum | 21086 | | | | | | | |
| Brevibacterium | lactofermentum | 21420 | | | | | | | |
| Brevibacterium | lactofermentum | 21086 | | | | | | | |
| Brevibacterium | lactofermentum | 31269 | | | | | | | |
| Brevibacterium | linens | 9174 | | | | | | | |
| Brevibacterium | linens | 19391 | | | | | | | |
| Brevibacterium | linens | 8377 | | | | | | | |
| Brevibacterium | paraffinolyticum | | | | | 11160 | | | |
| Brevibacterium | spec. | | | | | | 717.7 3 | | |
| Brevibacterium | spec. | | | | | | 717.7 3 | | |
| Brevibacterium | spec. | 14604 | | | | | | | |
| Brevibacterium | spec. | 21860 | | | | | | | |
| Brevibacterium | spec. | 21864 | | | | | | | |
| Brevibacterium | spec. | 21865 | | | | | | | |
| Brevibacterium | spec. | 21866 | | | | | | | |
| Brevibacterium | spec. | 19240 | | | | | | | |
| Corynebacterium | acetoacidophilu m | 21476 | | | | | | | |
| Corynebacteriu m | acetoacidophilu m | 13870 | | | | | | | |
| Corynebacteriu m | acetoglutamicu m | | | B1147 3 | | | | | |
| Corynebacteriu m | acetoglutamicu m | | | B1147 5 | | | | | |
| Corynebacteriu m | acetoglutamicu m | 15806 | | | | | | | |
| Corynebacteriu m | acetoglutamicu m | 21491 | | | | | | | |
| Corynebacteriu m | acetoglutamicu m | 31270 | | | | | | | |
| Corynebacteriu m | acetophilum | | | B3671 | | | | | |
| Corynebacteriu m | ammoniagenes | 6872 | | | | | | 2399 | |
| Corynebacteriu m | ammoniagenes | 15511 | | | | | | | |
| Corynebacteriu m | fujiokense | 21496 | | | | | | | |
| Corynebacteriu m | glutamicum | 14067 | | | | | | | |
| Corynebacteriu m | glutamicum | 39137 | | | | | | | |
| Corynebacteriu m | glutamicum | 21254 | | | | | | | |
| Corynebacteriu m | glutamicum | 21255 | | | | | | | |
| Corynebacteriu m | glutamicum | 31830 | | | | | | | |
| Corynebacteriu m | glutamicum | 13032 | | | | | | | |
| Corynebacteriu m | glutamicum | 14305 | | | | | | | |
| Corynebacteriu m | glutamicum | 15455 | | | | | | | |
| Corynebacteriu m | glutamicum | 13058 | | | | | | | |
| Corynebacteriu m | glutamicum | 13059 | | | | | | | |
| Corynebacteriu m | glutamicum | 13060 | | | | | | | |
| Corynebacteriu m | glutamicum | 21492 | | | | | | | |
| Corynebacteriu m | glutamicum | 21513 | | | | | | | |
| Corynebacteriu m | glutamicum | 21526 | | | | | | | |
| Corynebacteriu m | glutamicum | 21543 | | | | | | | |
| Corynebacteriu m | glutamicum | 13287 | | | | | | | |
| Corynebacteriu m | glutamicum | 21851 | | | | | | | |
| Corynebacteriu m | glutamicum | 21253 | | | | | | | |
| Corynebacteriu m | glutamicum | 21514 | | | | | | | |
| Corynebacteriu m | glutamicum | 21516 | | | | | | | |
| Corynebacteriu m | glutamicum | 21299 | | | | | | | |
| Corynebacteriu m | glutamicum | 21300 | | | | | | | |
| Corynebacteriu m | glutamicum | 39684 | | | | | | | |
| Corynebacteriu m | glutamicum | 21488 | | | | | | | |
| Corynebacteriu m | glutamicum | 21649 | | | | | | | |
| Corynebacteriu m | glutamicum | 21650 | | | | | | | |
| Corynebacteriu m | glutamicum | 19223 | | | | | | | |
| Corynebacteriu m | glutamicum | 13869 | | | | | | | |
| Corynebacteriu m | glutamicum | 21157 | | | | | | | |
| Corynebacteriu m | glutamicum | 21158 | | | | | | | |
| Corynebacteriu m | glutamicum | 21159 | | | | | | | |
| Corynebacteriu m | glutamicum | 21355 | | | | | | | |
| Corynebacteriu m | glutamicum | 31808 | | | | | | | |
| Corynebacteriu m | glutamicum | 21674 | | | | | | | |
| Corynebacteriu m | glutamicum | 21562 | | | | | | | |
| Corynebacteriu m | glutamicum | 21563 | | | | | | | |
| Corynebacteriu m | glutamicum | 21564 | | | | | | | |
| Corynebacteriu m | glutamicum | 21565 | | | | | | | |
| Corynebacteriu m | glutamicum | 21566 | | | | | | | |
| Corynebacteriu m | glutamicum | 21567 | | | | | | | |
| Corynebacteriu m | glutamicum | 21568 | | | | | | | |
| Corynebacteriu m | glutamicum | 21569 | | | | | | | |
| Corynebacteriu m | glutamicum | 21570 | | | | | | | |
| Corynebacteriu m | glutamicum | 21571 | | | | | | | |
| Corynebacteriu m | glutamicum | 21572 | | | | | | | |
| Corynebacteriu m | glutamicum | 21573 | | | | | | | |
| Corynebacteriu m | glutamicum | 21579 | | | | | | | |
| Corynebacteriu m | glutamicum | 19049 | | | | | | | |
| Corynebacteriu m | glutamicum | 19050 | | | | | | | |
| Corynebacteriu m | glutamicum | 19051 | | | | | | | |
| Corynebacteriu m | glutamicum | 19052 | | | | | | | |
| Corynebacteriu m | glutamicum | 19053 | | | | | | | |
| Corynebacteriu m | glutamicum | 19054 | | | | | | | |
| Corynebacteriu m | glutamicum | 19055 | | | | | | | |
| Corynebacteriu m | glutamicum | 19056 | | | | | | | |
| Corynebacteriu m | glutamicum | 19057 | | | | | | | |
| Corynebacteriu m | glutamicum | 19058 | | | | | | | |
| Corynebacteriu m | glutamicum | 19059 | | | | | | | |
| Corynebacteriu m | glutamicum | 19060 | | | | | | | |
| Corynebacteriu m | glutamicum | 19185 | | | | | | | |
| Corynebacteriu m | glutamicum | 13286 | | | | | | | |
| Corynebacteriu m | glutamicum | 21515 | | | | | | | |
| Corynebacteriu m | glutamicum | 21527 | | | | | | | |
| Corynebacteriu m | glutamicum | 21544 | | | | | | | |
| Corynebacteriu m | glutamicum | 21492 | | | | | | | |
| Corynebacteriu m | glutamicum | | | B8183 | | | | | |
| Corynebacteriu m | glutamicum | | | B8182 | | | | | |
| Corynebacteriu m | glutamicum | | | B1241 6 | | | | | |
| Corynebacteriu m | glutamicum | | | B1241 7 | | | | | |
| Corynebacteriu m | glutamicum | | | B1241 8 | | | | | |
| Corynebacteriu m | glutamicum | | | B1147 6 | | | | | |
| Corynebacteriu m | glutamicum | 21608 | | | | | | | |
| Corynebacterium | lilium | | P973 | | | | | | |
| Corynebacteriu m | nitrilophilus | 21419 | | | | 11594 | | | |
| Corynebacteriu m | spec. | | P444 5 | | | | | | |
| Corynebacteriu m | spec. | | P444 6 | | | | | | |
| Corynebacterium | spec. | 31088 | | | | | | | |
| Corynebacterium | spec. | 31089 | | | | | | | |
| Corynebacterium | spec. | 31090 | | | | | | | |
| Corynebacterium | spec. | 31090 | | | | | | | |
| Corynebacterium | spec. | 31090 | | | | | | | |
| Corynebacterium | spec. | 15954 | | | | | | | 20145 |
| Corynebacterium | spec. | 21857 | | | | | | | |
| Corynebacterium | spec. | 21862 | | | | | | | |
| Corynebacterium | spec. | 21863 | | | | | | | |

Die Abkürzungen haben folgende Bedeutung:
ATCC: American Type Culture Collection, Rockville, MD, USA
FERM: Fermentation Research Institute, Chiba, Japan
NRRL: ARS Culture Collection, Northern Regional Research Laboratory, Peoria, IL, USA
CECT: Coleccion Espanola de Cultivos Tipo, Valencia, Spain
NCIMB: National Collection of Industrial and Marine Bacteria Ltd., Aberdeen, UK
CBS: Centraalbureau voor Schimmelcultures, Baarn, NL
NCTC: National Collection of Type Cultures, London, UK
DSMZ: Deutsche Sammlung von Mikroorganismen und Zellkulturen, Braunschweig, Germany

Durch die erfindungsgemäßen Nukleinsäuren mit Promotoraktivität und den erfindungsgemäßen Expressionseinheiten ist es mit Hilfe der vorstehend beschriebenen, erfindungsgemäßen Verfahren möglich in den vorstehend beschriebenen, erfindungsgemäßen genetisch veränderten Mikroorganismen die Stoffwechselwege zu spezifischen biosynthetischen Produkten zu regulieren.

Dazu werden beispielsweise Stoffwechselwege die zu einem spezifischen biosynthetischen Produkt führen durch Verursachung oder Erhöhung der Transkriptionrate bzw. Expressionsrate von Genen dieses Biosyntheseweges verstärkt in dem die erhöhte Proteinmenge zu einer erhöhten Gesamtaktivität dieser Proteine des gewünschten Biosyntheseweges und damit zu einem verstärkten Stoffwechselfluß zu dem gewünschen biosynthetischen Produkt führt.

Weiterhin können Stoffwechselwege die von einem spezifischen biosynthetischen Produkt wegführen durch Reduzierung der Transkriptionrate bzw. Expressionsrate von Genen dieses wegführenden Biosyntheseweges abgeschwächt werden in dem die reduzierte Proteinmenge zu einer reduzierten Gesamtaktivität dieser Proteine des unerwünschten Biosyntheseweges und damit zusätzlich zu einem verstärkten Stoffwechselfluß zu dem gewünschen biosynthetischen Produkt führt.

Die erfindungsgemäßen genetisch veränderten Mikroorganismen sind beispielsweise in der Lage aus Glucose, Saccharose, Lactose, Fructose, Maltose, Melasse, Stärke, Cellulose oder aus Glycerin und Ethanol biosynthetische Produkte herzustellen.

Die Erfindung betrifft daher ein Verfahren zur Herstellung von biosynthetischen Produkten durch Kultivierung von erfindungsgemäßen, genetisch veränderten Mikroorganismen.

Je nach gewünschtem biosynthetischen Produkt muss die Transkriptionsrate bzw. Expressionsrate verschiedener Gene erhöht bzw. reduziert werden. In der Regel ist es vorteilhaft die Transkrioptionsrate bzw. Expressionsrate mehrere Gene zu verändern, d.h. die Transkrioptionsrate bzw. Expressionsrate einer Kombination von Gene zu Erhöhen und/oder die Transkrioptionsrate bzw. Expressionsrate einer Kombination von Gene zu reduzieren.

In den erfindungsgemäßen genetisch veränderten Mikroorganismen ist mindestens eine veränderte, dass heißt erhöhte oder reduzierte Transkriptionsrate bzw. Expressionsrate eines Gens auf eine erfindungsgemäße Nukleinsäure mit Promotoraktivität bzw. erfindungsgemäße Expressionseinheit zurückzuführen.

Weitere, zusätzliche veränderte, d.h. zusätzlich erhöhte oder zusätzlich reduzierte Transkriptionsraten bzw. Expressionsraten von weiteren Genen im genetisch veränderten Mikroorganismus können, müssen aber nicht auf die erfindungsgemäßen Nukleinsäuren mit Promotoraktivität bzw. die erfindungsgemäßen Expressionseinheiten zurück gehen.

Die Erfindung betrifft deshalb weiterhin ein Verfahren zur Herstellung von biosynthetischen Produkten durch Kultivierung von erfindungsgemäßen, genetisch veränderten Mikroorganismen.

Bevorzugte biosynthetische Produkte sind Feinchemikalien.

Der Begriff "Feinchemikalie" ist im Fachgebiet bekannt und beinhaltet Verbidnungen, die von einem Organismus produziert werden und in verschiedenen Industriezweigen Anwendungen finden, wie bspw., jedoch nicht beschränkt auf die pharmazeutische Industrie, die Landwirtschafts-, Kosmetik , Food und Feed-Industrie. Diese Verbindungen umfassen organische Säuren, wie beispielsweise Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### I. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität-ihrer Biosynthese mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation, s. Umbarger, H.E. (1978) Ann. Rev. Biochem. 47: 533 - 606). Glutamat wird durch reduktive Aminierung von - Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten- -Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### II. Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)- -alanin) kann entweder durch chemische Synthese oder durch Fermentation hergestellt werden. Die letzten Schritte bei der Pantothenat-Biosynthese bestehen aus der ATP-getriebenen Kondensation von -Alanin und Pantoinsäure. Die für die Biosyntheseschritte für die Umwandlung in Pantoinsäure, in -Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des -Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### III. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker verwendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### IV.Trehalose-Metabolismus und Verwendungen

Trehalose besteht aus zwei Glucosemolekülen, die über , -1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. Trends Biotech. 16(1998) 460-467; Paiva, C.L.A. und Panek, A.D. Biotech Ann. Rev. 2 (1996) 293-314; und Shiosaka, M. J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

Besonders bevorzugte biosynthetische Produkte sind ausgewählt aus der Gruppe organische Säuren, Proteine, Nukleotide und Nukleoside, sowohl proteinogene als auch nicht-proteinogene Aminosäuren, Lipide und Fettsäuren, Diole, Kohlehydrate, aromatische Verbindungen, Vitamine und Cofaktoren, Enzyme und Proteine.

Bevorzugte organische Säuren sind Weinsäure, Itaconsäure und Diaminopimelinsäure

Bevorzugte Nukleoside und Nukleotide sind beispielsweise beschrieben in Kuninaka, A. (1996) Nucleotides and related compounds, S. 561-612, in Biotechnology Bd. 6, Rehm et al., Hrsg. VCH: Weinheim und den darin enthaltenen Zitaten.

Bevorzugte biosynthetische Produkte sind weiterhin Lipide, gesättigte und ungesättigte Fettsäuren, wie beispielsweise Arachidonsäure, Diole wie beispielsweise Propandiol und Butandiol, Kohlenhydrate, wie beispielsweise Hyaluronsäure und Trehalose, aromatische Verbindungen, wie beispielsweise aromatische Amine, Vanillin und Indigo, Vitamine und Cofaktoren, wie beispielsweise beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research-Asien, abgehalten am 1.-3. Sept. 1994 in Penang, Malysia, AOCS Press (1995)), Enzyme Polyketide (Cane et al. (1998) Science 282: 63-68), und sämtliche anderen von Gutcho (1983) in Chemicals by Fermentation, Noyes Data Corporation, ISBN: 0818805086 und den darin angegebenen Literaturstellen, beschriebenen Chemikalien.

Besonders bevorzugte biosynthetische Produkte sind Aminosäuren, besonders bevorzugt essentielle Aminosäuren, insbeondere L-Glycin, L-Alanin, L-leucin, L-Methionin, L-Phenylalanin, L-Tryptophan, L-Lysin, L-Glutamin, L-Glutaminsäure, L-Serin, L-Prolin, L-Valin, L-Isoleucin, L-Cystein, L-Tyrosin, L-Histidin, L-Arginin, L-Asparagin, L-Asparaginsäure und L-Threonin, L-Homoserin, insbesondere L-Lysin, L-Methionin und L-Threonin. Im folgenden wird unter unter einer Aminosäure, wie beispielsweise Lysin, Methionin und Threonin, sowohl jeweils die L- und die D-Form der Aminosäure, vorzugsweise die L-Form, also beispielsweise L-Lysin, L-Methionin und L-Threonin verstanden.

Die Erfindung betrifft insbesondere ein Verfahren zur Herstellung von Lysin durch Kultivierung von genetisch veränderten Mikroorganismen mit erhöhter oder verursachter Expressionsrate mindestens eines Gens im Vergleich zum Wildtyp, wobei man
ch) die spezifische Expressionsaktivität im Mikroorganismus von mindestens einer endogenen, erfindungsgemäßen Expressionseinheit, die die Expression der endogenen Gene reguliert, im Vergleich zum Wildtyp erhöht oder
dh) die Expression von Genen im Mikroorganismus durch erfindungsgemäße Expressionseinheiten oder durch erfindungsgemäße Expressionseinheiten mit erhöhter spezifischer Expressionsaktivität gemäß Ausführungsform ch) reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Diaminopimelat- Dehydrogenase, Nukleinsäuren kodierend eine Diaminopimelat- Decarboxylase, Nukleinsäuren kodierend eine Dihydrodipicolinate-Synthetase, Nukleinsäuren kodierend eine Dihydridipicolinate-Reduktase, Nukleinsäuren kodierend eine Glycerinaldehyd-3-Phosphat Dehydrogenase, Nukleinsäuren kodierend eine 3-Phosphoglycerat-Kinase, Nukleinsäuren kodierend eine Pyruvat Carboxylase, Nukleinsäuren kodierend eine Triosephosphat-Isomerase, Nukleinsäuren kodierend einen Transkriptionellen Regulator LuxR, Nukleinsäuren kodierend einen Transkriptionellen Regulator LysR1, Nukleinsäuren kodierend einen Transkriptionellen Regulator LysR2, Nukleinsäuren kodierend eine Malat-Quinon-Oxodoreduktase, Nukleinsäuren kodierend eine Glucose-6-Phosphat-Deydrognease, Nukleinsäuren kodierend eine 6-Phosphogluconat-Dehydrognease, Nukleinsäuren kodierend eine Transketolase, Nukleinsäuren kodierend eine Transaldolase, Nukleinsäuren kodierend einen Lysin Exporter, Nukleinsäuren kodierend eine Biotin-Ligase, Nukleinsäuren kodierend eine Arginyl-t-RNA-Synthetase, Nukleinsäuren kodierend eine Phosphoenolpyruvat-Carboxylase, Nukleinsäuren kodierend eine Fruktose-1,6-bisphosphatase, Nukleinsäuren kodierend ein Protein OPCA, Nukleinsäuren kodierend eine 1-Phosphofructokinase und Nukleinsäuren kodierend eine 6-Phosphofructokinase.

Wie vorstehend bei den Verfahren beschrieben, wird die Regulation der Expression dieser Genen im Mikroorganismus durch erfindungsgemäße Expressionseinheiten oder durch erfindungsgemäße Expressionseinheiten mit erhöhter spezifischer Expressionsaktivität gemäß Ausführungsform ch) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer dieser endogenen Gene unter der Kontrolle der eingebrachten, erfindungsgemäßen Expressionseinheiten, gegebenenfalls mit erhöhter spezifischer Expressionsaktivität, erfolgt oder
dh2) ein oder mehrere dieser Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Lysin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Diaminopimelat- Dehydrogenase-Aktivität, Diaminopimelat-Decarboxylase-Aktivität, Dihydrodipicolinate-Synthetase-Aktivität, Dihydridipicolinate-Reduktase-Aktivität, Glycerinaldehyd-3-Phosphat Dehydrogenase-Aktivität, 3-Phosphoglycerat-Kinase-Aktivität, Pyruvat Carboxylase-Aktivität, Triosephosphat-Isomerase-Aktivität, Aktivität des Transkriptionellen Regulators LuxR, Aktivität des Transkriptionellen Regulators LysR1, Aktivität des Transkriptionellen Regulators LysR2, Malat-Quinon-Oxodoreduktase-Aktivität, Glucose-6-Phosphat-Deydrognease-Aktivität, 6-Phosphogluconat-Dehydrognease-Aktivität, Transketolase-Aktivität, Transaldolase-Aktivität, Lysin-Exporter-Aktivität, Arginyl-t-RNA-Synthetase-Aktivität, Phosphoenolpyruvat-Carboxylase-Aktivität, Fruktose-1,6-bisphosphatase-Aktivität, Protein OpcA-Aktivität, 1-Phosphofructokinase-Aktivität, 6-Phosphofructokinase-Aktivität und Biotin-Ligase-Aktivität aufweisen.

Eine weiter besonders bevorzugte Ausführungsform des vorstehend beschriebenen Verfahrens zur Herstellung von Lysin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, O-Acetylhomoserin-Sulfhydrylase-Aktivität, Phosphoenolpyruvat-Carboxykinase-Aktivität, Pyruvat-Oxidase-Aktivität, Homoserine-Kinase-Aktivität, Homoserin-Dehydrogenase-Aktivität, Threonin-Exporter-Aktivität, Threonin-Efflux-Protein-Aktivität, Asparaginase-Aktivität, Aspartat-Decarboxylase-Aktivität und Threonin-Synthase-Aktivität aufweisen.

Diese zusätzlichen, erhöhten oder reduzierten Aktivitäten mindestens einer der vorstehend beschriebenen Aktivitäten können, müssen aber nicht durch eine erfindungsgemäße Nukleinsäure mit Promotoraktivität und/oder eine erfindungsgemäße Expressionseinheit verursacht sein.

Die Erfindung betrifft ferner ein Verfahren zur Herstellung von Methionin durch Kultivierung von genetisch veränderten Mikroorganismen mit erhöhter oder verursachter Expressionsrate mindestens eines Gens im Vergleich zum Wildtyp, wobei man
ch) die spezifische Expressionsaktivität im Mikroorganismus von mindestens einer endogenen, erfindungsgemäßen Expressionseinheit, die die Expression der endogenen Gene reguliert, im Vergleich zum Wildtyp erhöht oder
dh) die Expression von Genen im Mikroorganismus durch erfindungsgemäße Expressionseinheiten oder durch erfindungsgemäße Expressionseinheiten mit erhöhter spezifischer Expressionsaktivität gemäß Ausführungsform ch) reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Homoserin Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin O-Acetyltransferase, Nukleinsäuren kodierend eine Cystahionin-gamma-Synthase, Nukleinsäuren kodierend eine Cystahionin-beta-Lyase, Nukleinsäuren kodierend eine Serin-Hydroxymethyltransferase, Nukleinsäuren kodierend eine O-Acetylhomoserin-Sulfhydrylase, Nukleinsäuren kodierend eine Methylen-Tetrahydrofolat-Reduktase, Nukleinsäuren kodierend eine Phosphoserin-Aminotransferase, Nukleinsäuren kodierend eine Phosphoserin-Phosphatase, Nukleinsäuren kodierend eine Serine Acetyl-TransferaseNukleinsäuren kodierend eine Cystein-Synthase Aktivität I, Nukleinsäuren kodierend eine Cystein-Synthase Aktivität II, Nukleinsäuren kodierend eine Coenzym B12-abhängige Methionin-Synthase-Aktivität, Nukleinsäuren kodierend eine Coenzym B12-unabhängige Methionin-Synthase-Aktivität, Nukleinsäuren kodierend eine Sulfat-Adenylyltransferase-Aktivität, Nukleinsäuren kodierend eine Phosphoadenosin-Phosphosulfat-Reductase-Aktivität, Nukleinsäuren kodierend eine Ferredoxin-Sulfit-Reduktase-Aktivität, Nukleinsäuren kodierend eine Ferredoxin NADPH-Reduktase Aktivität, Nukleinsäuren kodierend eine Ferredoxin-Aktivität, Nukleinsäuren kodierend ein Protein der Sulfat-Reduktion RXA077, Nukleinsäuren kodierend ein Protein der Sulfat-Reduktion RXA248, Nukleinsäuren kodierend ein Protein der Sulfat-Reduktion RXA247, Nukleinsäuren kodierend eine, RXA0655 Regulator undNukleinsäuren kodierend einen RXN2910 Regulator.

Wie vorstehend bei den Verfahren beschrieben, wird die Regulation der Expression dieser Genen im Mikroorganismus durch erfindungsgemäße Expressionseinheiten oder durch erfindungsgemäße Expressionseinheiten mit erhöhter spezifischer Expressionsaktivität gemäß Ausführungsform ch) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer dieser endogenen Gene unter der Kontrolle der eingebrachten, erfindungsgemäßen Expressionseinheiten, gegebenenfalls mit erhöhter spezifischer Expressionsaktivität, erfolgt oder
dh2) ein oder mehrere dieser Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Methionin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Homoserin Dehydrogenase-Aktivität, Glycerinaldehyd-3-Phosphat Dehydrogenase-Aktivität, 3-Phosphoglycerat Kinase-Aktivität, Pyruvat Carboxylase-Aktivität, Triosephosphat Isomerase-Aktivität, Homoserin O-Acetyltransferase-Aktivität, Cystahionin-gamma-Synthase-Aktivität, Cystahionin-beta-Lyase-Aktivität Serin-Hydroxymethyltransferase-Aktivität, O-Acetylhomoserin-Sulfhydrylase-Aktivität, Methylen-Tetrahydrofolat-Reduktase-Aktivität, Phosphoserin-Aminotransferase-Aktivität, Phosphoserin-Phosphatase-Aktivität, Serine AcetylTransferase-Aktivität, Cystein-Synthase-Aktivität, Cystein-Synthase II -Aktivität, Coenzym B12-abhängige Methionin-Synthase-Aktivität, Coenzym B12-unabhängige Methionin-Synthase-Aktivität, Sulfat-Adenylyltransferase-Aktivität, Phosphoadenosin-Phosphosulfat-Reductase-Aktivität, Ferredoxin-Sulfit-Reduktase-Aktivität, Ferredoxin NADPH-Reduktase Aktivität, Ferredoxin-Aktivität Aktivität Proteins der Sulfat-Reduktion RXA077, Aktivität eines Proteins der Sulfat-Reduktion RXA248, Aktivität eines Proteins der Sulfat-Reduktion RXA247, Aktivität eines RXA655-Regulators und Aktivität eines RXN2910-Regulators aufweisen

Eine weiter besonders bevorzugte Ausführungsform des vorstehend beschriebenen Verfahrens zur Herstellung von Methionin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Homoserine-Kinase-Aktivität, Threonin-Dehydratase-Aktivität, Threonin Synthase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthase-Aktivität, Dihydrodipicolinat Reduktase-Aktivität, und Diaminopicolinat Decarboxylase-Aktivität aufweisen.

Diese zusätzlichen, erhöhten oder reduzierten Aktivitäten mindestens einer der vorstehend beschriebenen Aktivitäten können, müssen aber nicht durch eine erfindungsgemäße Nukleinsäure mit Promotoraktivität und/oder eine erfindungsgemäße Expressionseinheit verursacht sein.

Die Erfindung betrifft ferner ein Verfahren zur Herstellung von Threonin durch Kultivierung von genetisch veränderten Mikroorganismen mit erhöhter oder verursachter Expressionsrate mindestens eines Gens im Vergleich zum Wildtyp, wobei man
ch) die spezifische Expressionsaktivität im Mikroorganismus von mindestens einer endogenen, erfindungsgemäßen Expressionseinheit, die die Expression der endogenen Gene reguliert, im Vergleich zum Wildtyp erhöht oder
dh) die Expression von Genen im Mikroorganismus durch erfindungsgemäße Expressionseinheiten oder durch erfindungsgemäße Expressionseinheiten mit erhöhter spezifischer Expressionsaktivität gemäß Ausführungsform ch) reguliert, wobei die Gene im Bezug auf die Expressionseinheiten heterolog sind,
und wobei die Gene ausgewählt sind aus der Gruppe Nukleinsäuren kodierend eine Aspartatkinase, Nukleinsäuren kodierend eine Aspartat-Semialdehyd-Dehydrogenase, Nukleinsäuren kodierend eine Glycerinaldehyd-3-Phosphat Dehydrogenase, Nukleinsäuren kodierend eine 3-Phosphoglycerat Kinase, Nukleinsäuren kodierend eine Pyruvat Carboxylase, Nukleinsäuren kodierend eine Triosephosphat Isomerase, Nukleinsäuren kodierend eine Homoserin-Kinase, Nukleinsäuren kodierend eine Threonin Synthase, Nukleinsäuren kodierend einen Threonin Exporter Carrier, Nukleinsäuren kodierend eine Glucose-6-Phosphat-Dehydrogenase, Nukleinsäuren kodierend eine Transaldolase, Nukleinsäuren kodierend eine Transketolase, Nukleinsäuren kodierend einer Malat-Quinon-Oxidoreductase, Nukleinsäuren kodierend eine 6-Phosphogluconat-Dehydrogenase, Nukleinsäuren kodierend einen Lysin-Exporter, Nukleinsäuren kodierend eine Biotin-Ligase, Nukleinsäuren kodierend eine Phosphoenolpyruvat-Carboxylase, Nukleinsäuren kodierend ein Threonin Efflux-Protein, Nukleinsäuren kodierend eine Fruktose-1,6-bisphosphatase, Nukleinsäuren kodierend ein OpcA Protein, Nukleinsäuren kodierend eine 1-Phosphofructokinase, Nukleinsäuren kodierend eine 6-Phosphofructokinase, und Nukleinsäuren kodierend eine Homoserin-Dehydrogenase

Wie vorstehend bei den Verfahren beschrieben, wird die Regulation der Expression dieser Genen im Mikroorganismus durch erfindungsgemäße Expressionseinheiten oder durch erfindungsgemäße Expressionseinheiten mit erhöhter spezifischer Expressionsaktivität gemäß Ausführungsform ch) dadurch erreicht, dass man
dh1) eine oder mehrere erfindungsgemäße Expressionseinheiten, gegebenenfalls mit erhöhter spezifischer Expressionsaktivität, in das Genom des Mikroorganismus einbringt, so dass die Expression eines oder mehrerer dieser endogenen Gene unter der Kontrolle der eingebrachten, erfindungsgemäßen Expressionseinheiten, gegebenenfalls mit erhöhter spezifischer Expressionsaktivität, erfolgt oder
dh2) ein oder mehrere dieser Gene in das Genom des Mikrorganismus einbringt, so dass die Expression eines oder mehrerer der eingebrachten Gene unter der Kontrolle der endogenen, erfindungsgemäßen Expressionseinheiten, gegebenenfalls mit erhöhter spezifischer Expressionsaktivität, erfolgt oder
dh3) ein oder mehrere Nukleinsäurekonstrukte, enthaltend eine erfindungsgemäße Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

Eine weiter bevorzugte Ausführungsform des vorstehend beschriebenen Verfahrens zur Herstellung von Threonin ist dadurch gekennzeichnet, dass die genetisch veränderten Mikroorganismen im Vergleich zum Wildtyp zusätzlich eine erhöhte Aktivität, mindestens einer der Aktivitäten, ausgewählt aus der Gruppe Aspartatkinase-Aktivität, Aspartat-Semialdehyd-Dehydrogenase-Aktivität, Glycerinal-dehyd-3-Phosphat Dehydrogenase-Aktivität, 3-Phosphoglycerat Kinase-Aktivität, Pyruvat Carboxylase-Aktivität, Triosephosphat Isomerase-Aktivität, Threonin Synthase-Aktivität, Aktivität eines Threonin Export-Carriers, Transaldolase-Aktivität, Transketolase-Aktivität, Glucose-6-Phosphat-dehydrogenase-Aktivität, Malat-Qinon-Oxidoreductase-Aktivität, Homoserin-Kinase-Aktivität, Biotin-Ligase-Aktivität, Phosphoenolpyruvat-Carboxylase-Aktivität, Threonin-Efflux-Protein-Aktivität, Protein OpcA-Aktivität, 1-Phosphofructokinase-Aktivität, 6-Phosphofructokinase-Aktivität, Fruktose 1,6 bisphosphatase-Aktivität, 6-Phosphogluconat-Dehydrogenase und Homoserin-Dehydrogenase-Aktivität aufweisen.

Eine weiter besonders bevorzugte Ausführungsform des vorstehend beschriebenen Verfahrens zur Herstellung von Threonin ist dadurch gekennzeichnet, dass die genetisch veränderten Mikroorganismen im Vergleich zum Wildtyp zusätzlich eine reduzierte Aktivität, mindestens einer der Aktivitäten, ausgewählt aus der Gruppe Threonin Dehydratase-Aktivität, Homoserin O-Acetyltransferase-Aktivität, Serin-Hydroxymethyltransferase-Aktivität, O-Acetylhomoserin-Sulfhydrylase-Aktivität, Meso-Diaminopimelat D-Dehydrogenase-Aktivität, Phosphoenolpyruvat-Carboxykinase-Aktivität, Pyruvat-Oxidase-Aktivität, Dihydrodipicolinat Synthetase-Aktivität, Dihydrodipicolinat Reduktase-Aktivität, Asparaginase-Aktivität, Aspartat-Decarboxylase-Aktivität, Lysin-Exporter-Aktivität, Acetolactat-Synthase-Aktivität , Ketol-Aid-Reductoisomerase-Aktivität , Branched chain aminotransferase- Aktivität , Coenzym B12-abhängige Methionin Synthase- Aktivität, Coenzym B12-unabhängige Methion Synthase- Aktivität, Dihydroxy-acid Dehydratase- Aktivität und Diaminopicolinat Decarboxylase-Aktivität aufweisen.

Diese zusätzlichen, erhöhten oder reduzierten Aktivitäten mindestens einer der vorstehend beschriebenen Aktivitäten können, müssen aber nicht durch eine erfindungsgemäße Nukleinsäure mit Promotoraktivität und/oder eine erfindungsgemäße Expressionseinheit verursacht sein.

Unter dem Begriff der "Aktivität" eines Proteins wird bei Enzymen die Enzymaktivität des entsprechenden Proteins, bei anderen Proteinen, beispielsweise Struktur oder Transport-Proteinen die physiologische Aktivität der Proteine verstanden.

Die Enzyme sind in der Regel in der Lage ein Substrat in ein Produkt umzuwandeln bzw. diesen Umwandlunsgschritt zu katalysieren.

Dementsprechend wird unter der "Aktivität" eines Enzyms die in einer bestimmten Zeit durch das Enzym umgesetzte Menge Substrat bzw. gebildete Menge Produkt verstanden.

Bei einer erhöhten Aktivität im Vergleich zum Wildtyp wird somit im Vergleich zum Wildtyp in einer bestimmten Zeit durch das Enzym die umgesetzte Menge Substrat bzw. die gebildete Menge Produkt erhöht.

Vorzugsweise beträgt diese Erhöhung der "Aktivität" bei allen vorstehend und nachstehend beschriebenen Aktivitäten mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der "Aktivität des Wildtyps".

Bei einer reduzierten Aktivität im Vergleich zum Wildtyp wird somit im Vergleich zum Wildtyp in einer bestimmten Zeit durch das Enzym die umgesetzte Menge Substrat bzw. die gebildete Menge Produkt reduziert.

Unter einer reduzierten Aktivität wird vorzugsweise die teilweise oder im wesentlichen vollständige, auf unterschiedliche zellbiologische Mechanismen beruhende Unterbindung oder Blockierung der Funktionalität diese Enzyms in einem Mikroorganismus verstanden.

Eine Reduzierung der Aktivität umfasst eine mengenmäßige Verringerung eines Enzyms bis hin zu einem im wesentlichen vollständigen Fehlen des Enzyms (d.h. fehlende Nachweisbarkeit der entsprechenden Aktivität oder fehlende immunologische Nachweisbarkeit des Enzyms). Vorzugsweise wird die Aktivität im Mikroorganismus im Vergleich zum Wildtyp um mindestens 5 %, weiter bevorzugt um mindestens 20 %, weiter bevorzugt um mindestens 50 %, weiter bevorzugt um 100 % reduziert. Insbesondere meint "Reduzierung" auch das vollständigen Fehlen der entsprechenden Aktivität.

Die Aktivität bestimmter Enzyme in genetisch veränderten Mikroorganismen sowie im Wildtyp und damit die Erhöhung oder Reduzierung der Enzymaktivität lassen sich nach bekannten Verfahren, wie beispielsweise Enzymassays ermitteln.

Beispielsweise wird unter eine Pyruvatcarboxylase ein Protein verstanden, das
die enzymatische Aktivität aufweist, Pyruvat in Oxaloacetat umzuwandeln.

Dementsprechend wird unter einer Pyruvatcarboxylase-Aktivität die in einer bestimmten Zeit durch das Protein Pyruvatcarboxylase umgesetzte Menge Pyruvat bzw. gebildete Menge Oxaloacetat verstanden.

Bei einer erhöhten Pyruvatcarboxylase-Aktivität gegenüber dem Wildtyp wird somit im Vergleich zum Wildtyp in einer bestimmten Zeit durch das Protein Pyruvatcarboxylase die umgesetzte Menge Pyruvat bzw. die gebildete Menge Oxaloacetat erhöht.

Vorzugsweise beträgt diese Erhöhung der Pyruvatcarboxylase-Aktivität mindestens 5 %, weiter bevorzugt mindestens 20 %, weiter bevorzugt mindestens 50 %, weiter bevorzugt mindestens 100 %, bevorzugter mindestens 300 %, noch bevorzugter mindestens 500 %, insbesondere mindestens 600 % der Pyruvatcarboxylase -Aktivität des Wildtyps.

Weiterhin wir beispielsweise unter eine Phosphoenolpyruvat-Carboxykinase-Aktivität die Enzymaktivität einer Phosphoenolpyruvat-Carboxykinase-verstanden.

Unter einer Phosphoenolpyruvat-Carboxykinase wird ein Protein verstanden, das die enzymatische Aktivität aufweist, Oxaloacetat in Phosphoenolpyruvat umzuwandeln.

Dementsprechend wird unter Phosphoenolpyruvat-Carboxykinase-Aktivität die in einer bestimmten Zeit durch das Protein Phosphoenolpyruvat umgesetzte Menge Oxaloacetat bzw. gebildete Menge Phosphoenolpyruvat verstanden.

Bei einer reduzierten Phosphoenolpyruvat-Carboxykinase-Aktivität gegenüber dem Wildtyp wird somit im Vergleich zum Wildtyp in einer bestimmten Zeit durch das Protein Phosphoenolpyruvat-Carboxykinase die umgesetzte Menge Oxaloacetat bzw. die gebildete Menge Phosphoenolpyruvat reduziert.

Eine Reduzierung der Phosphoenolpyruvat-Carboxykinase-Aktivität umfasst eine mengenmäßige Verringerung einer Phosphoenolpyruvat-Carboxykinase bis hin zu einem im wesentlichen vollständigen Fehlen der Phosphoenolpyruvat-Carboxykinase (d.h. fehlende Nachweisbarkeit von Phosphoenolpyruvat-Carboxykinase-Aktivität oder fehlende immunologische Nachweisbarkeit der Phosphoenolpyruvat-Carboxykinase). Vorzugsweise wird die Phosphoenolpyruvat-Carboxykinase-Aktivität im Vergleich zum Wildtyp um mindestens 5 %, weiter bevorzugt um mindestens 20 %, weiter bevorzugt um mindestens 50 %, weiter bevorzugt um 100 % reduziert. Insbesondere meint "Reduzierung" auch das vollständigen Fehlen der Phosphoenolpyruvat-Carboxykinase -Aktivität.

Die zusätzliche Erhöhung von Aktivitäten kann durch verschiedene Wege erfolgen, beispielsweise durch Ausschalten von hemmenden Regulationsmechanismen auf Expressions- und Proteinebene oder durch Erhöhung der Genexpression von Nukleinsäuren kodierend die vorstehend beschriebenen Proteine gegenüber dem Wildtyp.

Die Erhöhung der Genexpression der Nukleinsäuren kodierend die vorstehend beschriebenen Proteine gegenüber dem Wildtyp kann ebenfalls durch verschiedene Wege erfolgen, beispielsweise durch Induzierung des Gens durch Aktivatoren oder wie vorstehend beschrieben durch Erhöhung der Promotoraktivität oder Erhöhung der Expressionsaktivität oder durch Einbringen von einer oder mehrerer Genkopien in den Mikroorganismus.

Unter Erhöhung der Genexpression einer Nukleinsäure codierend ein Protein wird erfindungsgemäß auch die Manipulation der Expression der Mikroorganismus eigenen, endogenen Proteine verstanden.

Dies kann beispielsweise, wie vorstehend beschrieben durch Veränderung der Promotor- und/oder Expressionseinheits-Sequenzen der Gene erreicht werden. Eine solche Veränderung, die eine erhöhte Expressionsrate des Gens zur Folge hat, kann beispielsweise durch Deletion oder Insertion von DNA Sequenzen erfolgen.

Es ist, wie vorstehend beschrieben, möglich, die Expression der endogenen Proteine durch die Applikation exogener Stimuli zu verändern. Dies kann durch besondere physiologische Bedingungen, also durch die Applikation von Fremdsubstanzen erfolgen.

Zur Erzielung einer Erhöhung der Genexpression kann der Fachmann weitere unterschiedliche Maßnahmen einzeln oder in Kombination ergreifen. So kann beispielsweise die Kopienzahl der entsprechenden Gene erhöht werden, oder es kann die Promotor- und Regulationsregion oder die Ribosomenbindungsstelle, die sich stromaufwärts des Strukturgens befindet, mutiert werden. Durch induzierbare Promotoren ist es zusätzlich möglich, die Expression im Verlaufe der fermentativen Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biontechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift 0472869, im US Patent 4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58,.191-195 (1998)), bei Makrides (Microbiological Reviews 60 : 512-538 (1996) und in bekannten Lehrbüchern der Genetik und Molekularbiologie.

Weiterhin kann es für die Produktion von biosynthetischen Produkten, insbesondere L-Lysin, L-Methionin und L-Threonin, vorteilhaft sein, neben der Expression bzw. Verstärkung eines Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

In einer bevorzugten Ausführungsform erfolgt die Erhöhung der Genexpression einer Nukleinsäure kodierend eines der vorstehend beschriebenen Proteine durch Einbringen von mindestens einer Nukleinsäure kodierend ein entsprechendes Protein in den Mikroorganismus. Das Einbringen der Nukleinsäure kann chromsomal oder extrachromosomal erfolgen, also durch Erhöhung der Kopienzahl auf dem Chromosom und oder eine Kopie des Gens auf einem sich replizierenden Plasmid in dem Wirtsmikroorganismus.

Vorzugsweise erfolgt das Einbringen der Nukleinsäure, beispielsweise in Form einer Expressionskassete, enthaltend die Nukleinsäure, chromosomal, insbesondere durch die vorstehend beschriebene SacB-Methode.

Dazu kann prinzipiell jedes Gen, das eines der vorstehend beschriebenen Proteine kodiert verwendet werden.

Bei genomischen Nukleinsäure-Sequenzen aus eukaryontischen Quellen, die Introns enthalten, sind für den Fall das der Wirtsmikroorganismus nicht in der Lage ist oder nicht in die Lage versetzt werden kann, die entsprechenden Proteine zu exprimieren, bevorzugt bereits prozessierte Nukleinsäuresequenzen, wie die entsprechenden cDNAs zu verwenden.

Beispiele für die entsprechenden Gene sind in Tabelle 1 und 2 aufgelistet.

Vorzugsweise erfolgt die Reduzierung der vorstehend beschriebenen Aktivitäten in Mikroorganismen durch mindestens eines der nachfolgenden Verfahren:
- Einbringen mindestens einer sense-Ribonukleinsäuresequenz zur Induktion einer Kosuppression oder einer deren Expression gewährleistenden Expressionskassette
- Einbringen mindestens eines DNA- oder Protein-bindenden Faktors gegen das entsprechede -Gen, -RNA oder-Protein oder einer dessen Expression gewährleistenden Expressionskassette
- Einbringen mindestens einer den RNA-Abbau bewirkenden viralen Nukleinsäuresequenz oder einer deren Expression gewährleistenden Expressionskassette
- Einbringen mindestens eines Konstruktes zur Erzeugung eines Funktionsverlustes, wie beispielsweise die Generierung von Stopp-Kodons oder eine Verschiebungen im Leseraster, an einem Gen beispielsweise durch Erzeugung einer Insertion, Deletion, Inversion oder Mutation in einem Gen. Bevorzugt können Knockout-Mutanten mittels gezielter Insertion in das gewünschte Zielgen durch homologe Rekombination oder Einbringen von sequenzspezifischen Nukleasen gegen das Zielgen generiert werden.
- Einbringen eines Promotors mit reduzierter Promotoraktivität oder einer Expressionseinheit mit reduzierter Expressionsaktivität.

Dem Fachmann ist bekannt, dass auch weitere Verfahren im Rahmen der vorliegenden Erfindung zur Reduzierung seiner Aktivität oder Funktion eingesetzt werden können. Beispielsweise kann auch das Einbringen einer dominant-negativen Variante eines Proteins oder einer deren Expression gewährleistenden Expressionskassette vorteilhaft sein.

Dabei kann jedes einzelne dieser Verfahren eine Verminderung der Proteinmenge, mRNA-Menge und/oder Aktivität eines Proteins bewirken. Auch eine kombinierte Anwendung ist denkbar. Weitere Methoden sind dem Fachmann bekannt und können die Behinderung oder Unterbindung der Prozessierung des Proteins, des Transports des Proteins oder dessen mRNA, Hemmung der Ribosomenanlagerung, Hemmung des RNA-Spleißens, Induktion eines RNA abbauenden Enzyms und/oder Hemmung der Translationselongation oder -termination umfassen.

Im erfindungsgemäßen Verfahren zur Herstellung von biosynthetischen Produkten wird vorzugsweise dem Kultivierungsschritt der genetisch veränderten Mikroorganismen ein Isolieren von biosynthetischen Produkten aus den Mikroorganismen oder/oder aus der Fermentationsbrühe angeschlossen. Diese Schritte können gleichzeitig und/oder vorzugsweisie nach dem Kultivierungsschritt stattfinden.

Die erfindungsgemäßen genetisch veränderten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) zur Produktion von biosynthetischen Produkten, insbesondere L-Lysin, L-Methionin und L-Threonin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen

Als Schwefelquelle für die Herstellung von Feinchemikalien, insbesondere von Methionin, können anorganische Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Temperatur der Kultur liegt normalerweise bei 20°C bis 45°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf 0 bis 3 g/l gehalten, beziehungsweise abgesenkt wird.

Die Isolierung von biosynthetischen Produkten aus der Fermentationsbrühe und/oder den Mikroorganismen erfolgt in an sich bekannter Weise entsprechend den physikalisch-chemischen Eigenschaften des biosynthetischen Wertprodukts und den biosynthetischen Nebenprodukten.

Die Fermentationsbrühe kann anschließend beispielsweise weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich die biosynthetischen Produkte, insbesonder L-Lysin, L-Methionin und L-Threonin, weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die biosynthetischen Produkte können in unterschiedlichen Formen anfallen, beispielsweise in Form ihrer Salze oder Ester.

Die Identität und Reinheit der isolierten Verbindung(en) kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele näher beschrieben:

### Beispiel 1

Herstellung eines Integrationsplasmids zur Überexpression des pycA-Gens mit Hilfe der heterologen Expressionseinheit Pgro (SEQ ID 2)

Zur Amplifizierung des Promoters des Gens, das für das Chaperenin Gro ES kodiert, wurden die folgenden Oligonukleotide definiert.
SEQ. ID. NO 5:
   gro3: 5'- gccgcagcaaacccagtag -3'
SEQ. ID. NO: 6:
   gro11: 5'- agtcgacacgatgaatccctccatgagaaaa-3'

Die Primer wurden in eine PCR-Reaktion mit chromosomaler DNA von C. glutamicum ATCC13032 eingesetzt. Mit diesem Ansatz konnte ein DNA-Fragment amplifiziert werden, das der erwarteten Größe von 427 bp entsprach.

Zur Amplifizierung eines Teils des Gens, das für das Pyruvat-Caroboxylase kodiert, wurden die folgenden Oligonukleotide definiert.
SEQ. ID. NO. 7:
   pyc6: 5'-tttttctcatggagggattcatcgtgtcgactcacacatcttcaacgcttccag-3'
SEQ. ID. NO. 8:
   pyc3: 5'- cccgcagcaacgcacgcaagaaa-3'

Die Primer wurden in eine PCR-Reaktion mit chromosomaler DNA von C. glutamicum ATCC13032 eingesetzt. Mit diesem Ansatz konnte ein DNA-Fragment amplifiziert werden, das der erwarteten Größe von 1344 bp entsprach.

Die Primer gro11 und pyc6 enthalten eine überlappende Sequenz und sind an ihren 5'-Enden homolog zueinander.
Die oben erhaltenen PCR-Produkte wurden als Template für eine weitere PCR einsetzt, in der die folgenden Primer genutzt wurden.
SEQ. ID. NO. 9:
   gro12: 5'- gcattcgcgccgctcgtaacta-3'
SEQ. ID. NO. 10:
   pyc11: 5'- ggttcccgcgccctggtaa-3'

Mit diesem Ansatz konnte ein DNA-Fragment amplifiziert werden, das der erwarteten Größe von 1107 bp entsprach. Diese Pgro/pycA-Fusion wurde dann in den Vektor pCR2.1 (Firma Invitrogen GmbH, Karlsruhe, Deutschland) kloniert. In einem weiteren Schritt wurde die Fusion Pgro/pycA aus dem Plasmid pCR2.1 (Firma Invitrogen GmbH, Karlsruhe, Deutschland) als 1125 bp EcoRI-Fragment in den Integrationsvektor pK19 mob sacB SEQ ID NO 11 kloniert, der zuvor mit der Restriktionsendonuklease EcoRI geschnitten worden war. Das resultierende Plasmid wurde mit pk19 mob sacB Pgro pycA bezeichnet.
Zur Amplifikation eines 5'-Bereiches des pycA-Gens wurden folgende Oligonukleotide definiert:
SEQ. ID. NO. 12:
   pyc14: 5'- ccggcgaagtgtctgctcgcgtga-3'
SEQ. ID. NO. 13:
   pyc15: 5'-accccgccccagtttttc-3'

Die Primer wurden in eine PCR-Reaktion mit chromosomaler DNA von C. glutamicum ATCC13032 eingesetzt. Mit diesem Ansatz konnte ein DNA-Fragment amplifiziert werden, das der erwarteten Größe von 487 bp entsprach. Dieses DNA-Fragment wurde in den Vektor pCR2.1 (Firma Invitrogen GmbH, Karlsruhe, Deutschland) kloniert. Ein 593 bp Spel/Xbal-Fragment wurde dann anschließend in den Vektor pK19 mob sacB Psod ask kloniert, der zuvor mit dem Restriktionsenzym Nhel verdaut worden war. Das resultierende Plasmid wurde mit pK19 mob sacB Pgro pycA + US (SEQ. ID. NO. 14) bezeichnet. Bis zu diesem Schritt wurden alle Klonierungen in Escherichia coli XL-1 Blue (Firma Stratagene, Amsterdam, Nierderlande) durchgeführt.

Mit dem Transformationsplasmid pK19 mobsacB Pgro pycA + US wurde dann E. coli Mn522 (Firma Stratagene, Amsterdam, Nierderlande) zusammen mit dem Plasmid pTc15AcgIM nach Liebl, et al. (1989) FEMS Microbiology Letters 53:299-303 transformiert. Das Plasmid pTc15AcgIM ermöglicht die Methylierung von DNA nach dem Methylierungsmuster von Corynebacterium glutamicum (DE 10046870). Durch diesen Schritt wird eine anschließende Elektroporation von Corynebacterium glutamicum mit dem Integrationsplasmid pK19 mob sacB Pgro pycA + US ermöglicht. Aus dieser Elektroporation und der nachfolgenden Selektion auf CM-Platten (10 g/l Glucose; 2,5 g/l NaCl; 2 g/l Harnstoff, 10 g/l Bacto Peptone (Fa. Difco); 10 g/l Hefeextrakt, 22,0 g/L Agar (Difco)) mit Kanamycin (25 µg/ml) wurden mehrere Transkonjuganten erhalten.

Zur Selektion auf das zweite Rekombinationsereignis, das zur Excision des Vectors samt dem pycA-Promoter und dem pycA-Gen führen soll, wurden diese Transkonjuganten in CM-Medium über Nacht ohne Kanamycin angezogen und anschließend zur Selektion auf CM-Platten mit 10% Saccharose ausplattiert. Das auf dem Vektor pK19 mob sacB vorhandenen sacB-Gen kodiert für das Enzym Laevansucrase und führt bei Wachstum auf Saccharose zur Synthese von Laevan. Da Laevan für C. glutamicum toxisch ist, können nur C. glutamicum Zellen, die das Integrationsplasmid durch den zweiten Rekombinationsschritt verloren haben, auf Saccharose-haltigem Medium wachsen (Jäger et al., Journal of Bacteriology 174 (1992) 5462-5466). 100 Saccharose-resistente Klone wurden auf ihre Kanamycin-Sensitivität hin überprüft. Für 15 der getesteten Klone konnte neben der Resistenz gegenüber Saccharose auch eine Sensitivität gegenüber Kanamycin nachgewiesen werden. Ob auch der gewünschte Austausch der natürlichen Expressionseinheit durch die Pgro-Expressionseinheit erfolgt war, wurde mittels Polymerase-Kettenreaktion (PCR) überprüft. Für diese Analyse wurde chromosomale DNA aus dem Ausgangsstamm und den 15 Klonen isoliert. Hierzu wurden die jeweiligen Klone mit einem Zahnstocher von der Agarplatte abgenommen und in 100 µl H₂O suspendiert und 10 min bei 95°C aufgekocht. Jeweils 10 µl der erhaltenen Lösung wurden als Template in die PCR eingesetzt. Als Primer wurden Oligonukleotide verwendet, die zur Pgro-Expressionseinheit und dem pycA-Gen homolog sind.

Die PCR-Bedingungen wurden wie folgt gewählt: Vorabdenaturierung:5 min bei 95°C; Denaturierung 30 sec bei 95°C; Hybridisierung 30 sec bei 56°C; Amplifizierung 1 min bei 72°C; 30 Zyklen,; End-Extension 5 min bei 72°C. Im Ansatz mit der DNA des Ausgangsstammes konnte durch Wahl der Oligonukleotide kein PCR-Produkt entstehen. Nur bei Klonen, die durch die 2. Rekombination den Austausch der natürlichen Expressionseinheit (PpycA) gegen Pgro vollzogen haben, wurde ein Bande mit einer Größe von 310 bp erwartet. Insgesamt waren von den getesteten 15 Klonen 7 Klone positiv.

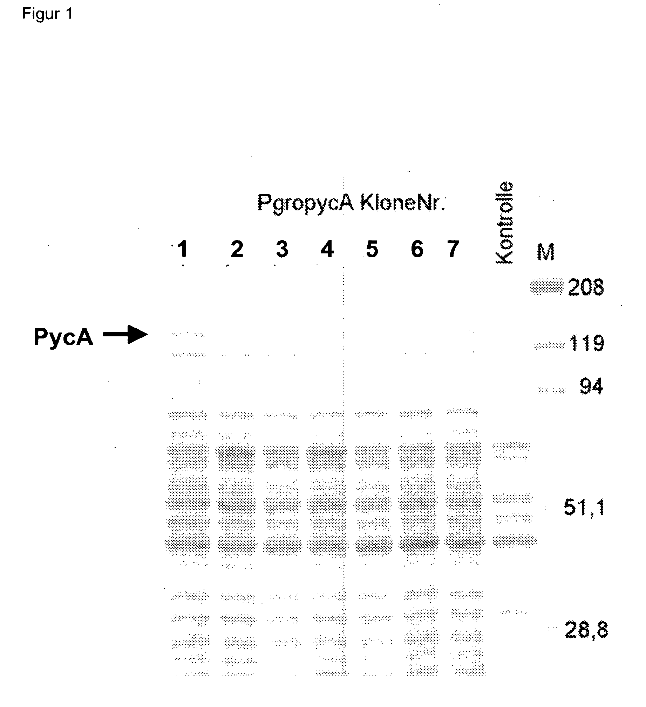

Die 7 positiven Klone und der Ausgangsstamm wurden anschließend in 10 ml CM-Medium (10 g/l Glucose; 2,5 g/l NaCl; 2 g/l Harnstoff, 10 g/l Bacto Peptone (Fa. Difco); 10 g/l Hefeextrakt) über Nacht angezogen. Anschließend wurde die Zellen pelletiert und in 0,5 ml Puffer (50 mM Tris, 10 mM MgCl₂, 50 mM KCl; pH7,7)) aufgenommen. Der Aufschluß der Zellen erfolgte mit Hilfe eines Ribolyzers (3 x 30 sec bei Stufe 6, Fa. Hybaid). Nach einer Proteinbestimmung mittels der Bradfordmethode wurden jeweils 15 µg Protein auf ein 10 %iges SDS-Gel aufgetragen und die Proteine aufgetrennt. Im Vergleich zum Ausgangsstamm konnte eine erhöhte Menge an PycA-Protein detektiert werden (Figur 1). Figur 1 zeigt ein 10%iges SDS-Gel der Pgro pycA-Klone.

### Beispiel 2

### Herstellung des Vektors pCLiK5MCS

Zunächst wurden Ampicillinresistenz und Replikationsursprung des Vektors pBR322 mit den Oligonukleotidprimern SEQ ID NO:15 und SEQ ID NO: 16 mit Hilfe der Polymerase-Kettenreaktion (PCR) amplifiziert.
SEQ ID NO:15
SEQ ID NO:16

Neben den zu pBR322 komplementären Sequenzen, enthält der Oligonukleotidprimer SEQ ID NO:15 in 5'-3' Richtung die Schnittstellen für die Restriktionsendonukleasen SmaI, BamHI, NheI und AscI und der Oligonukleotidprimer SEQ ID NO:16 in 5'-3' Richtung die Schnittstellen für die Restriktionsendonukleasen Xbal, Xhol, Notl und Dral. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,1 kb wurde mit dem GFX™PCR, DNA and Gel Band Purification Kit (Amersham Pharmacia, Freiburg) nach Angaben des Herstellers gereinigt. Die stumpfen Enden des DNA-Fragmentes wurden mit dem Rapid DNA Ligation Kit (Roche Diagnostics, Mannheim) nach Angaben des Herstellers miteinander ligiert und der Ligationsansatz nach Standardmethoden wie in Sambrook et al. (Molecular Cloning. A Laboratory Manual, Cold Spring Harbor, beschrieben (1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK1.

Ausgehend vom Plasmid pWLT1 (Liebl et al., 1992) als Template für eine PCR Reaktion wurde mit den Oligonukleotidprimern SEQ ID NO:17 und SEQ ID NO:18 eine Kanamycin-Resistenzcassette amplifiziert.
SEQ ID NO:17
   5'-GAGATCTAGACCCGGGGATCCGCTAGCGGGCTGCTAAAGGAAGCGGA-3'
SEQ ID NO:18:
   5'-GAGAGGCGCGCCGCTAGCGTGGGCGAAGAACTCCAGCA-3'

Neben den zu pWLT1 komplementären Sequenzen, enthält der Oligonukleotidprimer SEQ ID NO:17 in 5'-3' Richtung die Schnittstellen für die Restriktionsendonukleasen Xbal, Smal, BamHl, Nhel und der Oligonukleotidprimer SEQ ID NO:18 in 5'-3' Richtung die Schnittstellen für die Restriktionsendonukleasen Ascl und Nhel. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,3 kb wurde mit dem GFX™PCR, DNA and Gel Band Purification Kit (Amersham Pharmacia, Freiburg) nach Angaben des Herstellers gereinigt. Das DNA-Fragment wurde mit den Restriktionsendonukleasen Xbal und Ascl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK1 wurde ebenfalls mit den Restriktionsendonukleasen Xbal und Ascl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,1kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) und Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK2.

Der Vektor pCLiK2 wurde mit der Restriktionsendonuklease Dral (New England Biolabs, Beverly, USA) geschnitten. Nach Elektrophorese in einem 0,8%igen Agarosegel wurde ein ca. 2,3 kb großes Vektorfragment mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers religiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK3.

Ausgehend vom Plasmid pWLQ2 (Liebl et al., 1992) als Template für eine PCR Reaktion wurde mit den Oligonukleotidprimern SEQ ID NO:19 und SEQ ID NO: 20 der Replikationsursprung pHM1519 amplifiziert.
SEQ ID NO: 19
   5'-GAGAGGGCGGCCGCGCAAAGTCCCGCTTCGTGAA-3'
SEQ ID NO: 20:
   5'-GAGAGGGCGGCCGCTCAAGTCGGTCAAGCCACGC-3'

Neben den zu pWLQ2 komplementären Sequenzen, enthalten die Oligonukleotidprimer SEQ ID NO: 19 und SEQ ID NO: 20 Schnittstellen für die Restriktionsendonuklease Notl. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,7 kb wurde mit dem GFX™PCR, DNA and Gel Band Purification Kit (Amersham Pharmacia, Freiburg) nach Angaben des Herstellers gereinigt. Das DNA-Fragment wurde mit der Restriktionsendonuklease Notl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK3 wurde ebenfalls mit der Restriktionsendonuklease Notl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,3kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5.

Für die Erweiterung von pCLiK5 um eine "multiple cloning site" (MCS) wurden die beide synthetischen, weitestgehend komplementären Oligonukleotide SEQ ID NO: 21 und SEQ ID NO: 22, die Schnittstellen für die Restriktionsendonukleasen SwaI, XhoI, AatI, ApaI, Asp718, MluI, NdeI, SpeI, EcoRV, SalI, ClaI, BamHI, XbaI und SmaI enthalten, durch gemeinsames erhitzen auf 95°C und langsames abkühlen zu einem doppelsträngigen DNA-Fragment vereinigt.
SEQ ID NO: 21:
SEQ ID NO22:

Der Vektor pCLiK5 wurde mit den Restriktionsendonuklease Xhol und BamHl (New England Biolabs, Beverly, USA) geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 5,0 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem synthetischen Doppelsträngigen DNA-Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS.

Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS ist als SEQ ID NO: 23 aufgeführt.

### Beispiel 3

### Herstellung des Plasmids PmetA metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 24 und SEQ ID NO 25, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, ein das metA Gen inklusice des nichkodierenden 5'-Bereichs amplifiziert.
SEQ ID NO 24
   5`-GCGCGGTACCTAGACTCACCCCAGTGCT -3'
   und
SEQ ID NO 25
   5`-CTCTACTAGTTTAGATGTAGAACTCGATGT -3'

Das erhaltene DNA Fragment von ca. 1,3 kb Größe wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Asp718 und Spel (Roche Diagnostics, Mannheim) gespalten und das DNA Fragment mit GFX™PCR, DNA and Gel Band Purification Kit aufgereinigt.

Der Vektor pClik5MCS SEQ ID NO: 23 wurde mit den Restriktionsenzymen Asp718 und SpeI geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PmetA metA ist als SEQ ID NO 26: aufgeführt.

### Beispiel 9

### Herstellung des Plasmid pCLiK5MCS Pgro metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 27 und SEQ ID NO 28, der chromosomalen DNA als Template und Pfu Turbo Polymerase (Fa. Stratagene) wurde mit Hilfe der Polymerase-Kettenreaktion (PCR) nach Standardmethoden wie Innis et al. (1990) PCR Protocols. A Guide to Methods and Applications, Academic Press ein DNA Fragment von ca. 200 Basenpaaren aus dem nichtkodierenden 5'-Bereich (Region der Expressionseinheit) der Gens GroES (Pgro) amplifiziert.
SEQ ID NO: 27
   5`-GAGACTCGAGCGGCTTAAAGTTTGGCTGCC -3'
   und
SEQ ID NO :28
   5`-CCTGAAGGCGCGAGGGTGGGCATGATGAATCCCTCCATGAG -3'

Das erhaltene DNA Fragment wurde mit dem GFX™PCR, DNA and Gel Band Purification Kit (Amersham Pharmacia, Freiburg) nach Angaben des Herstellers gereinigt.

Ausgehend vom Plasmid PmetA metA als Template für eine PCR Reaktion wurde mit den Oligonukleotidprimern SEQ ID NO:29 und SEQ ID NO: 30 ein Teil von metA amplifiziert.
SEQ ID NO 29
   5'-CCCACCCTCGCGCCTTCAG -3'
   und
SEQ ID NO 30
   5`-CTGGGTACATTGCGGCCC -3'

Das erhaltene DNA Fragment von ungefähr 470 Basenpaaren wurde mit dem GFX™PCR, DNA and Gel Band Purification Kit nach Angaben des Herstellers gereinigt.

In einer weiteren PCR Reaktion wurden die beiden oben erhaltenen Fragmente gemeinsam als Template eingesetzt. Durch die mit dem Oligonukleotidprimer SEQ ID NO: 28 eingebrachten, zu metA homologen Sequenzen, kommt es im Zuge der PCR-Reaktion zu einer Anlagerung beider Fragmente aneinander und einer Verlängerung zu einem durchgehenden DNA-Strang durch die eingesetzte Polymerase. Die Standardmethode wurde dahingehend modifiziert, dass die verwendeten Oligonukleotidprimer SEQ ID NO: 27 und SEQ ID NO: 30 erst mit Beginn des 2. Zykluses dem Reaktionsansatz zugegeben wurden.

Das amplifizierte DNA Fragment von ungefähr 675 Basenpaaren wurde mit dem GFX™PCR, DNA and Gel Band Purification Kit nach Angaben des Herstellers gereinigt. Im Anschluß daran wurde es mit den Restriktionsenzymen XhoI und Ncol (Roche Diagnostics, Mannheim) gespalten und gelelektrophoretisch aufgetrennt. Anschließend wurde das ca. 620 Basenpaar große DNA Fragment mit GFX™PCR, DNA and Gel Band Purification Kit (Amersham Pharmacia, Freiburg) aus der Agarose aufgereinigt. Das Plasmid PmetA metA SEQ ID NO: 26 wurde mit den Restriktionsenzymen Ncol und Spel (Roche Diagnostics, Mannheim) gespalten. Nach gelelektrophoretischer Auftrennung wurde ein ca. 0,7 kb großes metA Fragment mit GFX™PCR, DNA and Gel Band Purification Kit aus der Agarose aufgereinigt.

Der Vektor pClik5MCS SEQ ID NO: 23 wurde mit den Restriktionsenzymen Xhol und Spel (Roche Diagnostics, Mannheim) geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment und dem metA-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS PGroESmetA ist als SEQ ID NO: 31 aufgeführt.

### Beispiel 10

### MetA-Aktivitäten

Der Stamm Corynebacterium glutamicum ATCC13032 wurde jeweils mit den Plasmiden pClik5 MCS, pClik MCS PmetA metA und pCLiK5MCS PGroESmetA nach der beschriebenen Methode (Liebl, et al. (1989) FEMS Microbiology Letters 53:299-303) transformiert. Die Transformationsmischung wurde auf CM-Platten plattiert, die zusätzlich 20mg/l Kanamycin enthielten, um eine Selektion auf Plasmid-haltige Zellen zu erreichen. Erhaltene Kan-resistente Klone wurden gepickt und vereinzelt.

C. glutamicum Stämme, die eines dieser Plasmidkonstrukte enthielten, wurden in MMA-Medium ((40 g/l Saccharose, 20 g/l (NHa)₂SO₄, 1 g/l KH₂PO₄, 1 g/l K₂HPO₄, 0,25g/l MgSO₄ x 7H₂O, 54 g Aces, 1 ml CaCl2 (10 g/l), 1 ml Protocatechoat (300 mg/10 ml), 1 ml Spurenelementelösung (10 g/l FeSO₄ x /H₂O, 10 g/l MnSO₄ x H₂O, 2 g/l ZnSO₄ x 7 H₂O, 0,2 g/l CuSO₄, 0,02 g/l NiCl₂ x 6 H₂O),100 µg/l Vitamin B₁₂, 0,3 mg/l Thiamin, 1 mM Leucin, 1 mg/l Pyridoxal HCl, 1 ml Biotin (100 mg/l), pH7,0) bei 30°C über Nacht angezogen. Die Zellen wurden bei 4°C abzentrifugiert und das zweimal mit kaltem Tris-HCl-Puffer (0,1 %, pH 8,0) gewaschen. Nach erneuter Zentrifugation wurden die Zellen in kalten Tris-HCl-Puffer (0,1%, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die enzymatische Aktivität von MetA wurde wie folgt durchgeführt. Die Reaktionsansätze von 1 ml enthielten 100 mM Kaliumphosphatpuffer (pH 7,5), 5mM MgCl2, 100 µM Acetyl CoA, 5mM L-Homoserine, 500 µM DTNB (Ellmans Reagenz) und Zellextrakt. Der Test wurde durch Zugabe von dem jeweiligen Proteinlysat gestartet und bei Raumtemperatur inkubiert. Es wurde dann eine Kinetig bei 412 nm über 10 min aufgenommen.

Die Ergebnisse sind in Tabelle 1 a gezeigt.

**Tabelle 1 a**

| Stamm | spezifische Aktivität [nMol/mg/min] |
|---|---|
| ATCC 13032 pClik5MCS | 12,6 |
| ATCC 13032 pClik5MCS PmetA metA | 50,7 |
| ATCC 13032 pCLiK5MCS PGroESmetA | 109,0 |

Die Aktivität von MetA konnte durch die Verwendung der heterologen Expressionseinheit erheblich gesteigert werden.

### Beispiel 11

### Herstellung des Plasmids pClik5MCS metA ohne Startcodon

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimern SEQ ID NO 32 bis SEQ ID NO 33, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, die terminationsregion des groEL Gens amplifiziert.
SEQ ID NO 32
   5'- GGATCTAGAGTTCTGTGAAAAACACCGTG-3'
SEQ ID NO 33
   5'- GCGACTAGTGCCCCACAAATAAAAAACAC-3'

Die erhaltenen DNA Fragmente von ca. 60 bp Größe wurden mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Xbal und Bcnl (Roche Diagnostics, Mannheim) gespalten und das DNA Fragment mit GFX™PCR, DNA and Gel Band Purification Kit aufgereinigt.

Der Vektor pClik5MCS SEQ ID NO: 23 wurde mit dem Restriktionsenzymen Xbal geschnitten und ein 5 kb großes Fragment nach elektrophoretischer Auftrennung mit GFX™PCR, DNA and Gel Band Purification Kit isoliert.

Das Vektorfragment wurde zusammen mit dem 60 bp großen Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandenen Plasmid pCLiK5MCS PgroES term SEQ ID NO: 34 aufgeführt.

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer SEQ ID NO 35 und SEQ ID NO 36, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, das metA Gen ohne Sartcodon amplifiziert.
SEQ ID NO 35
   5'-GAGACATATGCCCACCCTCGCGCCTTCAGG -3'
   und
SEQ ID NO 36
   5'-CTCTACTAGTTTAGATGTAGAACTCGATGT -3'

Das erhaltene DNA Fragment von ca. 1,2 kb Größe wurde mit dem GFX™PCR, DNA and Gel Band Purification Kit (Amersham Pharmacia, Freiburg) nach Angaben des Herstellers gereinigt. Im Anschluß daran wurde es mit den Restriktionsenzymen Ndel und Bcnl (Roche Diagnostics, Mannheim) gespalten und das DNA Fragment mit GFX™PCR, DNA and Gel Band Purification Kit aufgereinigt.

Der Vektor pClik5MCS groEL term SEQ ID NO: 34 wurde mit den Restriktionsenzymen Ndel und Bcnl geschnitten und ein 5 kb großes Fragment nach elektrophoretischer Auftrennung mit GFX™PCR, DNA and Gel Band Purification Kit isoliert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS metA ohne Startcodon ist als SEQ ID NO: 37 aufgeführt.

### Beispiel 12

Konstruktion von Expressionseinheiten Pgro mit unterschiedlichen spezifischen Expressionsaktivitäten durch unterschiedliche RBS-Sequenzen und Abstände zum Startcodon von metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimern SEQ ID NO 38 bis SEQ ID NO 43, der chromosomalen DNA als Template und Pfu Turbo Polymerase (Fa. Stratagene) wurden mit Hilfe der Polymerase-Kettenreaktion (PCR) nach Standardmethoden, wie in Innis et al. (1990) PCR Protocols. A Guide to Methods and Applications, Academic Press beschrieben, die verschiedenen Expressionseinheiten amplifiziert. Dabei diente der Oligonukleotidprimer 1701 (SEQ ID NO 38) als sense Primer und wurde mit den anderen Oligonukleotidprimern kombiniert.
SEQ ID NO 38
   Oligonukleotidprimer 1701
   5'- GAGACTCGAGCGGCTTAAAGTTTGGCTGCC-3'
SEQ ID NO 39
   Oligonukleotidprimer 1828
   5'- ctctcatatgcAATCCCTCCATGAGAAAAATT-3'
SEQ ID NO 40
   Oligonukleotidprimer 1831
   5'- ctctcatatgcgcggccgcAATCCCTCCATGAGAAAAATT-3'
SEQ ID NO 41
   Oligonukleotidprimer 1832
   5'- ctctcatatgcAAtctctccATGAGAAAAATTTTGTGTG-3'
SEQ ID NO 42
   Oligonukleotidprimer 1833
   5'- ctctcatatgcAAtctcctcATGAGAAAAATTTTGTGTG-3'
SEQ ID NO 43
   Oligonukleotidprimer 1834
   5'- ctctcatatgcAAtcccttcATGAGAAAAATTTTGTGTG-3'

Die erhaltenen DNA Fragmente von ca. 200 bp Größe wurden mit dem GFX™PCR, DNA and Gel Band Purification Kit (Amersham Pharmacia, Freiburg) nach Angaben des Herstellers gereinigt.

Der Vektor pBS KS+ (SEQ ID NO: 44) wurde mit dem Restriktionsenzym EcoRV geschnitten und ein 2,9 kb großes Fragment nach elektrophoretischer Auftrennung mit GFX™PCR, DNA and Gel Band Purification Kit isoliert.

Das Vektorfragment wurde zusammen mit den PCR-Fragmenten mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandenen Plasmidewurden mit pKS Pgro 1701/1828, pKS Pgro 1701/1831, pKS Pgro 1701/1832, pKS Pgro 1701/1833 und pKS Pgro 1701/1834 bezeichnet.

Diese Plasmide wurden anschließend mit den Rektriktionenzymen Ndel und Xhol geschnitten. Die erhaltenen DNA Fragmente von ca. 200 bp Größe wurden isoliert und mit dem GFX™PCR, DNA and Gel Band Purification Kit (Amersham Pharmacia, Freiburg) nach Angaben des Herstellers gereinigt.

Der Vektor pCLiK5MCS metA ohne Sartcodon SEQ ID NO: 37 wurde mit den Restriktionsenzymen Ndel und Xhol geschnitten und ein 5 kb großes Fragment nach elektrophoretischer Auftrennung mit GFX™PCR, DNA and Gel Band Purification Kit isoliert.

Das Vektorfragment wurde zusammen mit den 200 bp großen Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandenen Plasmide pCLiK5MCS Pgro 1701/1828 metA, pCLiK5MCS Pgro 1701/1831 metA , pCLiK5MCS Pgro 1701/1832 metA, pCLiK5MCS Pgro 1701/1833 metA und pCLiK5MCS Pgro 1701/1834 metA sind als SEQ ID NO: 45 bis 49 aufgeführt.

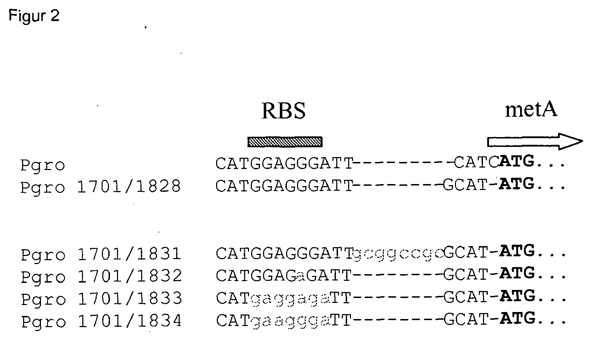

Der Expressionseinheit Pgro wurde durch die Wahl der Olugonukleotide wie in Figur 2 beschrieben verändert.

Der Stamm Corynebacterium glutamicum ATCC13032 wurde jeweils mit den Plasmiden pClik5 MCS, pClik MCS Pgro metA, pCLiK5MCS Pgro 1701/1828 metA, , pCLiK5MCS Pgro 1701/1831 metA, pCLiK5MCS Pgro 1701/1832 metA , pCLiK5MCS Pgro 1701/1833 metA und pCLiK5MCS Pgro 1701/1834 nach der beschriebenen Methode (Liebl, et al. (1989) FEMS Microbiology Letters 53:299-303) transformiert. Die Transformationsmischung wurde auf CM-Platten plattiert, die zusätzlich 20mg/l Kanamycin enthielten, um eine Selektion auf Plasmid-haltige Zellen zu erreichen. Erhaltene Kan-resistente Klone wurden gepickt und vereinzelt.

C. glutamicum Stämme, die eines dieser Plasmidkonstrukte enthielten, wurden in MMA-Medium (40 g/l Saccharose, 20 g/l (NH₄)₂SO₄, 1 g/l KH₂PO₄, 1 g/l K₂HPO₄, 0,25g/l MgSO₄ x 7H₂O, 54 g Aces, 1 ml CaCl2 (10 g/l), 1 ml Protocatechuat (300 mg/10 ml), 1 ml Spurenelementelösung (10 g/l FeSO₄ x /H₂O, 10 g/l MnSO₄ x H₂O, 2 g/l ZnSO₄ x 7 H₂O, 0,2 g/l CuSO₄, 0,02 g/l NiCl₂ x 6 H₂O),100 µg/l Vitamin B₁₂, 0,3 mg/l Thiamin, 1mM Leucin, 1 mg/l Pyridoxal HCl, 1 ml Biotin (100 mg/l), pH7,0) bei 30°C für 5 h angezogen. Die Zellen wurden bei 4°C abzentrifugiert und das zweimal mit kaltem Tris-HCl-Puffer (0,1 %, pH 8,0) gewaschen. Nach erneuter Zentrifugation wurden die Zellen in kalten Tris-HCl-Puffer (0,1 %, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendorfcup überführt. Der Proteingehalt wurde nach Bradford, M.M. (1976) Anal. Biochem. 72:248-254 bestimmt.

Die enzymatische Aktivität von MetA wurde wie folgt durchgeführt. Die Reaktionsansätze von 1 ml enthielten 100 mM Kaliumphosphatpuffer (pH 7,5), 5mM MgCl2, 100 µM Acetyl CoA, 5mM L-Homoserine, 500 µM DTNB (Ellmans Reagenz) und Zellextrakt. Der Test wurde durch Zugabe von dem jeweiligen Proteinlysat gestartet und bei Raumtemperatur inkubiert. Es wurde dann eine Kinetik bei 412 nm über 10 min aufgenommen.

Die Ergebnisse sind in Tabelle 2a gezeigt.

**Tabelle 2a**

| Stamm | spezifische Aktivität [nMol/mg/min] |
|---|---|
| ATCC 13032 pClik5MCS | 7,5 |
| ATCC 13032 pCLiK5MCS Pgro metA | 109,0 |
| ATCC 13032 pCLiK5MCS Pgro 1701/1828 metA | 30,6 |
| ATCC 13032 pCLiK5MCS Pgro 1701/1831 metA | 8,7 |
| ATCC 13032 pCLiK5MCS Pgro 1701/1832 metA | 60,6 |
| ATCC 13032 pCLiK5MCS Pgro 1701/1833 metA | 217,3 |
| ATCC 13032 pCLiK5MCS Pgro 1701/1835 metA | 96,3 |

## Patentansprüche

1. Verfahren zur Erhöhung der Transkriptionsrate von Genen in coryneformen Bakterien im Vergleich zum Wildtyp durch Regulation der Transkription von Genen im coryneformen Bakterium durch Nukleinsäuren mit Promotoraktivität gemäß SEQ.ID.NO. 1 oder eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist,
wobei die Gene mit den Nukleinsäuren mit Promotoraktivität funktionell verknüpft sind und diese funktionelle Verknüpfung der Nukleinsäuren mit Promotoraktivität mit den Genen im Wildtyp nicht vorkommt
**dadurch gekennzeichnet, dass** die Regulation der Transkription von Genen im coryneformen Bakterium durch die Nukleinsäuren mit Promotoraktivität gemäß SEQ.ID.NO. 1 oder eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist, dadurch erreicht wird, dass man
b1) eine oder mehrere der Nukleinsäuren mit Promotoraktivität gemäß SEQ.ID.NO. 1 oder eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist in das Genom des coryneformen Bakteriums einbringt, so dass die Transkription eines oder mehrerer endogenen Gene unter der Kontrolle der eingebrachten Nukleinsäure mit Promotoraktivität erfolgt und die Gene mit den Nukleinsäuren mit Promotoraktivität funktionell verknüpft sind oder
b2) ein oder mehrere Gene in das Genom des corynformen Bakteriums einbringt, so dass die Transkription der eingebrachten Gene unter der Kontrolle der endogenen Nukleinsäuren mit Promotoraktivität gemäß SEQ.ID.NO. 1 oder eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist, erfolgt und die Gene mit den Nukleinsäuren mit Promotoraktivität funktionell so verknüpft sind, dass die eingebrachten Gene am 3'-Ende der endogenen Nukleinsäure mit Promotoraktivität positioniert werden oder
b3) ein oder mehrere Nukleinsäurekonstrukte, enthaltend eine Nukleinsäure mit Promotoraktivität gemäß SEQ.ID.NO. 1 oder eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist und funktionell verknüpft eine oder mehrere zu transkribierende Nukleinsäuren in das coryneforme Bakterium einbringt.

2. Verfahren nach Anspruch 1, **dadurch gekennzeichnet, dass** die Gene ausgewählt sind aus der Gruppe Nukleinsäuren kodierend ein Protein aus dem Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindungen, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

3. Verfahren nach Anspruch 2, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulater LuxR, Transkriptioneller Regulator LysR!, Transkriptioneller Regulator LysR2, Malat-Quinon-Oxireduktase, Glucose-6-Phosphat-Dehydrogenase, 6-Phosphogluconat-Dehydrogenase, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase,Threonin-Synthase, Threanin-Exporter-Carrier, Threonin-Dehydratase, Pyruvat-Oxidase, Lysin-Exporter, Biotin-Ligase, Cystein-Synthase I, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-Sulfit-Reduktase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Frukose-1,6-biphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

4. Genetisch verändertes coryneformes Bakterium, wobei die genetische Veränderung zu einer Erhöhung der Transkriptionsrate von mindestens einem Gen im Vergleich zum Wildtyp führt und bedingt ist durch
Regulation der Transkription von Genen im coryneformen Bakterium durch Nukleinsäuren mit Promotoraktivität
A) gemäß SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist,
wobei die Gene mit den Nukleinsäuren mit Promotoraktivität funktionell verknüpft sind und diese funktionelle Verknüpfung der Nukleinsäuren mit Promotoraktivität mit den Genen im Wildtyp nicht vorkommt,
**dadurch gekennzeichnet, dass** die Regulation der Transkription von Genen im coryneformen Bakterium durch Nukleinsäuren mit Promotoraktivität
A) gemäß SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist,
dadurch erreicht wird, dass man
b1) eine oder mehrere der Nukleinsäuren mit Promotoraktivität in das Genom des coryneformen Bakteriums einbringt, so dass die Transkription eines oder mehrerer endogenen Gene unter der Kontrolle der eingebrachten Nukleinsäure mit Promotoraktivität erfolgt und die Gene mit den Nukleinsäuren mit Promotoraktivität funktionell verknüpft sind oder
b2) ein oder mehrere Gene in das Genom des coryneformen Bakteriums einbringt, so dass die Transkription eines oder mehrerer der eingebrachten Gene unter der Kontrolle der endogenen Nukleinsäuren mit Promotoraktivität erfolgt und die Gene mit den Nukleinsäuren mit Promotoraktivität funktionell funktionell so verknüpft sind, dass die eingebrachten Gene am 3'-Ende der endogenen Nukleinsäure mit Promotoraktivität positioniert werden oder
b3) ein oder mehrere Nukleinsäurekonstrukte, enthaltend eine Nukleinsäure mit Promotoraktivität und funktionell verknüpft eine oder mehrere zu transkribierende Nukleinsäuren in das coryneforme Bakterium einbringt.

5. Genetisch verändertes coryneformes Bakterium Mikroorganismus gemäss Anspruch 4, **dadurch gekennzeichnet, dass** die Gene ausgewählt sind aus der Gruppe Nukleinsäuren kodierend ein Protein aus dem Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindungen, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

6. Genetisch verändertes coryneformes Bakterium gemäß Anspruch 5, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulater LuxR, Transkriptioneller Regulator LysR!, Transkriptioneller Regulator LysR2, Malat-Quinon-Oxireduktase, Glucose-6-Phosphat-Dehydrogenase, 6-Phosphogluconat-Dehydrogenase, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase,Threonin-Synthase, Threonin-Exporter-Carrier, Threonin-Dehydratase, Pyruvat-Oxidase, Lysin-Exporter, Biotin-Ligase, Cystein-Synthase I, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-Sulfit-Reduktase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Frukose-1,6-biphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

## Claims

1. A method for increasing the transcription rate of genes in coryneform bacteria compared with the wild type by regulating the transcription of genes in the coryneform bacterium by nucleic acids having promoter activity according to SEQ. ID. NO. 1 or a sequence derived from this sequence by substitution, insertion or deletion of nucleotides and having an identity of at least 90% at the nucleic acid level with the sequence SEQ. ID. NO. 1,
said genes being functionally linked to the nucleic acids having promoter activity and said functional linkage of the nucleic acids having promoter activity to said genes not occurring in the wild type
wherein the regulation of the transcription of genes in the coryneform bacterium by the nucleic acids having promoter activity according to SEQ. ID. NO. 1 or a sequence derived from this sequence by substitution, insertion or deletion of nucleotides and having an identity of at least 90% at the nucleic acid level with the sequence SEQ. ID. NO. 1 is achieved by
b1) introducing one or more nucleic acids having promoter activity according to SEQ. ID. NO. 1 or a sequence derived from this sequence by substitution, insertion or deletion of nucleotides and having an identity of at least 90% at the nucleic acid level with the sequence SEQ. ID. NO. 1 into the genome of the coryneform bacterium so that transcription of one or more endogenous genes takes place under the control of the introduced nucleic acid having promoter activity and the genes are functionally linked to the nucleic acids having promoter activity, or
b2) introducing one or more genes into the genome of the coryneform bacterium so that transcription of the introduced genes takes place under the control of the endogenous nucleic acids having promoter activity according to SEQ. ID. NO. 1 or a sequence derived from this sequence by substitution, insertion or deletion of nucleotides and having an identity of at least 90% at the nucleic acid level with the sequence SEQ. ID. NO. 1, and the genes are functionally linked to the nucleic acids having promoter activity such that the genes introduced are positioned at the 3' end of the endogenous nucleic acid having promoter activity, or
b3) introducing one or more nucleic acid constructs comprising a nucleic acid having promoter activity according to SEQ. ID. NO. 1 or a sequence derived from this sequence by substitution, insertion or deletion of nucleotides and having an identity of at least 90% at the nucleic acid level with the sequence SEQ. ID. NO. 1, and functionally linked one or more nucleic acids to be transcribed, into the coryneform bacterium.

2. The method according to claim 1, wherein the genes are selected from the group of nucleic acids encoding a protein from the biosynthetic pathway of proteinogenic and non-proteinogenic amino acids, nucleic acids encoding a protein from the biosynthetic pathway of nucleotides and nucleosides, nucleic acids encoding a protein from the biosynthetic pathway of organic acids, nucleic acids encoding a protein from the biosynthetic pathway of lipids and fatty acids, nucleic acids encoding a protein from the biosynthetic pathway of diols, nucleic acids encoding a protein from the biosynthetic pathway of carbohydrates, nucleic acids encoding a protein from the biosynthetic pathway of aromatic compounds, nucleic acids encoding a protein from the biosynthetic pathway of vitamins, nucleic acids encoding a protein from the biosynthetic pathway of cofactors and nucleic acids encoding a protein from the biosynthetic pathway of enzymes, where the genes may optionally comprise further regulatory elements.

3. The method according to claim 2, wherein the proteins from the biosynthetic pathway of amino acids are selected from the group of aspartate kinase, aspartate-semialdehyde dehydrogenase, diaminopimelate dehydrogenase, diaminopimelate decarboxylase, dihydrodipicolinate synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR1, transcriptional regulator LysR2, malate-quinone oxireductase, glucose-6-phosphate dehydrogenase, 6-phosphogluconate dehydrogenase, transketolase, transaldolase, homoserine O-acetyltransferase, cystathionine gamma-synthase, cystathionine beta-lyase, serine hydroxymethyltransferase, O-acetylhomoserine sulfhydrylase, methylenetetrahydrofolate reductase, phosphoserine aminotransferase, phosphoserine phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cysteine synthase II, coenzyme B12-dependent methionine synthase, coenzyme B12-independent methionine synthase, sulfate adenylyltransferase subunit 1 and 2, phosphoadenosine-phosphosulfate reductase, ferredoxin-sulfite reductase, ferredoxin NADP reductase, 3-phosphoglycerate dehydrogenase, RXA00655 regulator, RXN2910 regulator, arginyl-tRNA synthetase, phosphoenolpyruvate carboxylase, threonine efflux protein, serine hydroxymethyltransferase, fructose-1,6-bisphosphatase, protein of sulfate reduction RXA077, protein of sulfate reduction RXA248, protein of sulfate reduction RXA247, protein OpcA, 1-phosphofructokinase and 6-phosphofructokinase.

4. A genetically modified coryneform bacterium, where the genetic modification leads to an increase of the transcription rate of at least one gene compared with the wild type, and is dependent on regulating the transcription of genes in the coryneform bacterium by nucleic acids having promoter activity
A) according to SEQ. ID. NO. 1 or
B) a sequence derived from this sequence by substitution, insertion or deletion of nucleotides and having an identity of at least 90% at the nucleic acid level with the sequence SEQ. ID. NO. 1,
said genes being functionally linked to the nucleic acids having promoter activity and said functional linkage of the nucleic acids having promoter activity to said genes not occurring in the wild type,
wherein the regulation of the transcription of genes in the coryneform bacterium by nucleic acids having promoter activity
A) according to SEQ. ID. NO. 1 or
B) a sequence derived from this sequence by substitution, insertion or deletion of nucleotides and having an identity of at least 90% at the nucleic level with the sequence SEQ. ID. NO. 1, is achieved by
b1) introducing one or more nucleic acids having promoter activity into the genome of the coryneform bacterium so that transcription of one or more endogenous genes takes place under the control of the introduced nucleic acid having promoter activity and the genes are functionally linked to the nucleic acids having promoter activity, or
b2) introducing one or more genes into the genome of the coryneform bacterium so that transcription of one or more of the introduced genes takes place under the control of the endogenous nucleic acids having promoter activity and the genes are functionally linked to the nucleic acids having promoter activity such that the genes introduced are positioned at the 3' end of the endogenous nucleic acid having promoter activity, or
b3) introducing one or more nucleic acid constructs comprising a nucleic acid having promoter activity, and functionally linked one or more nucleic acids to be transcribed, into the coryneform bacterium.

5. The genetically modified coryneform bacterium microorganism according to claim 4, wherein the genes are selected from the group of nucleic acids encoding a protein from the biosynthetic pathway of proteinogenic and non-proteinogenic amino acids, nucleic acids encoding a protein from the biosynthetic pathway of nucleotides and nucleosides, nucleic acids encoding a protein from the biosynthetic pathway of organic acids, nucleic acids encoding a protein from the biosynthetic pathway of lipids and fatty acids, nucleic acids encoding a protein from the biosynthetic pathway of diols, nucleic acids encoding a protein from the biosynthetic pathway of carbohydrates, nucleic acids encoding a protein from the biosynthetic pathway of aromatic compounds, nucleic acids encoding a protein from the biosynthetic pathway of vitamins, nucleic acids encoding a protein from the biosynthetic pathway of cofactors and nucleic acids encoding a protein from the biosynthetic pathway of enzymes, where the genes may optionally comprise further regulatory elements.

6. The genetically modified coryneform bacterium according to claim 5, wherein the proteins from the biosynthetic pathway of amino acids are selected from the group of aspartate kinase, aspartate-semialdehyde dehydrogenase, diaminopimelate dehydrogenase, diaminopimelate decarboxylase, dihydrodipicolinate synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR1, transcriptional regulator LysR2, malate-quinone oxireductase, glucose-6-phosphate dehydrogenase, 6-phosphogluconate dehydrogenase, transketolase, transaldolase, homoserine O-acetyltransferase, cystathionine gamma-synthase, cystathionine beta-lyase, serine hydroxymethyltransferase, O-acetylhomoserine sulfhydrylase, methylenetetrahydrofolate reductase, phosphoserine aminotransferase, phosphoserine phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cysteine synthase II, coenzyme B12-dependent methionine synthase, coenzyme B12-independent methionine synthase, sulfate adenylyltransferase subunit 1 and 2, phosphoadenosine-phosphosulfate reductase, ferredoxin-sulfite reductase, ferredoxin NADP reductase, 3-phosphoglycerate dehydrogenase, RXA00655 regulator, RXN2910 regulator, arginyl-tRNA synthetase, phosphoenolpyruvate carboxylase, threonine efflux protein, serine hydroxymethyltransferase, fructose-1,6-bisphosphatase, protein of sulfate reduction RXA077, protein of sulfate reduction RXA248, protein of sulfate reduction RXA247, protein OpcA, 1-phosphofructokinase and 6-phosphofructokinase.

## Revendications

1. Procédé pour augmenter le taux de transcription de gènes dans des bactéries corynéformes par comparaison avec le type sauvage, par régulation de la transcription de gènes dans des bactéries corynéformes par des acides nucléiques ayant une activité promotrice selon SEQ ID NO:1 ou une séquence dérivant de cette séquence par substitution, insertion ou délétion de nucléotides, et qui présente une identité d'au moins 90 %, au niveau des acides nucléiques, avec la séquence SEQ ID NO:1,
dans lequel les gènes sont fonctionnellement liés aux acides nucléiques ayant une activité promotrice, et cette liaison fonctionnelle des acides nucléiques ayant une activité promotrice dans les gènes de type sauvage n'a pas lieu,
**caractérisé en ce que** la régulation de la transcription de gènes dans la bactérie corynéforme est réalisée par les acides nucléiques ayant une activité promotrice selon SEQ ID NO:1 ou une séquence dérivant de cette séquence par substitution, insertion ou délétion de nucléotides, et qui présente une identité d'au moins 90 % au niveau des acides nucléiques avec la séquence SEQ ID NO:1, par le fait que
b1) on introduit dans le génome de la bactérie corynéforme un ou plusieurs des acides nucléiques ayant une activité promotrice selon SEQ ID NO:1 ou une séquence dérivant de cette séquence par substitution, insertion ou délétion de nucléotides, et qui présente une identité d'au moins 90 % au niveau des acides nucléiques avec la séquence SEQ ID NO:1, de telle sorte que la transcription d'un ou plusieurs gènes endogènes ait lieu sous le contrôle de l'acide nucléique introduit ayant une activité promotrice, et que les gènes soient fonctionnellement liés aux acides nucléiques ayant une activité promotrice, ou
b2) on introduit un ou plusieurs gènes dans le génome de la bactérie corynéforme, de telle sorte que la transcription des gènes introduits se fasse sous le contrôle des acides nucléiques endogènes ayant une activité promotrice selon SEQ ID NO:1 ou une séquence dérivant de cette séquence par substitution, insertion ou délétion de nucléotides, et qui présente une identité d'au moins 90 % au niveau des acides nucléiques avec la séquence SEQ ID NO:1, et que les gènes soient fonctionnellement liés aux acides nucléiques ayant une activité promotrice de telle sorte que les gènes introduits soient positionnés à l'extrémité 3' de l'acide nucléique ayant une activité promotrice, ou
b3) on introduit dans la bactérie corynéforme une ou plusieurs constructions d'acides nucléiques, contenant un acide nucléique ayant une activité promotrice selon SEQ ID NO:1 ou une séquence dérivant de cette séquence par substitution, insertion ou délétion de nucléotides, et qui présente une identité d'au moins 90 % au niveau des acides nucléiques avec la séquence SEQ ID NO:1 et, fonctionnellement liés, un ou plusieurs des acides nucléiques à transcrire.

2. Procédé selon la revendication 1, **caractérisé en ce que** les gènes sont choisis dans le groupe des acides nucléiques codant pour une protéine de la voie de biosynthèse des acides aminés protéinogènes et non protéinogènes, des acides nucléiques codant pour une protéine de la voie de biosynthèse des nucléotides et des nucléosides, des acides nucléiques codant pour une protéine de la voie de biosynthèse des acides organiques, des acides nucléiques codant pour une protéine de la voie de biosynthèse des lipides et des acides gras, des acides nucléiques codant pour une protéine de la voie de biosynthèse des diols, des acides nucléiques codant pour une protéine de la voie de biosynthèse des hydrates de carbone, des acides nucléiques codant pour une protéine de la voie de biosynthèse des composés aromatiques, des acides nucléiques codant pour une protéine de la voie de biosynthèse des vitamines, des acides nucléiques codant pour une protéine de la voie de biosynthèse des cofacteurs, et des acides nucléiques codant pour une protéine de la voie de biosynthèse des enzymes, les gènes pouvant éventuellement contenir des éléments régulateurs supplémentaires.

3. Procédé selon la revendication 2, **caractérisé en ce que** les protéines de la biosynthèse des acides aminés sont choisies dans le groupe consistant en l'aspartate kinase, l'aspartate-semialdéhyde-déshydrogénase, la diaminopimélate-déshydrogénase, la diaminopimélate-décarboxylase, la dihydrodipicolinate-synthétase, la dihydrodipicolinate-réductase, la glycérinaldéhyde-3-phosphate-déshydrogénase, la 3-phosphoglycérate-kinase, la pyruvate-carboxylase, la triosephosphate-isomérase, le régulateur transcriptionnel LuxR, le régulateur transcriptionnel LysR1, le régulateur transcriptionnel LysR2, la malate-quinone-oxyréductase, la glucose-6-phosphate-déshydrogénase, la 6-phosphogluconate-déshydrogénase, la transkétolase, la transaldolase, l'homosérine-O-acétyltransférase, la cystathionine-gamma-synthase, la cystathionine-bêta-lyase, la sérine-hydroxyméthyltransférase, l'O-acétylhomosérine-sulfhydrylase, la méthylène-tétrahydrofolate-réductase, la phosphosérine-aminotransférase, la phosphosérine-phosphatase, la sérine-acétyl-transférase, l'homosérine-déshydrogénase, l'homosérine-kinase, la thréonine-synthase, le support d'exportation de la thréonine, la thréonine-déshydratase, la pyruvate-oxydase, l'exportateur de lysine, la biotine-ligase, la cystéine-synthase I, la cystéine-synthase II, la méthionine-synthase coenzyme B12-dépendante, la méthionine-synthase coenzyme B12-indépendante, les sous-unités 1 et 2 de la sulfate adénylyltransférase, la phosphoadénosine phosphosulfate réductase, la ferrédoxine-sulfite-réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate-carboxylase, la protéine d'efflux de la thréonine, la sérine hydroxyméthyltransférase, la fructose-1,6-biphosphatase, la protéine de réduction des sulfates RXA077, la protéine de réduction des sulfates RXA248, la protéine de réduction des sulfates RXA247, la protéine OpcA, la 1-phosphofructokinase et la 6-phosphofructokinase.

4. Bactérie corynéforme génétiquement modifiée, dans laquelle la modification génétique conduit à une augmentation du taux de transcription d'au moins un gène par comparaison avec le type sauvage, et est provoquée par une régulation de la transcription de gènes dans la bactérie corynéforme par des acides nucléiques ayant une activité promotrice
A) selon SEQ ID NO:1, ou
B) une séquence dérivant de cette séquence par substitution, insertion ou délétion de nucléotides, et qui présente une identité d'au moins 90 % au niveau des acides nucléiques avec la séquence SEQ ID NO:1,
les gènes étant fonctionnellement liés aux acides nucléiques ayant une activité promotrice, et cette liaison fonctionnelle des acides nucléiques ayant une activité promotrice aux gènes du type sauvage n'ayant pas lieu,
**caractérisée en ce que** la régulation de la transcription de gènes dans la bactérie corynéforme est réalisée par des acides nucléiques ayant une activité promotrice
A) selon SEQ ID NO:1 ou
B) une séquence dérivant de cette séquence par substitution, insertion ou délétion de nucléotides, et qui présente une identité d'au moins 90 % au niveau des acides nucléiques avec la séquence SEQ ID NO:1,
par le fait que
b1) on introduit dans le génome de la bactérie corynéforme un ou plusieurs des acides nucléiques ayant une activité promotrice, de telle sorte que la transcription d'un ou plusieurs gènes endogènes s'effectue sous le contrôle de l'acide nucléique introduit ayant une activité promotrice, et que les gènes soient fonctionnellement liés aux acides nucléiques ayant une activité promotrice, ou
b2) on introduit dans le génome de la bactérie corynéforme un ou plusieurs gènes, de telle sorte que la transcription d'un ou plusieurs des gènes introduits s'effectue sous le contrôle des acides nucléiques endogènes ayant une activité promotrice, et que les gènes soient fonctionnellement liés aux acides nucléiques ayant une activité promotrice de telle sorte que les gènes introduits soient positionnés à l'extrémité 3' de l'acide nucléique endogène ayant une activité promotrice, ou
b3) on introduit dans la bactérie corynéforme une ou plusieurs constructions d'acides nucléiques, contenant un acide nucléique ayant une activité promotrice et, fonctionnellement liés, un ou plusieurs acides nucléiques à transcrire.

5. Microorganisme bactérien corynéforme génétiquement modifié selon la revendication 4, **caractérisé en ce que** les gènes sont choisis dans le groupe des acides nucléiques codant pour une protéine de la voie de biosynthèse des acides aminés protéinogènes et non protéinogènes, des acides nucléiques codant pour une protéine de la voie de biosynthèse des nucléotides et des nucléosides, des acides nucléiques codant pour une protéine de la voie de biosynthèse des acides organiques, des acides nucléiques codant pour une protéine de la voie de biosynthèse des lipides et des acides gras, des acides nucléiques codant pour une protéine de la voie de biosynthèse des diols, des acides nucléiques codant pour une protéine de la voie de biosynthèse des hydrates de carbone, des acides nucléiques codant pour une protéine de la voie de biosynthèse des composés aromatiques, des acides nucléiques codant pour une protéine de la voie de biosynthèse des vitamines, des acides nucléiques codant pour une protéine de la voie de biosynthèse des cofacteurs, et des acides nucléiques codant pour une protéine de la voie de biosynthèse des enzymes, les gènes pouvant éventuellement contenir des éléments régulateurs supplémentaires.

6. Bactérie corynéforme génétiquement modifiée selon la revendication 5, **caractérisée en ce que** les protéines de la biosynthèse des acides aminés sont choisies dans le groupe consistant en l'aspartate kinase, l'aspartate-semialdéhyde-déshydrogénase, la diaminopimélate-déshydrogénase, la diaminopimélate-décarboxylase, la dihydrodipicolinate-synthétase, la dihydrodipicolinate-réductase, la glycérinaldéhyde-3-phosphate-déshydrogénase, la 3-phosphoglycérate-kinase, la pyruvate-carboxylase, la triosephosphate-isomérase, le régulateur transcriptionnel LuxR, le régulateur transcriptionnel LysR1, le régulateur transcriptionnel LysR2, la malate-quinone-oxyréductase, la glucose-6-phosphate-déshydrogénase, la 6-phosphogluconate-déshydrogénase, la transkétolase, la transaldolase, l'homosérine-O-acétyltransférase, la cystathionine-gamma-synthase, la cystathionine-bêta-lyase, la sérine-hydroxyméthyltransférase, l'O-acétylhomosérine-sulfhydrylase, la méthylène-tétrahydrofolate-réductase, la phosphosérine-aminotransférase, la phosphosérine-phosphatase, la sérine-acétyl-transférase, l'homosérine-déshydrogénase, l'homosérine-kinase, la thréonine-synthase, le support d'exportation de la thréonine, la thréonine-déshydratase, la pyruvate-oxydase, l'exportateur de lysine, la biotine-ligase, la cystéine-synthase I, la cystéine-synthase II, la méthionine-synthase coenzyme B12-dépendante, la méthionine-synthase coenzyme B12-indépendante, les sous-unités 1 et 2 de la sulfate adénylyltransférase, la phosphoadénosine phosphosulfate réductase, la ferrédoxine-sulfite-réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate-carboxylase, la protéine d'efflux de la thréonine, la sérine hydroxyméthyltransférase, la fructose-1,6-biphosphatase, la protéine de réduction des sulfates RXA077, la protéine de réduction des sulfates RXA248, la protéine de réduction des sulfates RXA247, la protéine OpcA, la 1-phosphofructokinase et la 6-phosphofructokinase.
